(19)

**Europäisches Patentamt**

**European Patent Office**

**Office européen des brevets**

(11)  **EP 4 311 543 A1**

(12) **EUROPEAN PATENT APPLICATION**

(43) Date of publication:
**31.01.2024  Bulletin 2024/05**

(21) Application number: **23151848.1**

(22) Date of filing: **16.01.2023**

(51) International Patent Classification (IPC):
***A61K 9/50*** (2006.01)

(52) Cooperative Patent Classification (CPC):
**A61K 9/501; A61K 9/5089**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.07.2022  EP 22187339**

(71) Applicant: **Galvita AG
4058 Basel (CH)**

(72) Inventors:
- **PUCHKOV, Maxim
  4148 Pfeffingen (CH)**
- **HAAG, Roland
  79541 Lörrach (DE)**

(74) Representative: **Vossius & Partner
Patentanwälte Rechtsanwälte mbB
Siebertstraße 3
81675 München (DE)**

(54)  **METHODS FOR LOADING TEMPLATE INVERTED CARRIERS**

(57)  The invention relates to method for loading a carrier particle with secondary internal structures, the method comprising the steps of: i) providing a carrier particle with secondary internal structures; ii) bringing the carrier particle into contact with a loading liquid comprising a loading agent, wherein the loading liquid is partially wetting the carrier particle; and iii) removing the loading liquid, whereby the loading agent remains on the carrier particle; characterized in that the carrier particle is obtainable by the steps of: a) combining a carrier material with a template material, wherein the carrier material forms a primary structure around the template material; b) transforming the template material; c) removing the transformed template material; and d) obtaining carrier particles with secondary internal structures. Further, the invention relates to a carrier particle with secondary internal structures obtainable by the method according to the invention.

EP 4 311 543 A1

**Description**

The invention relates to method for loading a carrier particle with secondary internal structures, the method comprising the steps of: i) providing a carrier particle with secondary internal structures; ii) bringing the carrier particle into contact with a loading liquid comprising a loading agent, wherein the loading liquid is partially wetting the carrier particle; and iii) removing the loading liquid, whereby the loading agent remains on the carrier particle; characterized in that the carrier particle is obtainable by the steps of: a) combining a carrier material with a template material, wherein the carrier material forms a primary structure around the template material; b) transforming the template material; c) removing the transformed template material; and d) obtaining carrier particles with secondary internal structures. Further, the invention relates to a carrier particle with secondary internal structures obtainable by the method according to the invention.

Drug delivery of solid pharmaceutical compositions is accompanied by several challenges to the design. The disintegration time of the solid pharmaceutical composition delays the onset of the therapeutic effect. Drug loading capacities limit the maximal dosage per solid pharmaceutical composition. Insufficient mechanical stability can have a negative influence on the shelf life of the solid pharmaceutical composition. Swallowing of solid pharmaceuticals compositions can represent a problem for patients, in particular pediatric and/or geriatric patients.

Improvement of a desired drug delivery property of a solid pharmaceutical composition usually results in an undesired effect to another property of the solid pharmaceutical composition. For example, hard mechanical stable solid pharmaceutical compositions usually have a longer disintegration time (see, e.g., Kitazawa, S. et al., 1975, The Journal of pharmacy and pharmacology, 27(10), 765-770) but may be more difficult to swallow.

Templated drug carrier particles are widely used in modern drug delivery and are used for site-specific (e.g., tissue-specific) delivery of active pharmaceutical components (see, e.g., Rosenholm, J. M., et al., 2010 Nanoscale, 2(10), 1870-1883). Techniques to produce carrier particles that are hollow in liquids are known in the art (see, e.g., WO1999047253; Donath, E., et al., 1998, Angewandte Chemie International Edition, 37(16), 2201-2205). However, no attention was paid to the application of carrier particles to improve the properties of solid pharmaceutical compositions in administrable form.

Therefore, there is a need for improved means and methods to obtain solid pharmaceutical compositions with desired drug delivery properties.

The above technical problem is solved by the embodiments disclosed herein and as defined in the claims.

Accordingly, the invention relates to, inter alia, the following embodiments:

1. A method for loading a carrier particle with secondary internal structures, the method comprising the steps of:

i) providing a carrier particle with secondary internal structures;

ii) bringing the carrier particle into contact with a loading liquid comprising a loading agent, wherein the loading liquid is partially wetting the carrier particle; and

iii) removing the loading liquid, whereby the loading agent remains on the carrier particle;
characterized in that the carrier particle is obtainable by the steps of:

a) combining a carrier material with a template material, wherein the carrier material forms a primary structure around the template material;

b) transforming the template material;

c) removing the transformed template material; and

d) obtaining carrier particles with secondary internal structures.

2. The method of embodiment 1, wherein bringing the carrier particle into contact with a loading liquid comprises a spray loading method and/or a single droplet loading method, preferably a method selected from the group consisting of: fluidized bed, spouted bed, and fluidized bed with Wurster insert.

3. The method according to embodiment 1 or 2, wherein the template material is an inorganic material or consists primarily of inorganic material.

4. The method according to any one of the embodiments 1 to 3, wherein the carrier material is an inorganic material or consists primarily of inorganic material.

5. The method according to embodiment 3 or 4, wherein the carrier material and the template material are inorganic salts or consist primarily of inorganic salts.

6. The method according to any one of embodiments 1 to 5, wherein combining a carrier material with a template material comprises chemical precipitation, layering and/or crystallization of the carrier material on the template material.

7. The method according to any one of embodiments 1 or 6, wherein transforming the template material comprises heating to a temperature from 600 °C to 1200 °C.

8. The method according to embodiment 7, wherein transforming the template material comprises heating to a temperature from 600 °C to 900 °C.

9. The method according to embodiment 7 or 8, wherein the step of transforming the template material comprises calcination.

10. The method according to any one of embodiments 7 to 9, wherein the step of transforming the template material comprises a subsequent addition of water.

11. The method according to embodiment 10, wherein the addition of water is an exothermic reaction.

12. The method according to any one of embodiments 1 to 11, wherein removing the template material comprises dissolution of the transformed template material to form secondary internal structures.

13. The method according to any one of embodiments 3 to 12, wherein the template material comprises calcium carbonate.

14. The method according to any one of embodiments 4 to 13, wherein the carrier material comprises at least one salt and/or complex selected from the group of calcium phosphate and magnesium phosphate.

15. The method according to embodiment 14, wherein the carrier particles have a diameter of 1 to 300 $\mu$m.

16. The method according to embodiment 14 or 15, wherein the carrier particles have a surface area between 15m2/g to 400m2/g.

17. The method according to any one of embodiments 14 to 16, wherein the secondary internal structure comprises pores having a diameter size in the range of $\geq$ 0.2 $\mu$m and $\leq$ 1.5 $\mu$m.

18. The method according to any one of the embodiments 1 to 17, wherein the loading liquid is provided in form of droplets, preferably wherein the droplet has an average diameter size in the range of 50$\mu$m and 3mm.

19. The method according do any one of the embodiments 1 to 18, wherein the carrier particle is provided in a batch comprising a plurality of particles and having a batch volume, wherein the volume of the loading liquid in contact with the batch is smaller than the batch volume.

20. The method according to any one of embodiments 14 to 19, wherein the total volume of the secondary internal structures in the obtained carrier particles with secondary internal structures is in the range of $\geq$ 10% to $\leq$ 90% of the particle volume.

21. The method of any one of the embodiments 2 to 20, wherein removing the loading liquid comprises evaporation.

22. A loaded carrier particle with secondary internal structures obtainable by the method according to any one of embodiments 1 to 21.

23. The carrier particle according to embodiment 22, wherein the carrier particle is loaded $\geq$ 60% v/v.

24. The carrier particle according to embodiment 22 or 23, wherein the loading agent is a therapeutic agent, preferably wherein the therapeutic agent is selected from the group consisting of: ivermectin, midazolam, nalbuphine, buprenorphine, omeprazole, esomeprazole, caffeine, rizatriptan, zolmitriptan, and sumatriptan.

25. A method for the production of a compacted loaded carrier matter, the method comprising the steps of:

   a) loading a carrier particle according to any one of embodiments 1 to 21; and

   b) compacting the loaded carrier particles with secondary internal structures to obtain the compacted loaded carrier matter.

26. A solid pharmaceutical composition comprising the loaded carrier particle according to any one of embodiments 22 to 24 or the compacted loaded carrier matter produced according to embodiment 25.

27. The solid pharmaceutical composition according to embodiment 26, the compacted loaded carrier matter produced according to embodiment 25 or the loaded carrier particle according to embodiment 24, wherein the therapeutic agent is selected from the group of anxiolytic agents, sedative agents, narcotic agents, antidepressant agents, antimigraine agents, anti-inflammatory agents, and anti-infective agents, preferably an agent selected from the group consisting of: ivermectin, midazolam, nalbuphine, buprenorphine, omeprazole, esomeprazole, caffeine, rizatriptan, zolmitriptan and sumatriptan.

28. The solid pharmaceutical composition according to embodiment 26 or 27, wherein the solid pharmaceutical composition comprises at least one adjuvant.

29. The solid pharmaceutical composition according to embodiment 28, wherein the at least one adjuvant is selected from the group of disintegrants, lubricants, and flowability enhancement agents.

30. The solid pharmaceutical composition according to embodiment 28 or 29, wherein the at least one adjuvant is selected from the group taste altering agents, smell altering agents, and appearance altering agents.

31. The solid pharmaceutical composition according to embodiment 30, wherein the taste altering agent is selected from the group of artificial sweeteners, acidity modifiers, gums, cellulose derivatives, hard fats, and salts.

32. The solid pharmaceutical composition according to any of embodiments 26 to 31, the compacted loaded carrier matter produced according to embodiment 23, or the loaded carrier particle according to embodiment 24 for use in treatment.

33. The solid pharmaceutical composition for use according to embodiment 32, the compacted loaded carrier matter for use according to embodiment 32, or the loaded carrier particle for use according to embodiment 32 for use in the treatment of a geriatric disease or disorder.

34. The solid pharmaceutical composition for use according to embodiment 32, the compacted loaded carrier matter for use according to embodiment 32 or the loaded carrier particle for use according to embodiment 32 for use in the treatment of a pediatric disease or disorder; or
the solid pharmaceutical composition for use according to embodiment 33, the compacted loaded carrier matter for use according to embodiment 33, or the loaded carrier particle for use according to embodiment 33, wherein the geriatric disease or disorder is a geriatric and pediatric disease or disorder.

35. The solid pharmaceutical composition for use according to embodiment 32, the compacted loaded carrier matter for use according to embodiment 32, or the loaded carrier particle for use according to embodiment 32 for use in the treatment of a disease or disorder selected from the group of anxiety disorders, bipolar disorders, pain, infections, migraine, sleeping disorders, and depressive disorders; or
the solid pharmaceutical composition for use according to embodiment 33 or 34, the compacted loaded carrier matter for use according to embodiment 33 or 34 or the loaded carrier particle for use according to embodiment 33 or 34, wherein the pediatric disease or disorder, the geriatric disease or disorder or the geriatric and pediatric disease or disorder are selected from the group of anxiety disorders, bipolar disorders, pain, infections, migraine, sleeping disorders, and depressive disorders.

36. The solid pharmaceutical composition for use according to embodiment 32, the compacted loaded carrier matter for use according to embodiment 32, or the loaded carrier particle for use according to embodiment 32, for use in the treatment of a veterinary disease or disorder.

37. The solid pharmaceutical composition according to any of embodiments 26 to 31, the compacted loaded carrier matter produced according to embodiment 25, or the loaded carrier particle according to embodiment 24 for use in diagnostic purposes.

38. The solid pharmaceutical composition according to embodiment 37, the compacted loaded carrier matter produced according to embodiment 37 or the loaded carrier particle according to embodiment 37 for use in scintigraphy.

Accordingly, in a first embodiment, the invention relates to a method for loading a carrier particle with secondary internal structures, the method comprising the steps of: i) providing a carrier particle with secondary internal structures; ii) bringing the carrier particle into contact with a loading liquid comprising a loading agent, wherein the loading liquid is partially wetting the carrier particle; and iii) removing the loading liquid, whereby the loading agent remains on the carrier particle; characterized in that the carrier particle is obtainable by the steps of: a) combining a carrier material with a template material, wherein the carrier material forms a primary structure around the template material; b) transforming the template material; c) removing the transformed template material; and d) obtaining carrier particles with secondary internal structures.

It was surprisingly found, that loading methods wherein the carrier particles are incompletely wetted are particularly advantageous in that the loading occurs to a higher degree inside the particle and to a lesser degree on the surface of the outer surface.

The steps of ii) bringing the carrier particle into contact with a loading liquid and iii) removing the loading liquid may occur simultaneously and/or sequentially, preferably simultaneously. Without being bound by theory, the beneficial effect is likely linked to the simultaneous presence of the gaseous (for liquid removal), liquid (loading liquid addition) and solid (loaded loading agent) phase. This helps to exploit the capillary forces of the structure of the carrier particles facilitates beneficial loading.

The inventors further found that carrier particles loaded as described herein have flowability and/or compressibility properties that is/are beneficial in the production of solid pharmaceutical compositions. These properties are unexpectedly improved compared to non-loaded carrier particles.

In certain embodiments, the invention relates to the method of the invention, wherein the loading liquid is provided in form of droplets.

In certain embodiments, the invention relates to the method of the invention, wherein bringing the carrier particle into contact with a loading liquid comprises a spray loading method and/or a single droplet loading method. In some embodiments, the single droplet loading method further comprises mechanical stirring of the carrier particles.

In certain embodiments, the invention relates to the method of the invention, wherein bringing the carrier particle into contact with a loading liquid comprises a method selected from the group consisting of: fluidized bed, spouted bed, and fluidized bed with Wurster insert.

The inventors found that methods, wherein the loading liquid is provided in droplet form are particularly efficient in wetting a large portion of a carrier particle batch partially.

In certain embodiments, the invention relates to the method of the invention, wherein the droplet has an average diameter size in the range of $50\mu m$ and 3mm, preferably in the range of $100\mu m$ and 1mm.

The inventors found that droplets in a certain size range can exploit capillary forces particularly well in the context of the invention. The optimal droplet size may further depend on the pore size of the carrier particles.

In certain embodiments, the invention relates to the method of the invention, wherein the carrier particle is provided in a batch comprising a plurality of particles and having a batch volume, wherein the volume of the loading liquid in contact with the batch is smaller than the batch volume.

The inventors found that the volume of loading liquid depends on the volume of the carrier particle batch.

In certain embodiments, the invention relates to the method of the invention, wherein removing the loading liquid comprises evaporation.

In certain embodiments, the invention relates to a method for the production of carrier particles with secondary internal structures comprising the steps of a) combining a carrier material with a template material, wherein the carrier material forms a primary structure around the template material; b) transforming the template material; c) removing the transformed template material, and d) obtaining carrier particles with secondary internal structures.

It was surprisingly found that carrier particles exhibit the desired drug delivery properties when produced with a template material that undergoes a transformation as described herein. The means and methods provided herein are thus useful in the medical care of patients, especially (but not limited to) pediatric and geriatric patients. The improved means and methods provided herein allow obtaining solid pharmaceutical compositions with enhanced drug delivery properties including, without limitation, effectiveness, safety, pharmacokinetic properties, physical stability, chemical stability, drug loading capacity and/or disintegration time.

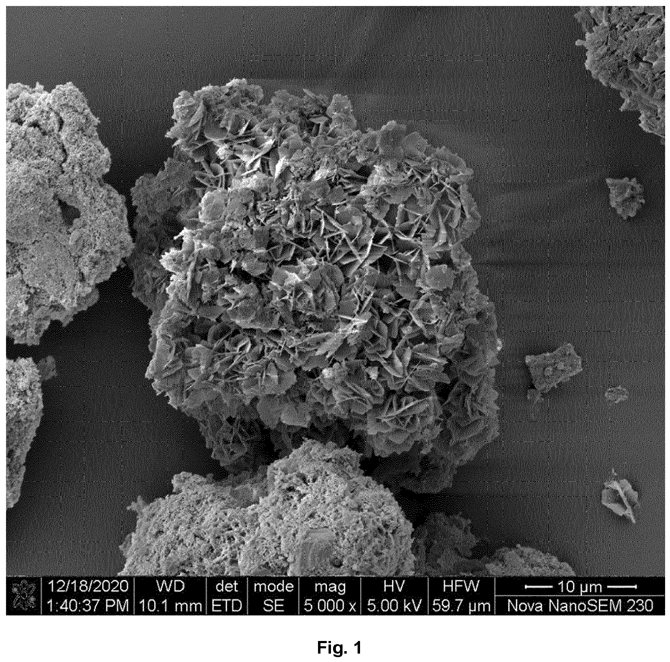

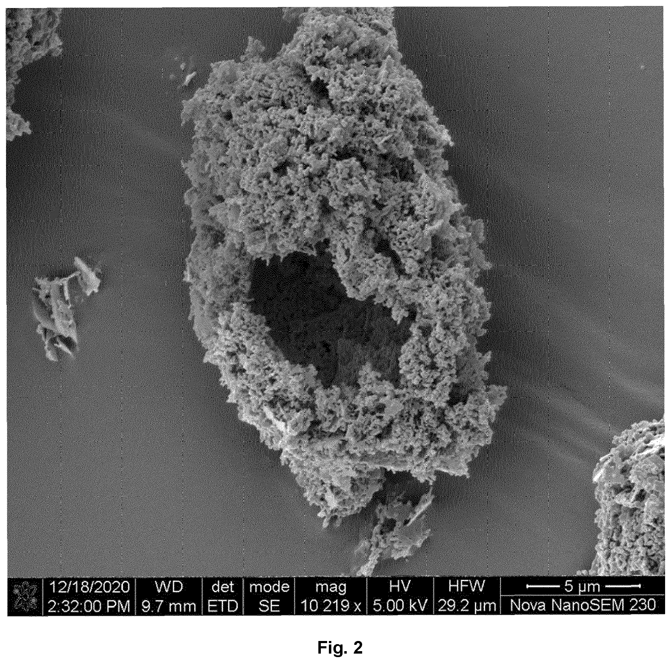

The term "carrier particle", as used herein, refers to a material that is nontoxic or not substantially toxic to a subject, which can be used to improve a desired drug delivery property of a solid pharmaceutical composition. The carrier particle described herein has no or no substantial therapeutic effect upon administration to a subject unless it is loaded with a therapeutic agent. In some embodiments, the carrier particle described herein is pharmacologically inert unless it is loaded with a therapeutic agent. In some embodiments, the carrier particle described herein does not or not substantially dissolve in water. The desired drug delivery properties described herein of the solid pharmaceutical composition include, without limitation, effectiveness, safety, pharmacokinetic properties (e.g., bioavailability), physical stability, chemical stability, drug loading capacity, and/or disintegration time. In some embodiments, the desired drug delivery properties of a solid pharmaceutical composition are physical stability, drug loading capacity, and disintegration time. In some embodiments, the desired drug delivery properties of a solid pharmaceutical composition are high drug loading capacity of the solid pharmaceutical composition (e.g., the drug loading capacity of v/v $\geq$50%, $\geq$55%, $\geq$60%, $\geq$65%, $\geq$70%, $\geq$75%, $\geq$80%, preferably $\geq$60%, more preferably between 60%, and 85%), low disintegration time of the solid pharmaceutical composition (e.g., $\leq$15s, $\leq$14s, $\leq$13s, $\leq$12s, $\leq$11s, $\leq$10s, preferably $\leq$10s) and/or physical stability (e.g., tablet hardness of $\geq$200N, $\geq$210N $\geq$220N, $\geq$230N, $\geq$240N, or $\geq$250N, for an 11mm tablet or $\geq$40N, $\geq$50N, $\geq$60N for a 6mm tablet, preferably $\geq$50N for an 6mm tablet (see, e.g., Example 4)). A carrier particle according to the invention can have any shape, preferably a carrier particle according to the invention has a shape similar to that of a sphere, a spheroid, and/or a bead (see, e.g., Figure 1). Removal of the template material can result in at least one pore in the otherwise largely uniform structure (see, e.g., Figure 2). The carrier particle preferably can form a hollow structure in a dry environment. As such, the carrier particle described herein does not or not substantially collapse upon drying.

The term "primary structure" as used herein, refers to the layer of a carrier material that encompasses the template material. In some embodiments, the primary structure comprises further structure elements (e.g., petals as in Figure 1) that increase the surface area of the carrier particle.

The term "secondary internal structure", as used herein, refers to a hollow internal structure (see, e.g., Figure 2), wherein the internal surface of the hollow internal structure is dense in crystallization initiation points. Therefore, the secondary internal structure enables crystallization inside the carrier particle.

The term "carrier material", as used herein, refers to a material or a mixture that comprises the raw material for the carrier particle of the invention. In some embodiments, the carrier material described herein is an inorganic salt or comprises an inorganic salt to a substantial degree. In some embodiments, the carrier material described herein is insoluble or poorly soluble in water. In some embodiments, the carrier material is dissolved in a solvent. In some embodiments, the carrier material or a precursor of the carrier material is a liquid. In some embodiments, the carrier material described herein is a non-polymer or comprises a non-polymer to a substantial degree.

The term "template material", as used herein, refers to a solid material comprising particles suitable to serve as a template to enable the formation of the primary structure of the carrier particles. The particles in the template material preferably have the shape of a sphere, a spheroid, and/or a bead. In some embodiments, the template material described herein is a non-polymer or comprises a non-polymer to a substantial degree. In some embodiments, the template material described herein has a uniform or almost uniform particle size distribution. In some embodiments, the template material described herein has a distribution width (as defined by the formula: (D90 - D10)/D50)) of about $\leq$5, about $\leq$4.5, about $\leq$4, about $\leq$3.5, about $\leq$3, about $\leq$2.8, about $\leq$2.4, about $\leq$2, about $\leq$1.8, about $\leq$1.6, about $\leq$1.4, about $\leq$1.2, about $\leq$1, about $\leq$0.9, about $\leq$0.8, about $\leq$0.7, about $\leq$0.6, about $\leq$0.5, about $\leq$0.4, about $\leq$0.3, about $\leq$0.2, or about $\leq$0.1. As such the template material is any material that is transformable and has sufficient stability to hold the carrier material. To avoid the dissolution of the template material during the step of combining a carrier material with a template material, a template material poorly soluble in a combining liquid should be used. In some embodiments, the template material described herein, is poorly soluble in at least one organic solvent selected from the group of dichloromethane, diethyl ether, toluene, ethanol, methanol, dimethyl sulfoxide, supercritical $CO_2$, dimethyl ketone, 2-propanol, 1-propanol, saturated alkanes, alkenes, alkadienes, fatty acids, glycerol, silicon oils, gamma-Butyrolactone, and tetrahydrofuran. In some embodiments, the template material described herein, is poorly soluble in water. In some embodiments, the template material described herein, is poorly soluble in an aqueous solution comprising solubility altering agents (e.g. salt water). In some embodiments, the term "poorly soluble" as described herein refers to a solubility at 25°C of about <100mg/L, <80mg/L, <60mg/L, <40mg/L, <20mg/L, <10mg/L, <9mg/L, <8mg/L, <7mg/L, <6mg/L, <5mg/L, <4mg/L, <3mg/L, <2mg/L, <1mg/L, <0.9mg/L, <0.8mg/L, <0.7mg/L, <0.6mg/L, <0.5mg/L, <0.4mg/L, <0.3mg/L, <0.2mg/L, <100$\mu$g/L, <90$\mu$g/L, <80pg/L, <70$\mu$g/L, <60$\mu$g/L, <50$\mu$g/L, <40$\mu$g/L, <30$\mu$g/L, <25$\mu$g/L or <20$\mu$g/L.

In some embodiments, the template material described herein comprises a salt. In some embodiments, the template material described herein comprises an organic salt. In some embodiments, the template material described herein is a carbonate salt or comprises a carbonate salt to a substantial degree. In some embodiments, the template material described herein comprises a basic oxide.

The term "transforming", as used herein, refers to changing the properties of the template material by at least one physical step and at least one chemical step that in combination enable removal of the template material. The physical step of "transforming" comprises providing energy to the material. In some embodiments, the energy is applied in form of a rise in temperature, and/or alteration of pressure. In some embodiments, the physical step of "transforming" induces an endothermic chemical reaction in the template material. The chemical step of "transforming" comprises providing a chemical reactant to the template material. In some embodiments, the reactant provided in the chemical step of "transforming" reacts with the template material but not or not substantially with the carrier material. In some embodiments, the chemical reactant provided in the chemical step of "transforming" is provided in liquid, dissolved, and/or gaseous form.

The method of the invention enables the production of carrier particles with secondary internal structures. In some embodiments, these secondary internal structures enable high drug loading, because, without being bound by theory, the carrier particles can be loaded with the drug inside the secondary internal structures and not only on the surface of the carrier particles. The loaded agent or drug can leave the carrier by diffusion through the porous carrier wall. In some embodiments, the method of the invention enables the production of carrier particles that have certain stability at a target site (e.g., on the mucosa of a patient). Therefore, these carrier particles can remain at a target site (e.g., by adhesion to the mucosa) and enable specific drug delivery. In some embodiments, the method of the invention enables the production of carrier particles that mask the unpleasant taste of a loaded agent, because the loaded agent is continuously released at the site of absorption. The release rate of the loaded agent can be controlled by geometry of the template material and/or by diffusion rate modifiers such as disintegrants. Therefore, the unpleasant taste diffuses to a lesser extent to the locations of perceptions (e.g., the tongue).

The secondary internal structure described herein enables efficient drug loading on the inside of the carrier particle. Further, the secondary internal structure is accessible via pores e.g., for loading solvents. In some embodiments, the method of the invention enables the production of a carrier particle that can be loaded with less effort and/or has a particularly high loading capacity.

In some embodiments, the method of the invention enables the production of a carrier particle that has a particularly large surface area that is beneficial for interparticle forces. These interparticle forces act between the carrier particles in absence of water and increase the mechanical stability of carrier particle clusters. This increased mechanical stability reduces the need for additional stabilization material in the use of the carrier particles in pharmaceutical compositions such as solid pharmaceutical compositions, e.g., tablets. In some embodiments, the interparticle forces acting between carrier particles produced according to the method of the invention can be diminished by water enabling a low disintegration time of pharmaceutical compositions such as solid pharmaceutical compositions, e.g., tablets, comprising the carrier particle according to the invention.

Accordingly, the invention is at least in part based on the surprising finding that the method of the invention enables the production of carrier particles with secondary internal structures that are beneficial to enhance one or more desired drug delivery properties.

In certain embodiments, the invention relates to the method according to the invention, wherein the template material is an inorganic material or consists primarily of inorganic material.

The term "consists primarily of", as used herein, in the context of a material refers to consisting of at least 50%, at least 60%, at least 70%, at least 80%, at least 90%, at least 95%, at least 96%, at least 97%, at least 98% or at least 99% of the material.

In certain embodiments, the invention relates to the method according to the invention, wherein the carrier material is an inorganic material or consists primarily of inorganic material.

In certain embodiments, the invention relates to the method according to the invention, wherein the carrier material and the template material are inorganic salts or consist primarily of inorganic salts.

The inventors found that inorganic materials/salts enable the production of stable non-toxic carrier particles with secondary internal structures of a particular desired size that are beneficial to enhance one or more desired drug delivery properties.

Accordingly, the invention is at least in part based on the surprising finding that the method of the invention enables the production of carrier particles with secondary internal structures that are beneficial to enhance one or more desired drug delivery properties.

In certain embodiments, the invention relates to the method according to the invention, wherein the template material is suspended in a liquid before combining a carrier material with a template material.

The template material can be suspended in a combining liquid (e.g., water) under stirring in a reaction vessel (e.g., as described in Example 1). The set agitation speed ensures stable turbulent mixing to impede particle agglomeration, which enables the treatment of the particles individually.

In certain embodiments, the invention relates to the method according to the invention, wherein combining a carrier material with a template material comprises adding the template material described herein and the carrier material described herein to a combining liquid. In some embodiments, the combining liquid described herein is at least one organic solvent selected from the group of dichloromethane, diethyl ether, toluene, ethanol, methanol, dimethyl sulfoxide,

supercritical $CO_2$, dimethyl ketone, 2-propanol, 1-propanol, saturated alkanes, alkenes, alkadienes, fatty acids, glycerol, silicon oils, gamma-Butyrolactone, and tetrahydrofuran. In some embodiments, the combining liquid described herein is water. In some embodiments, the combining liquid described herein is an aqueous solution comprising solubility altering agents (e.g. salt water).

To avoid dissolution of the template material during the step of combining a carrier material with a template material, an appropriate ratio of the amount of template material compared to the amount of the combining liquid should be used. This appropriate ratio depends on the solubility of the template material in the combining liquid. In some embodiments amount of the template material and combining liquid is chosen such that less than about 0.05%(w/w), less than about 0.04%(w/w), less than about 0.03%(w/w), less than about 0.02%(w/w), less than about 0.01 %(w/w), less than about 0.0095%(w/w), less than about 0.009%(w/w), less than about 0.0085%(w/w), less than about 0.0008%(w/w), less than about 0.0075%(w/w), less than about 0.007%(w/w), less than about 0.0065%(w/w), less than about 0.06%(w/w), less than about 0.0055%(w/w), or less than about 0.005%(w/w) of the template material are dissolved in the combining liquid.

In certain embodiments, the invention relates to the method according to the invention, wherein combining a carrier material with a template material comprises chemical precipitation, layering, and/or crystallization of the carrier material on the template material.

The term "chemical precipitation", as used herein, refers to the process of conversion of a chemical substance from a solution into a solid by converting the substance into an insoluble form.

In certain embodiments, the invention relates to the method according to the invention, wherein combining a precursor of the carrier material forms the carrier material in a chemical reaction with the surface of the template material. In some embodiments, the soluble precursor of the carrier material described herein is phosphoric acid.

The inventors found that the conversion grade is relevant in embodiments wherein combining a precursor of the carrier material forms the carrier material in a chemical reaction with the surface of the template material.

Further the inventors found that a too low conversion grade can cause particles with holes or broken shells, whereas a too high conversion can reduce the size of the inner cavity and produces more external crystals for example of dicalcium phosphate, which further converts to hydroxyapatite slabs.

In some embodiments, the conversion grade described herein is between about 30% and about 60%, between about 35% and 55%, or between about 40% and about 50%.

The temperature during the chemical precipitation described herein can have a substantial influence on the material.

For example, dicalcium phosphate as it is a less thermodynamically stable form than the hydroxyapatite. Therefore, too low temperatures and fast or uncontrolled orthophosphoric acid addition to calcium carbonate will trigger its precipitation and yield more dicalcium phosphate resulting in separate crystals that are more difficult to process.

In some embodiments, the temperature during the chemical precipitation is about 60°C or higher, preferably between about 60°C and about 100°C, more preferably between about 70°C and about 95°C, more preferably between about 80°C and about 95°C.

In certain embodiments, the invention relates to the method according to the invention, wherein a soluble precursor of the carrier material is added in a solution to the template material and distributed on the template material by the addition of a reactant that converts the soluble precursor of the carrier material to the insoluble carrier material. In some embodiments, the soluble precursor of the carrier material described herein is sodium phosphate or calcium chloride (e.g., as Despotovic, R., et al., 1975, Calc. Tis Res. 18, 13-26).

The term "layering", as used herein, refers to a technique for adding at least one layer of the carrier on the template material.

Any layering technique known in the art may be used (see, e.g., Decher, G. H. J. D., et al., 1992, Thin solid films, 210, 831-835; Donath, E., et al., 1998, Angewandte Chemie International Edition, 37(16), 2201-2205; Caruso, F, et al., 1998, Science, 282(5391), 1111-1114). In some embodiments, electrostatic interactions (e.g., as described in Decher, G. H. J. D., et al., 1992, Thin solid films, 210, 831-835), hydrogen bonding (e.g., as described in Such, G. K. et al., 2010, Chemical Society Reviews, 40(1), 19-29), hydrophobic interactions (e.g., as described in Serizawa, T., Kamimura, S., et al., 2002, Langmuir, 18(22), 8381-8385), and/or covalent coupling (e.g., as described in Zhang, Y., et al., 2003, Macromolecules, 36(11), 4238-4240), electroplating and electrodeposition (e.g., as described in Chandran, R., Panda, S.K. & Mallik, A. A short review on the advancements in electroplating of CuInGaSe2 thin films. Mater Renew Sustain Energy 7, 6 (2018)) are exploited to prepare at least one layer on the template material, particularly to prepare multilayered films on the template material.

The term "crystallization", as used herein, refers to the process of conversion of a chemical substance from a super-saturated solution.

In certain embodiments, the invention relates to the method according to the invention, wherein the carrier material is added in a super-saturated solution to the template material and distributed on the template material by the initiation of chemical precipitation.

In certain embodiments, the invention relates to the method according to the invention, wherein combining a carrier material with a template material comprises chemical precipitation and crystallization of the carrier material on the template material.

In certain embodiments, the invention relates to the method according to the invention, wherein combining a carrier material with a template material comprises chemical layering and crystallization of the carrier material on the template material.

In certain embodiments, the invention relates to the method according to the invention, wherein combining a carrier material with a template material comprises chemical precipitation and layering of the carrier material on the template material.

The chemical precipitation process can be carried out by pumping a solution of a precursor of the template material onto the carrier material or into the liquid comprising the carrier material (e.g., as described in Example 1). During this process, the carrier material can start growing (e.g., in the form of a crystalline lamellae structure) on the surface of template material and thus forming the stratum layer. In certain embodiments, the template material as described herein is converted to the carrier material. In certain embodiments, the template material as described herein is converted to at least about 20%, about 30%, about 40%, about 50%, about 60%, or about 70% to the carrier material.

Chemical precipitation, layering, and/or crystallization enable fine and/or uniform distribution of the carrier material on the template material. This fine and/or uniform distribution affects the formation of the secondary internal structures.

Accordingly, the invention is at least in part based on the surprising finding that the method of the invention enables the production of carrier particles with particularly fine and/or uniform secondary internal structures by using chemical precipitation, layering, and/or crystallization of the carrier material on the template material.

In certain embodiments, the invention relates to the method according to the invention wherein transforming the template material comprises heating to a temperature from about 600 °C to about 1200 °C, preferably about 600 to about 900°C, preferably about 600"C to 839°C, preferably about 650°C to about 700°C.

In certain embodiments, the invention relates to the method according to the invention wherein transforming the template material comprises heating to a temperature from 840 °C to 1200 °C.

The conditions can be optimized to avoid interparticle condensation during the heating step, which can result in redispersability problems. While in some embodiments no further agents to avoid interparticle condensation need to be added (see e.g., Example 1), in other embodiments agents to avoid interparticle condensation (e.g., anti-sintering agents) are added during and/or before the heating step described herein. Such anti-sintering agents are described for example in Okada, M., et al., 2014, Journal of nanoparticle research, 16(7), 1-9.

The transformation of the template material described herein can be done at any suitable temperature or any suitable temperature range. To enable the transformation of the template material described herein the minimal suitable temperature for transformation is set at a certain temperature e.g., about 210°C (e.g., for silver and gold carbonate as the template material), about 840°C (e.g., for calcium carbonate as the template material), about 900°C, about 1000°C, or about 1200°C (e.g., for potassium and/or sodium carbonates as template material). The person skilled in the art can identify the appropriate minimal suitable temperature from the decomposition temperature of the template material. An increased temperature can shorten the transformation time, however, melting of the carrier material may have an undesired effect on the carrier particles such as incomplete carrier particle formation or reduced carrier particle hardness. To avoid melting of the carrier material, the maximal suitable temperature for the transformation of the template material described herein is set below the melting temperature of the carrier material. Deformation and/or loss of desired structures (e.g., petals on the surface of the carrier particles, see e.g., Figure 1) that enhance the surface area of the carrier particles can already occur at temperatures below the melting temperature of the carrier material. Accordingly, in certain embodiments, the maximal suitable temperature for the transformation of the template material described herein is set about 100°C, about 200°C, about 400°C, about 500°C, or about 600°C below the melting temperature of the carrier material.

In certain embodiments, the invention relates to the method according to the invention wherein transforming the template material comprises heating to a temperature from about the decomposition temperature of the template material to about the melting temperature of the carrier material, preferably from about the decomposition temperature of the template material to about 400°C below the melting temperature of the carrier material, more preferably about the decomposition temperature of the template material to about 500°C below the melting temperature of the carrier material.

In certain embodiments, the invention relates to the method according to the invention wherein transforming the template material comprises heating to a temperature from 840°C to 1600°C, preferably from 840°C to 1200°C, more preferably around 1100°C.

The duration of the heating for transforming the template material described herein depends on various factors such as the template material, the carrier material, the temperature range, particle size, and/or the desired carrier particle surface area.

The duration of the heating for transforming the template material described herein may for example be about 1 hour as described in Example 1. In certain embodiments, the duration of the heating for transforming the template material described herein is between about 5 min and about 24 h, about 10 min and about 12 h, 20 min and about 4 h.

The heating for transforming the template material described herein (e.g., to a temperature in a certain range, e.g., between 840 °C to 1200 °C or 600"C to 900°C) can be achieved by any heating pattern such as a linear increase of temperature or with one or more preheating steps. The preheating steps described herein may comprise keeping the temperature at a certain temperature level for a certain time before heating the template material to a temperature in a certain range, e.g., from 840 °C to 1200 °C or 600°C to 900°C. Preheating allows for example removal of undesired volatile components such as solvents.

In some embodiments, the pressure is reduced during the heating for transforming the template material to a temperature in a certain range, e.g., from 840 °C to 1200 °C.

In some embodiments, the pressure is increased during the heating for transforming the template material to a temperature in a certain range, e.g., from 840 °C to 1200 °C.

In some embodiments, the heating for transforming the template material induces an endothermic chemical reaction.

In some embodiments, an inert substance (e.g., noble gas) is supplied to avoid side reactions during the heating for the transforming the template material to a temperature in a certain range, e.g., from 840 °C to 1200 °C.

In some embodiments, the heating for transforming the template material induces the evaporation of volatile fractions of the template material.

The heating to a temperature in a certain range, e.g., from 840 °C to 1200 °C, may initiate the transformation of the template material but does not or not to the same extent alter the carrier material. This enables the removal of the transformed template material based on the altered properties. Lower temperature (e.g. about 600°C to about 839°C or 600°C to about 900°C) can be used to maintain the petals' structure to a larger degree, which can increase the resulting tablet hardness.

In case the temperatures are higher than the recommended range, the fine petal structure of the particles is molten and is reduced, the flexibility of the petals is reduced; therefore, the hardness of the tablets produced with such overheated material is strongly reduced. Pharmaceutical compacts made with overheated material show capping and lamination and cannot be used comparably well in pharmaceutical formulations.

Accordingly, the invention is at least in part based on the surprising finding that the method of the invention, wherein the method of the invention comprises a heating step for the transformation of the template material enables the production of carrier particles with secondary internal structures that are beneficial to enhance one or more desired drug delivery properties.

In certain embodiments, the invention relates to the method according to the invention, wherein the step of transforming the template material comprises calcination.

The term "calcination", as used herein, refers to heating a solid or a mixture comprising a solid to high temperatures (e.g., a temperature from 840 °C to 1200 °C or 600°C to 900°C) under the supply of air or oxygen to the solid or the mixture.

In some embodiments, the calcination according to the invention induces decomposition of template material comprising a carbonate (e.g., carbonate salts such as calcium carbonate) to carbon dioxide.

In some embodiments, the calcination according to the invention induces decomposition of template material comprising a metallic carbonate to a metallic oxide, preferably to a basic oxide.

In some embodiments, the calcination according to the invention induces the decomposition of hydrated template material by the removal of water.

In some embodiments, the calcination according to the invention induces the decomposition of volatile matter in the template material.

Accordingly, the invention is at least in part based on the surprising finding that the method of the invention, wherein the method of the invention comprises a calcination step for the transformation of the template material enables the production of carrier particles with secondary internal structures that are beneficial to enhance one or more desired drug delivery properties.

In certain embodiments, the invention relates to the method according to the invention, wherein the step of transforming the template material comprises a subsequent addition of water.

The subsequent addition of water according to the invention transforms the template material in a chemical reaction but does not alter or unsubstantially alter the carrier material. This enables the removal of the transformed template material based on the altered properties.

In some embodiments, the subsequent addition of water according to the invention reacts with a metallic oxide.

Accordingly, the invention is at least in part based on the surprising finding that the method of the invention, wherein the transformation step method of the invention comprises the addition of water enables the production of carrier particles with secondary internal structures that are beneficial to enhance one or more desired drug delivery properties.

In certain embodiments, the invention relates to the method according to the invention wherein the addition of water enables an exothermic reaction.

The term "exothermic reaction", as used herein, refers to a reaction for which the overall standard enthalpy change is negative.

The subsequent addition of water according to the invention transforms the template material in an exothermic chemical reaction but does not alter or unsubstantially alter the carrier material. This enables the removal of the transformed template material based on the altered properties.

The basic oxide described herein, is not toxic or unsubstantially toxic at the dose used in the context of the invention. In some embodiments, the subsequent addition of water according to the invention reacts with a basic oxide. In some embodiments, the subsequent addition of water according to the invention reacts with at least one basic oxide selected from the group of lithium oxide, sodium oxide, potassium oxide, rubidium oxide, cesium oxide, magnesium oxide, calcium oxide, strontium oxide, barium oxide, and bismuth(III) oxide. In some embodiments, the subsequent addition of water according to the invention reacts with magnesium oxide and/or calcium oxide.

The exothermic reaction as described herein can facilitate subsequent removal of the template material. The forces released during the exothermic reaction and/or the properties of the products of the exothermic reaction can decrease density and/or increase solubility. For example, the exothermic reaction of calcium oxide with a density of $3.34 g/cm^3$ with water results in calcium hydroxide with a density of $2.21 g/cm^3$.

Accordingly, the invention is at least in part based on the surprising finding that the method according to the invention wherein the addition of water through an exothermic reaction supports the secondary structure formation and facilitates subsequent template material removal.

In certain embodiments, the invention relates to the method according to the invention, wherein removing the template material comprises dissolution of the transformed template material to form secondary internal structures.

The secondary internal structures can be formed by the removal of the transformed template material by dissolution in a solvent that dissolved the transformed template material but not the carrier material.

In some embodiments, removing the template material comprises dissolution of the transformed template material with water or an aqueous solution. In some embodiments, the pH of the aqueous solution is altered before the dissolution of the transformed template material to increase the solubility of the transformed template material or decrease the solubility of the carrier material in the aqueous solution.

In some embodiments, removing the template material comprises the dissolution of the transformed template with an organic solvent.

The removal of the template material by dissolution is particularly mild to the carrier material. Therefore, this mild removal supports the maintenance of the primary carrier material structure and enables the formation of secondary internal structures that are particularly beneficial for crystallization during the drug loading process.

Accordingly, the invention is at least in part based on the surprising finding that the method according to the invention, wherein removing the template material comprises dissolution of the transformed template material supports the formation of the secondary internal structures.

In certain embodiments, the template material of the invention comprises a metal carbonate.

In certain embodiments, the template material of the invention comprises at least one metal carbonate selected from the group of $Li_2CO_3$, $LiHCO_3$, $Na_2CO_3$, $NaHCO_3$, $Na_3H(CO_3)_2$, $MgCO_3$, $Mg(HCO_3)_2$, $Al_2(CO_3)_3$, $K_2CO_3$, $KHCO_3$, $CaCO_3$, $Ca(HCO_3)_2$, $MnCO_3$, $FeCO_3$,$NiCO_3$, $Cu_2CO_3$, $CuCO_3$, $ZnCO_3$, $Rb_2CO_3$, $PdCO_3$, $Ag_2CO_3$, $Cs_2CO_3$, $CsHC0_3$, $BaCO_3$, and $(BiO)_2CO_3$.

In certain embodiments, the template material of the invention comprises at least one metal selected from the group of Fe, Mg, Al, Mn, V, Ti, Cu, Ga, Ge, Ag, Au, Sm, U, Zn, Pt and Sn. In certain embodiments, the template material of the invention comprises at least one non-metal selected from the group of Si, S, Sb, I, and C.

In certain embodiments, the template material of the invention comprises more than 50%, 60%, 70%, 80%, 90%, 95%, 96%, 97%, 98% or 99% metal carbonate.

In certain embodiments, the template material of the invention comprises more than 50%, 60%, 70%, 80%, 90%, 95%, 96%, 97%, 98% or 99% of at least one metal carbonate selected from the group of $Li_2CO_3$, $LiHCO_3$, $Na_2CO_3$, $NaHCO_3$, $Na_3H(CO_3)_2$, $MgCO_3$, $Mg(HCO_3)_2$, $Al_2(CO_3)_3$, $K_2CO_3$, $KHCO_3$, $CaCO_3$, $Ca(HCO_3)_2$, $MnCO_3$, $FeCO_3$,$NiCO_3$, $Cu_2CO_3$, $CuCO_3$, $ZnCO_3$, $Rb_2CO_3$, $PdCO_3$, $Ag_2CO_3$, $Cs_2CO_3$, $CsHCO_3$, $BaCO_3$, and $(BiO)_2CO_3$.

In certain embodiments, the template material of the invention comprises more than 50%, 60%, 70%, 80%, 90%, 95%, 96%, 97%, 98% or 99% magnesium carbonate.

In certain embodiments, the invention relates to the method according to the invention, wherein the template material comprises calcium carbonate.

In certain embodiments, the template material of the invention comprises more than 50%, 60%, 70%, 80%, 90%, 95%, 96%, 97%, 98% or 99% calcium carbonate.

In some embodiments, the calcium carbonate as described herein comprises anhydrous calcium carbonate, complexes comprising calcium carbonate and/or hydrated calcium carbonate such as $CaCO_3 \cdot H_2O$ and/or calcium carbonate hexahydrate.

In some embodiments, the calcium carbonate as described herein is anhydrous calcium carbonate.

The metal carbonates described herein can be used as a basis to produce a carrier material with distinct properties (e.g., an insoluble metal phosphate by a reaction of the metal carbonate with $H_3PO_4$) on the surface of the template material and can be transformed as described herein.

Accordingly, the invention is at least in part based on the surprising finding that the method of the invention is particularly efficient when the template material comprises a metal carbonate such as calcium carbonate.

In certain embodiments, the invention relates to the method according to the invention, wherein the carrier material comprises at least one salt and/or complex selected from the group of calcium phosphate and magnesium phosphate.

In certain embodiments, the invention relates to the method according to the invention, wherein the carrier material comprises at least one salt and/or complex of magnesium phosphate.

In certain embodiments, the invention relates to the method according to the invention, wherein the carrier material comprises at least one salt and/or complex of calcium phosphate.

Calcium phosphate and magnesium phosphate have a particularly low solubility in water and show a reasonable heat resistance. Furthermore, calcium phosphate and magnesium phosphate are typically pharmacologically inert and non-toxic. Therefore, calcium phosphate and magnesium phosphate are robust, non-toxic, and allow the transformation of the template material as described herein without decomposition.

Accordingly, the invention is at least in part based on the surprising finding that the method of the invention is particularly efficient when the carrier material comprises at least one salt and/or complex selected from the group of calcium phosphate and magnesium phosphate.

The template material can have various structures, e.g., powder (e.g., a powder with D50 of about: 1.9$\mu$m, 2.3$\mu$m, 3.2$\mu$m, 4.5$\mu$m, 5.5$\mu$m, 6.5$\mu$mo or 14$\mu$m; a powder with a particle size range of about: 1 to 100 $\mu$m, 100$\mu$m to 300$\mu$m or 300$\mu$m to 600$\mu$m) or nanoparticles.

In certain embodiments, the invention relates to the method according to the invention, wherein the template material comprises particles that have a diameter of 1 to 300 $\mu$m.

In certain embodiments, the invention relates to the method according to the invention, wherein the template material consists of particles wherein about 50%, about 60%, about 70%, about 80%, about 90%, about 95%, about 98%, about 99% of the particles that have a diameter of 1 to 300 $\mu$m. In certain embodiments, the invention relates to the according to the invention, wherein the template material comprises particles that have a median diameter of about 1 to 300 $\mu$m, about 1 to 250 $\mu$m, about 1 to 200 $\mu$m, about 1 to 150 $\mu$m, about 1 to 100 $\mu$m, about 1 to 90 $\mu$m, about 1 to 80 $\mu$m, about 1 to 70 $\mu$m, about 1 to 60 $\mu$m, about 1 to 50 $\mu$m, about 1 to 40 $\mu$m, about 1 to 30 $\mu$m or about 1 to 20 $\mu$m.

The particle size of the template material influences the diameter of the carrier particle. In certain embodiments, the invention relates to the according to the invention, wherein the particles of the template material have about the same median diameter as the median diameter of the carrier particles. In embodiments wherein the template material and the carrier material are combined by layering and/or crystallization as described herein, the carrier particle has a similar or larger median diameter compared to the template material.

In embodiments wherein the template material and the carrier material are combined by chemical precipitation as described herein, the carrier particle has a similar or smaller median diameter compared to the template material.

The person skilled in the art can predict the carrier material from the template material, carrier material, and the techniques used for combining the template material with the carrier material as described herein.

In certain embodiments, the invention relates to the according to the invention, wherein the carrier particles have a diameter of 1 to 300 $\mu$m.

Apart from adapting the parameters and materials used in the method of the invention, particles of a certain size can be obtained by methods known in the art, including milling, sieving (see, e.g., Patel, R. P., et al., 2014, Asian Journal of Pharmaceutics (AJP), 2(4); DAVID, J., and PETER, R., 2006, Fundamentals of Early Clinical Drug Development: From Synthesis Design to Formulation, 247; US5376347A). Particle size and shape measurements can be made using any method known in the art, such as laser diffraction or in situ microscopy (Kempkes, M., Eggers, J., & Mazzotti, M., 2008, Chemical Engineering Science, 63(19), 4656-4675; Allen, T. (2013). Particle size measurement. Springer).

In some applications, a particular low carrier particle diameter is desired. In certain embodiments, the invention relates to the method according to the invention, wherein the carrier particles have a diameter of about 1 to 20 $\mu$m, about 1 to 15 $\mu$m, about 1 to 10 $\mu$m, or about 1 to 5 $\mu$m for use in intrapulmonary administration and/or nasal administration. In some applications, a particular low carrier particle diameter is desired to increase the diffusion surface and accelerate the release of the loaded agent.

In some applications, a larger carrier particle diameter is desired to enhance the flowability of the carrier particles and to facilitate further processing. In certain embodiments, the invention relates to the method according to the invention, wherein the carrier particles have a diameter of about 5 to 300 $\mu$m, about 10 to 250 $\mu$m, about 15 to 200 $\mu$m, or about 20 to 150 $\mu$m.

Accordingly, the invention is at least in part based on the surprising finding that the method of the invention wherein the carrier particles have a diameter in a certain range can be particularly useful for further processing (e.g.,

flowability) and/or application (e.g., diffusion surface) of the carrier particle produced according to the method of the invention.

In certain embodiments, the invention relates to the method according to the invention, wherein the carrier particles have a surface area between 15m2/g to 400 m2/g or 30m2/g to 400m2/g.

In certain embodiments, the invention relates to the method according to the invention, wherein the carrier particles have a surface area between about 15m2/g to 400 m2/g about 30m2/g to 400m2/g, about 50m2/g to 350m2/g, about 70m2/g to 320m2/g, about 90m2/g to 300m2/g or about 100m2/g to 280m2/g as measured by 5-point BET (Brunnauer-Emmet-Teller) surface area analysis with nitrogen as a gas.

Alternatively, the surface area of carrier particles can be measured by any method known in the art (see, e.g., Akashkina, L.V., Ezerskii, M.L., 2000, Pharm Chem J 34, 324-326; Bauer, J. F., 2009, Journal of Validation Technology, 15(1), 37-45).

The surface area of the carrier particles can be altered e.g., by the particle size of the carrier material, the carrier material, and/or changing the surface structure by the parameters used in the method of the invention (e.g., heat, duration of heating).

In certain embodiments, the invention relates to the carrier particle according to the invention, wherein the carrier particle is used as an adsorber.

A greater specific surface of carrier particles described herein allows strong Van der Waals interactions once the particles are brought in contact. This effect results in higher tensile strength of the final dosage forms. These Van der Waals interactions can be diminished by the addition of water and support the disintegration of particle clusters.

Accordingly, the invention is at least in part based on the surprising finding that the method of the invention enables mechanical stability and disintegration capabilities if the carrier particles have a surface area between 15m2/g to 400 m2/g, preferably 30m2/g to 400m2/g.

In certain embodiments, the invention relates to the method according to the invention, wherein the secondary internal structure comprises pores having a diameter size in the range of $\geq 0.2\ \mu$m and $\leq 1.5\ \mu$m.

In certain embodiments, the invention relates to the method according to the invention, wherein the secondary internal structure comprises pores having a diameter size of about $\geq 0.2\ \mu$m, about $\geq 0.3\ \mu$m, about $\geq 0.4\ \mu$m, about $\geq 0.5\ \mu$m, about $\geq 0.6\ \mu$m, about $\geq 0.7\ \mu$m, about $\geq 0.8\ \mu$m, about $\geq 0.9\ \mu$m, about $\geq 1\ \mu$m, about $\geq 1.1\ \mu$m, about $\geq 1.2\ \mu$m, about $\geq 1.3\ \mu$m, or about $1.5\ \mu$m.

In certain embodiments, the invention relates to the method according to the invention, wherein the secondary internal structure comprises pores having a diameter size in the range of about $\geq 0.2\ \mu$m to $\leq 1.5\ \mu$m, about $\geq 0.3\ \mu$m to $\leq 1.5\ \mu$m, about $\geq 0.4\ \mu$m to $\leq 1.5\ \mu$m, about $\geq 0.5\ \mu$m to $\leq 1.5\ \mu$m, about $\geq 0.6\ \mu$m to $\leq 1.5\ \mu$m, about $\geq 0.7\ \mu$m to $\leq 1.5\ \mu$m, about $\geq 0.8\ \mu$m to $\leq 1.5\ \mu$m, about $\geq 0.9\ \mu$m to $\leq 1.5\ \mu$m, about $\geq 1\ \mu$m to $\leq 1.5\ \mu$m, about $\geq 1.1\ \mu$m to $\leq 1.5\ \mu$m, about $\geq 1.2\ \mu$m to $\leq 1.5\ \mu$m or about $\geq 1.3\ \mu$m to $\leq 1.5\ \mu$m.

The pore size of carrier particles can be measured by any method known in the art (see, e.g. Markl, D. et al., 2018, International Journal of Pharmaceutics, 538(1-2), 188-214).

The porous structure that can be formed by the method of the invention enables pores of a, particularly, large size. This large pore size facilitates drug loading on the carrier particle and accelerates drug release from the carrier particle.

A pore size diameter greater than 90% of the diameter of the particles of the template material results in unstable carrier particles. Therefore, the maximal pore size depends on the size particles of the template material.

In certain embodiments, the invention relates to the method according to the invention, wherein the secondary internal structure comprises pores having a diameter size of about $\leq 270\ \mu$m, about $\leq 225\ \mu$m, about $\leq 180\ \mu$m, about $\leq 135\ \mu$m, about $\leq 90\ \mu$m, about $\leq 81\ \mu$m, about $\leq 72\ \mu$m, about $\leq 63\ \mu$m, about $\leq 54\ \mu$m, about $\leq 45\ \mu$m, about $\leq 36\ \mu$m, about $\leq 27\ \mu$m, or about $\leq 18\ \mu$m diameter. Accordingly, the invention is at least in part based on the surprising finding that the method of the invention, wherein the secondary internal structure comprises pores that have a certain diameter size is particularly useful for the subsequent drug loading and drug release of the carrier particles produces according to the method of the invention.

In certain embodiments, the invention relates to the method according to the invention, wherein the total volume of the secondary internal structures in the obtained carrier particles with secondary internal structures is in the range of $\geq 10\%$ to $\leq 90\%$ of the particle volume as determined by image analysis of SEM-FIB and SEM of resin-embedded particles' crossection images. Alternative analytical methods to measure the volume ratio of the internal structure and particle include porosity calculation as a ratio of tapped bulk of the carrier material to the true crystalline density of the carrier material.

The total volume of the secondary internal structures refers to the volume inside the particle inside that results from the removal of the template material (See e.g., Figure 2). In certain embodiments, the total volume of the secondary internal structures described herein is the average internal volume of the carrier particles obtained according to the method of the invention.

In certain embodiments, the total volume of the secondary internal structures described herein is the median internal volume of the carrier particles obtained according to the method of the invention.

In certain embodiments, the invention relates to the method according to the invention, wherein the total volume of the secondary internal structures in the obtained carrier particles with secondary internal structures is more than about 10%, about 15%, about 20%, about 25%, about 30%, about 35%, about 40%, about 50%, about 60%, about 70%, or about 80% of the particle volume.

In certain embodiments, the invention relates to the method according to the invention, wherein the total volume of the secondary internal structures in the obtained carrier particles with secondary internal structures is more than about 10%, about 15%, about 20%, about 25%, about 30%, about 35%, about 40%, about 50%, about 60%, about 70%, or about 80% of the particle volume.

In certain embodiments, the invention relates to the method according to the invention, wherein the total volume of the secondary internal structures in the obtained carrier particles with secondary internal structures is in the range of about $\geq 10\% - \leq 90\%$, about $\geq 15\% - \leq 90\%$, about $\geq 20\% - \leq 90\%$, about $\geq 25\% - \leq 90\%$, about $\geq 30\% - \leq 90\%$, about $\geq 35\% - \leq 90\%$, about $\geq 40\% - \leq 90\%$, about $\geq 45\% - \leq 90\%$, about $\geq 50\% - \leq 90\%$, about $\geq 55\% - \leq 90\%$, about $\geq 60\% - \leq 90\%$, about $\geq 65\% - \leq 90\%$, about $\geq 70\% - \leq 90\%$, about $\geq 10\% - \leq 80\%$, about $\geq 15\% - \leq 80\%$, about $\geq 20\% - \leq 80\%$, about $\geq 25\% - \leq 80\%$, about $\geq 30\% - \leq 80\%$, about $\geq 35\% - \leq 80\%$, about $\geq 40\% - \leq 80\%$, about $\geq 45\% - \leq 80\%$, about $\geq 50\% - \leq 80\%$, about $\geq 55\% - \leq 80\%$, about $\geq 60\% - \leq 80\%$, about $\geq 65\% - \leq 80\%$, about $\geq 70\% - \leq 80\%$, about $\geq 10\% - \leq 70\%$, about $\geq 15\% - \leq 70\%$, about $\geq 20\% - \leq 70\%$, about $\geq 25\% - \leq 70\%$, about $\geq 30\% - \leq 70\%$, about $\geq 35\% - \leq 70\%$, about $\geq 40\% - \leq 70\%$, about $\geq 45\% - \leq 70\%$, about $\geq 50\% - \leq 70\%$, about $\geq 55\% - \leq 70\%$, about $\geq 60\% - \leq 70\%$, about $\geq 65\% - \leq 70\%$, about $\geq 10\% - \leq 60\%$, about $\geq 15\% - \leq 60\%$, about $\geq 20\% - \leq 60\%$, about $\geq 25\% - \leq 60\%$, about $\geq 30\% - \leq 60\%$, about $\geq 35\% - \leq 60\%$, about $\geq 40\% - \leq 60\%$, about $\geq 45\% - \leq 60\%$, about $\geq 50\% - \leq 60\%$, about $\geq 55\% - \leq 60\%$, about $\geq 10\% - \leq 50\%$, about $\geq 15\% - \leq 50\%$, about $\geq 20\% - \leq 50\%$, about $\geq 25\% - \leq 50\%$, about $\geq 30\% - \leq 50\%$, about $\geq 35\% - \leq 50\%$, about $\geq 40\% - \leq 50\%$ or about $\geq 45\% - \leq 50\%$ of the particle volume.

In certain embodiments, the invention relates to a loaded carrier particle with secondary internal structures obtainable by the method according to the invention.

In certain embodiments, the invention relates to the loaded carrier particle according to the invention, wherein the carrier particle has a loading capacity of $\geq 72\%$ v/v, $\geq 70\%$ v/v, $\geq 68\%$ v/v, $\geq 66\%$ v/v, $\geq 64\%$ v/v, $\geq 62\%$ v/v, or $\geq 60\%$ v/v.

In certain embodiments, the invention relates to the loaded carrier particle according to the invention, wherein the carrier particle has a loading capacity of $\geq 60\%$ v/v.

The term "loading capacity", as used herein, refers to the volume of the carrier particle that can be used for loading of an agent compared to the volume of the whole carrier particle. Accordingly, a carrier particle with a loading capacity of 60% v/v can load an agent having 60% of the volume of the carrier particle. The volume of the carrier particle is calculated from the diameter of the carrier particle. Therefore, the volume of the internal structure is part of the volume of the carrier particle for this calculation.

In some embodiments, an agent that is loaded on the carrier particle is comprised of a loading solvent and the loading solvent is removed to complete loading.

The agent to be loaded is dissolved in the loading solvent and put in contact with the carrier particle. The loading solvent can be removed by any solvent removal method known to the person skilled in the art. In some embodiments the loading solvent is removed by a method selected from the group of evaporation, vacuum-assisted evaporation, atmospheric drying, vacuum-freeze drying, freeze drying at atmospheric pressure, spray drying, spray drying in fluidized bed apparatus, microwave assisted drying, electrospray-assisted drying, dielectric drying, fluidized-bed assisted drug loading, and solvent-sorption method.

In some embodiments, the solvent-sorption method comprises high shear granulation.

The choice of the appropriate loading solvent depends on solvent toxicity, solvent partial vapor pressure, properties of the agent to be loaded (e.g., pH-stability and/or solubility of the agent to be loaded) and/or properties of the carrier material.

In some embodiments, the loading solvent described herein comprises at least one organic solvent, preferably at least one organic solvent selected from the group of dichloromethane, diethyl ether, toluene, ethanol, methanol, dimethyl sulfoxide, supercritical $CO_2$, dimethyl ketone, 2-propanol, 1-propanol, saturated alkanes, alkenes, alkadienes, fatty acids, glycerol, silicon oils, gamma-Butyrolactone, and tetrahydrofuran. In some embodiments, the loading solvent described herein is water.

Some loading solvents such as water have high surface tension and may therefore require additional measures to support entering the pore(s) of the carrier particle of the invention despite the exceptionally large pore size. In some embodiments, the loading solvent described herein comprises at least one surface-active agent such as a tenside. In some embodiments, the addition of the loading solvent occurs under increased pressure, to support the loading solvent by entering into the inside of the carrier particle.

In some embodiments, loading on and into the carrier particle according to the invention comprises the addition of an antisolvent that reduces the solubility of the agent to be loaded in the loading solvent. In some embodiments, the antisolvent is at least one antisolvent selected from the group of water, dichloromethane, diethyl ether, toluene, ethanol, methanol, dimethyl sulfoxide, supercritical $CO_2$, dimethyl ketone, 2-propanol, 1-propanol, saturated alkanes, alkenes, alkadienes, fatty acids, glycerol, silicon oils, gamma-Butyrolactone, and tetrahydrofuran.

In some embodiments, the loading solvent is removed by evaporation, e.g., by increased temperature and/or decreased pressure. The maximal temperature for the removal of the loading solvent depends on the heat stability of the loaded agent.

In certain embodiments, the invention relates to the carrier particle according to the invention, wherein the carrier particle is used as a placebo.

In certain embodiments, the invention relates to the loaded carrier particle according to the invention, wherein the loaded carrier particle comprises a therapeutic agent.

The term "therapeutic agent", as used herein, refers to a compound or a composition of matter that upon administration to a subject in a therapeutically effective amount, provides a therapeutic benefit to the subject. A therapeutic agent may be any type of drug, medicine, pharmaceutical, hormone, antibiotic, growth factor, and/or bioactive material, used for treating, controlling, or preventing diseases or medical conditions. Those skilled in the art will appreciate that the term "therapeutic agent" is not limited to drugs that have received regulatory approval.

The loaded carrier particle of the invention can enable site-specific therapeutic agent delivery by adhesion to the target site. In certain embodiments, the loaded carrier particle of the invention adheres to a mucosa. In certain embodiments, the loaded carrier particle of the invention adheres to nasal, buccal, sublingual, intrabronchial, vaginal, urethral, or rectal tissue to enable site-specific therapeutic agent delivery.

In certain embodiments, the therapeutic agent described herein is an enzyme that acts locally (e.g., buccal application of lysozyme).

In certain embodiments, the therapeutic agent described herein is a peptide, hormone, and/or small molecule that acts locally (e.g., bronchial corticosteroid application) and/or systemically (e.g., bronchial insulin application). In certain embodiments, the therapeutic agent described herein is an antidiabetic drug such as insulin. Therefore, in certain embodiments, the loaded carrier particle of the invention enables nasal, buccal, sublingual, intrabronchial, vaginal, urethral, or rectal administration of a therapeutic agent that is typically administered by injection or infusion.

The loaded carrier particle of the invention can enable site-specific therapeutic agent delivery and avoid a substantial first-pass effect and/or degradation by location-specific conditions (e.g., stomach acid) and/or enzymes (e.g., digestion enzymes or liver enzymes). In certain embodiments, the therapeutic agent described herein is a therapeutic agent that gets degraded by the GI-tract and/or the liver.

In some embodiments, the therapeutic agent described herein is known by the person skilled in the art to have a therapeutic effect upon oral administration of an effective amount to a subject. In certain embodiments, the therapeutic agent described herein is a small molecule. In some embodiments, the therapeutic agent described herein has a molecular weight of <1600 Da, <1500 Da, <1400 Da, <1300 Da, <1200 Da, <1100 Da, <1000 Da, <900 Da, <800 Da, <700 Da, <600 Da, or <500 Da.

The loaded carrier particle according to the invention, wherein the loaded carrier particle comprises a therapeutic agent can be used for storage of the therapeutic agent, for treatment and/or for an analytical method, or can be further processed.

Accordingly, the invention is at least in part based on the surprising finding that the loaded carrier particle according to the invention, wherein the loaded carrier particle comprises a therapeutic agent can have multipurpose uses.

In certain embodiments, the invention relates to a method for the production of a compacted carrier matter, the production of the compacted carrier matter comprises the production method according to the invention, wherein the method further comprises a step of compacting the carrier particles with secondary internal structures to obtain the compacted carrier matter.

In certain embodiments, the invention relates to a method for the production of a compacted carrier matter, the method comprising the steps of: a) producing a carrier particle according to the invention; and b) compacting the carrier particles with secondary internal structures to obtain the compacted carrier matter.

In certain embodiments, the invention relates to a method for the production of a compacted carrier matter, the method comprising the steps of: a) providing the carrier particle according to the invention; and b) compacting the carrier particles with secondary internal structures to obtain the compacted carrier matter.

In certain embodiments, the invention relates to a method for the production of a compacted carrier matter, the method comprising the steps of: a) producing a carrier particle according to the invention; and providing the carrier particle according to the invention; and b) compacting the carrier particles with secondary internal structures to obtain the compacted carrier matter.

In certain embodiments, the invention relates to a method for the production of a compacted loaded carrier matter, the method comprising the steps of: a) loading a carrier particle according to the invention; and b) compacting the loaded carrier particles with secondary internal structures to obtain the compacted loaded carrier matter.

The term "compacted carrier matter", as used herein, refers to clusters of more than one carrier particle with adhesive forces acting between the carrier particles.

The term "compacting", as used herein, refers to applying pressure to more than one particle (e.g., carrier particle) to form compacted carrier matter, wherein the carrier particle at least partially remains adherent to each other upon release of the pressure. Techniques for compacting are known to the person skilled in the art (see, e.g., Odeku, O. A. et al., 2007, Pharmaceutical Reviews, 5(2)). Examples of techniques for compaction include, without limitation tableting, roller compaction, slugging, briquetting and/or centrifugation.

The compacted loaded carrier matter described herein is particularly stable and can be used for the obtainment of a particularly stable pharmaceutical composition (see e.g., Example 3). During compaction, the large surface areas of the loaded carrier particles as described herein form strong interparticle Van Der Waals adhesion forces that enable mechanical stability. Upon intake, water enters between the particles (e.g., by capillary forces), the distance-dependent Van Der Waals adhesion forces diminish, and the compacted loaded carrier matter disintegrates.

Accordingly, the invention is at least in part based on the surprising finding that the compacted loaded carrier matter described herein enables particular mechanical stability and/or fast disintegration time.

In certain embodiments, the invention relates the loaded carrier particle of the invention for use in a solid pharmaceutical composition.

In certain embodiments, the invention relates to a solid pharmaceutical composition comprising the loaded carrier particle according to the invention.

In certain embodiments, the invention relates to a solid pharmaceutical composition comprising the compacted loaded carrier matter produced according to the invention.

In certain embodiments, the solid pharmaceutical composition described herein is a solid pharmaceutical composition for oral, sublingual, buccal, nasal, bronchial, rectal, urethral, and/or intravaginal administration.

In certain embodiments, the solid pharmaceutical composition described herein, is a granulate, a tablet, a capsule, or a suppository.

In certain embodiments, the solid pharmaceutical composition described herein is a solid pharmaceutical composition for oral administration.

In certain embodiments, the solid pharmaceutical composition described herein is a solid pharmaceutical composition for oral administration that is disintegrated by water to facilitate swallowing and/or to enable site-specific delivery.

In certain embodiments, the solid pharmaceutical composition described herein is a solid pharmaceutical composition for oral administration selected from the group of effervescent tablet, orally disintegrating tablet, dispersible tablet, efference granulate, orally disintegrating granulate, and dispersible granulate.

In certain embodiments, the solid pharmaceutical composition described herein is a solid pharmaceutical composition for oral administration is a film-coated tablet and/or comprises a film-coated granulate. The film that coats the tablet or granulate described herein can have various functions such as taste masking, smell masking, appearance altering, and modifying the release of the therapeutic agent. In certain embodiments, film-coated tablet and/or comprises a film-coated granulate described herein are designed for at least one modified-release form selected of the group of immediate-release, delayed-release (e.g., timed-release), and pH-controlled-release. Methods to design a modified-release tablet are known to the person skilled in the art (see, e.g., US6419954; Pietrzak, K. et al., 2015, European journal of pharmaceutics and biopharmaceutics, 96, 380-387; de Sousa Rodrigues, L. A., et al., 2013, Colloids and Surfaces B: Biointerfaces, 103, 642-651). In these embodiments, the invention enables the fast and effective release of the therapeutic agent at the desired time point and/or compact storing of the therapeutic agent in the release compartment of the film-coated tablet and/or a film-coated granulate described herein. In certain embodiments, film-coated granulate described herein is comprised in a tablet and the coating enables pH-controlled release even after disintegrating of the tablet or after breaking the tablet.

In certain embodiments, the solid pharmaceutical composition described herein is a tablet or a capsule that can be disintegrated before intake or disintegrated in the mouth.

Some of the solid pharmaceutical composition designs mentioned above are associated with a lack of physical resistance (e.g., in blister packs) and limited ability to incorporate high concentrations of therapeutic agents. The invention provides the means and methods to produce compact and stable solid pharmaceutical compositions comprising high concentrations of therapeutic agents.

Accordingly, the invention is at least in part based on the surprising finding that the solid pharmaceutical composition described herein has particular desired drug delivery properties.

In certain embodiments, the invention relates to the solid pharmaceutical composition according to the invention, wherein the therapeutic agent is selected from the group of anxiolytic agents, sedative agents, narcotic agents, antidepressant agents, anti-migraine agents, anti-inflammatory agents, and anti-infective agents.

In certain embodiments, the invention relates to the compacted loaded carrier matter produced according to the invention or wherein the therapeutic agent is selected from the group of anxiolytic agents, sedative agents, narcotic agents, antidepressant agents, anti-migraine agents, anti-inflammatory agents, and anti-infective agents.

In certain embodiments, the invention relates to the loaded carrier particle according to the invention, wherein the therapeutic agent is selected from the group of anxiolytic agents, sedative agents, narcotic agents, antidepressant agents, anti-migraine agents, anti-inflammatory agents, and anti-infective agents.

In certain embodiments, the invention relates to the solid pharmaceutical composition according to the invention, wherein the therapeutic agent is an anxiolytic agent.

In certain embodiments, the invention relates to the compacted loaded carrier matter produced according to the invention or wherein the therapeutic agent is an anxiolytic agent.

In certain embodiments, the invention relates to the loaded carrier particle according to the invention, wherein the therapeutic agent is an anxiolytic agent.

The term "anxiolytic agent", as used herein, refers to a pharmaceutical compound used to treat symptoms in patients with anxiety or emotional disorders including stress, anxiety, neurosis, and obsessive-compulsive disorder. Anti-anxiety drugs are usually divided into two broad categories: benzodiazepines and non-benzodiazepines. In some embodiments, the anxiolytic agent described herein is benzodiazepine. In some embodiments, the anxiolytic agent described herein is a benzodiazepine selected from the group of clonazepam, diazepam, estazolam, flunitrazepam, lorazepam, midazolam, nitrazepam, oxazepam, triazolam, temazepam, chlordiazepoxide, alprazolam, clobazam, clorazepate, etizolam.

In some embodiments, the anxiolytic agent described herein is a non-benzodiazepine. In some embodiments, the anxiolytic agent described herein comprises at least one non-benzodiazepine selected from the class of serotonin 1A agonists, barbiturates, carbamates, antihistamines, opioids, and Z-drugs. In some embodiments, the anxiolytic agent described herein comprises at least one non-benzodiazepine selected from the group of buspirone, amobarbital, aprobarbital, butabarbital, mephobarbital, methohexital, pentobarbital, phenobarbital, primidone, secobarbital, thiopental, meprobamate, carisoprodol, tybamate, lorbamate, zaleplon, zolpidem, zopiclone, eszopiclone, chlorpheniramine, dexchlorpheniramine, dimenhydrinate, diphenhydramine, promethazine, trimeprazine, gabapentin, pregabalin, tramadol, tapentadol, morphine, diamorphine, hydromorphone, oxymorphone, oxycodone, hydrocodone, methadone, propoxyphene, meperidine, fentanyl, codeine, carfentanil, remifentanil, alfentanil, sufentanil, phenibut, mebicar, and gamma-hydroxybutyric acid.

In certain embodiments, the invention relates to the solid pharmaceutical composition according to the invention, wherein the therapeutic agent is a sedative agent.

In certain embodiments, the invention relates to the compacted loaded carrier matter produced according to the invention or wherein the therapeutic agent is a sedative agent.

In certain embodiments, the invention relates to the loaded carrier particle according to the invention, wherein the therapeutic agent is a sedative agent.

The term "sedative agent", as used herein, refers to a substance that induces sedation by reducing irritability or excitement. In some embodiments, the sedative agent described herein comprises at least one sedative agent selected from the class of barbiturates, benzodiazepines, Z-drugs, general anesthetics, herbal sedatives, methaqualone/methaqualone analogs, skeletal muscle relaxants, opioids, and antipsychotics. In some embodiments, the sedative agent described herein comprises at least one sedative agent selected from the group of amobarbital, aprobarbital, butabarbital, mephobarbital, methohexital, pentobarbital, phenobarbital, primidone, secobarbital, thiopental, clonazepam, diazepam, estazolam, flunitrazepam, lorazepam, midazolam, nitrazepam, oxazepam, triazolam,temazepam, chlordiazepoxide, alprazolam, clobazam, clorazepate, etizolam, zaleplon, zolpidem, zopiclone, eszopiclone, chlorpheniramine, dexchlorpheniramine, dimenhydrinate, diphenhydramine, promethazine, trimeprazine, ketamine, esketamine, afloqualone, cloroqualone, diproqualone, etaqualone, methaqualone, methylmethaqualone, mebroqualone, mecloqualone, nitromethaqualone, cannabinoids, baclofen, meprobamate, carisoprodol, cyclobenzaprine, metaxalone, methocarbamol, tizanidine, clonidine, chlorzoxazone, orphenadrine, gabapentin, pregabalin, tramadol, tapentadol, morphine, diamorphine, hydromorphone, oxymorphone, oxycodone, hydrocodone, methadone, propoxyphene, meperidine, fentanyl, codeine, carfentanil, remifentanil, alfentanil, sufentanil, olanzapine, clozapine, thiothixene, haloperidol, fluphenazine, prochlorperazine, trifluoperazine, loxapine, quetiapine, asenapine, gamma-hydroxybutyric acid and dextromethorphan.

In certain embodiments, the invention relates to the solid pharmaceutical composition according to the invention, wherein the therapeutic agent is a narcotic agent.

In certain embodiments, the invention relates to the compacted loaded carrier matter produced according to the invention or wherein the therapeutic agent is a narcotic agent.

In certain embodiments, the invention relates to the loaded carrier particle according to the invention, wherein the therapeutic agent is a narcotic agent.

The term "narcotic agent", as used herein, refers to a psychoactive compound with numbing or paralyzing properties. In some embodiments, the narcotic agent described herein comprises at least one narcotic agent selected from the class of barbiturates, benzodiazepines, Z-drugs, general anesthetics, and opioids. In some embodiments, the narcotic agent described herein comprises at least one narcotic agent selected from the group of amobarbital, aprobarbital, butabarbital, mephobarbital, methohexital, pentobarbital, phenobarbital, primidone, secobarbital, thiopental, clonazepam, diazepam, estazolam, flunitrazepam, lorazepam, midazolam, nitrazepam, oxazepam, triazolam, temazepam, chlordiazepoxide, alprazolam, clobazam, clorazepate, etizolam, zaleplon, zolpidem, zopiclone, eszopiclone, chlorpheniramine, dexchlorpheniramine, dimenhydrinate, diphenhydramine, promethazine, trimeprazine, ketamine, esketamine, tramadol, tapentadol, morphine, diamorphine, hydromorphone, oxymorphone, oxycodone, hydrocodone, methadone, propoxyphene, meperidine, fentanyl, codeine, carfentanil, remifentanil, alfentanil, sufentanil, dextromethorphan.

In some embodiments, the sedative agent described herein is also an anxiolytic agent. In some embodiments, the narcotic agent described herein is also an anxiolytic agent. In some embodiments, the sedative agent described herein is also a narcotic agent.

In certain embodiments, the invention relates to the solid pharmaceutical composition according to the invention, wherein the therapeutic agent is an antidepressant agent.

In certain embodiments, the invention relates to the compacted loaded carrier matter produced according to the invention or wherein the therapeutic agent is an antidepressant agent.

In certain embodiments, the invention relates to the loaded carrier particle according to the invention, wherein the therapeutic agent is an antidepressant agent.

The term "antidepressant agent", as used herein, refers to a therapeutic agent with properties useful in the treatment of a depressive disorder.

In some embodiments, the antidepressant agent described herein is an antidepressant agent selected from the class of SSRI, SNRI, SMS, SARI, NRI, NDRI, TCA, TeCA, MAOI. In some embodiments, the antidepressant agent described herein is an antidepressant agent selected from the group of Agomelatine, Esketamine, Ketamine, Tandospirone, Tianeptine, Metralindole, Moclobemide, Pirlindole, Toloxatone, Caroxazone, Selegiline, Isocarboxazid, Phenelzine, Tranylcypromine, Amoxapine, Maprotiline, Mianserin, Mirtazapine, Setiptiline, Amitriptyline, Amitriptylinoxide, Clomipramine, Desipramine, Dibenzepin, Dimetacrine, Dosulepin, Doxepin, Imipramine, Lofepramine, Melitracen, Nitroxazepine, Nortriptyline, Noxiptiline, Opipramol, Pipofezine, Protriptyline, Trimipramine, Bupropion, Atomoxetine, Reboxetine, Teniloxazine, Viloxazine, Trazodone, Vilazodone, Vortioxetine, Desvenlafaxine, Duloxetine, Levomilnacipran, Milnacipran, Venlafaxine, Citalopram, Escitalopram, Fluoxetine, Fluvoxamine, Paroxetine, and Sertraline.

In certain embodiments, the invention relates to the solid pharmaceutical composition according to the invention, wherein the therapeutic agent is an anti-migraine agent.

In certain embodiments, the invention relates to the compacted loaded carrier matter produced according to the invention or wherein the therapeutic agent is an anti-migraine agent.

In certain embodiments, the invention relates to the loaded carrier particle according to the invention, wherein the therapeutic agent is an anti-migraine agent.

The term "anti-migraine agent", as used herein, refers to a therapeutic agent with properties useful in the treatment of acute migraine symptoms.

In some embodiments, the anti-migraine agent described herein is an agent selected from the group of paracetamol, NSAID, and triptans. In some embodiments, the anti-migraine agent described herein is an NSAID selected from the group of acetylsalicylic acid, ibuprofen, naproxen, diclofenac, indomethacin, piroxicam, and phenylbutazone. In some embodiments, the anti-migraine agent described herein is a triptan selected from the group of sumatriptan, almotriptan, eletriptan, frovatriptan, naratriptan, rizatriptan, and zolmitriptan. In some embodiments, the anti-migraine agent described herein is an NSAID and triptan combination. In some embodiments, the anti-migraine agent described herein is an NSAID and antiemetic combination e.g. NSAID and domperidone.

In certain embodiments, the invention relates to the solid pharmaceutical composition according to the invention, wherein the therapeutic agent is an anti-inflammatory agent.

In certain embodiments, the invention relates to the compacted loaded carrier matter produced according to the invention or wherein the therapeutic agent is an anti-inflammatory agent.

In certain embodiments, the invention relates to the loaded carrier particle according to the invention, wherein the therapeutic agent is an anti-inflammatory agent.

The term "anti-inflammatory agent", as used herein, refers to a therapeutic agent that reduces inflammation and/or swelling. In some embodiments, the anti-inflammatory agent described herein is an anti-inflammatory agent selected from the group of NSAIDs, antileukotrienes, immune selective anti-inflammatory derivatives, glucocorticoids, steroids. In some embodiments, the anti-inflammatory agent described herein is an anti-inflammatory agent selected from the group of acetylsalicylic acid, ibuprofen, naproxen, diclofenac, indomethacin, piroxicam, phenylbutazone, prednisone, betamethasone, budesonide, cortisone, dexamethasone, hydrocortisone, methylprednisolone, prednisolone, tramcinolone, fluticasone, infliximab, adalimumab, certolizumab pegol, golimumab, etanercept, curcumin, IL-1RA, canakinumab, allopurinol, colchicine, prednisone, pentoxifylline, rosuvastatin and oxypurinol.

In certain embodiments, the invention relates to the solid pharmaceutical composition according to the invention, wherein the therapeutic agent is an anti-infective agent.

In certain embodiments, the invention relates to the compacted loaded carrier matter produced according to the invention or wherein the therapeutic agent is an anti-infective agent.

In certain embodiments, the invention relates to the loaded carrier particle according to the invention, wherein the therapeutic agent is an anti-infective agent.

The term "anti-infective agent", as used herein, refers to a therapeutic agent with properties useful in the treatment against an infection of a pathogen. An anti-infective agent may have, inter alia, properties of preventing, inhibiting, suppressing, reducing, adversely impacting, and/or interfering with the growth, survival, replication, function, and/or dissemination of a pathogen. In some embodiments, the anti-infective agent described herein comprises at least one therapeutic agent selected from the class of amebicides, anthelmintic, antifungals, antimalarial agents, antibiotics, and antiviral drugs.

The term "anthelmintic", as used herein, refers to a therapeutic agent with properties useful in the treatment against an infection of a helminth such as flukes, roundworms, and tapeworms. An anthelmintic may have, inter alia, properties of preventing, inhibiting, suppressing, reducing, adversely impacting, and/or interfering with the growth, survival, replication, function, and/or dissemination of a helminth. In some embodiments, the anthelmintic described herein comprises at least one therapeutic agent selected from the group of pyrantel, ivermectin, mebendazole, albendazole, praziquantel, and miltefosine.

In some embodiments, the anti-infective agent described herein is an antifungal. The term "antifungal", as used herein, refers to a therapeutic agent with properties useful in the treatment against a fungus infection. An antifungal may have, inter alia, properties of preventing, inhibiting, suppressing, reducing, adversely impacting, and/or interfering with the growth, survival, replication, function, and/or dissemination of a fungus. In some embodiments, the antifungal described herein comprises at least one antifungal selected from the class of azole antifungals, echinocandins, and polyenes. In some embodiments, the antifungal described herein comprises at least one antifungal selected from the group of voriconazole, itraconazole, posaconazole, fluconazole, ketoconazole, clotrimazole, and miconazole.

In some embodiments, the anti-infective agent described herein is an amebicide. The term "amebicide", as used herein, refers to a therapeutic agent with properties useful in the treatment against an amoeba infection. An amebicide may have, inter alia, properties of preventing, inhibiting, suppressing, reducing, adversely impacting, and/or interfering with the growth, survival, replication, function, and/or dissemination of an amoeba. In some embodiments, the amebicide described herein comprises at least one therapeutic agent selected from the group of nitazoxanide, chloroquine, paromomycin, metronidazole, and tinidazole,

In some embodiments, the anti-infective agent described herein is an antibiotic. The term "antibiotic", as used herein, refers to a therapeutic agent with properties useful in the treatment against a bacteria-related disease. An antibiotic may have, inter alia, properties of preventing, inhibiting, suppressing, reducing, adversely impacting, and/or interfering with the growth, survival, replication, function, and/or dissemination of a bacterium. In some embodiments, the antibiotic described herein comprises at least one antibiotic selected from the class of macrolides (e.g., erythromycin), penicillins (e.g., nafcillin), cephalosporins (e.g., cefazolin), carbapenems (e.g., imipenem), monobactam (e.g., aztreonam), other beta-lactam antibiotics, beta-lactam inhibitors (e.g., sulbactam), oxalines (e.g. linezolid), aminoglycosides (e.g., gentamicin), chloramphenicol, sufonamides (e.g., sulfamethoxazole), glycopeptides (e.g., vancomycin), quinolones (e.g., ciprofloxacin), tetracyclines (e.g., minocycline), trimethoprim, metronidazole, clindamycin, mupirocin, rifamycins (e.g., rifampin), streptogramins (e.g., quinupristin and dalfopristin), lipoprotein (e.g., daptomycin) and polyenes (e.g., amphotericin B). In some embodiments, the antibiotic described herein comprises at least one antibiotic selected from the group of erythromycin, nafcillin, cefazolin, imipenem, aztreonam, gentamicin, sulfamethoxazole, vancomycin, ciprofloxacin, trimethoprim, rifampin, metronidazole, clindamycin, teicoplanin, mupirocin, azithromycin, clarithromycin, ofloxacin, lomefloxacin, norfloxacin, nalidixic acid, sparfloxacin, pefloxacin, amifloxacin, gatifloxacin, moxifloxacin, gemifloxacin, enoxacin, fleroxacin, minocycline, linezolid, temafloxacin, tosufloxacin, clinafloxacin, sulbactam, clavulanic acid and any combination thereof.

In some embodiments, the anti-infective agent described herein is an antiviral drug. The term "antiviral drug", as used herein, refers to a therapeutic agent with properties useful in the treatment of a virus-related disease. The properties useful in the treatment of a virus-related disease may include, inter alia, properties of preventing, inhibiting, suppressing, reducing, adversely impacting, and/or interfering with the growth, survival, replication, function, and/or dissemination of a virus. In some embodiments, the antiviral drug described herein comprises at least one antiviral drug selected from the class of nucleoside analogs, pyrophosphate analogs, nucleoside reverse transcriptase inhibitors, nonnucleoside reverse transcriptase inhibitors, protease inhibitors, integrase inhibitors, entry inhibitors, acyclic guanosine analogs, acyclic nucleoside phosphonate analogs, and 5-substituted 2'-deoxyuridine analogs.

In some embodiments, the antiviral drug described herein comprises at least one antiviral drug selected from the group of abacavir, acyclovir, adefovir, amantadine, amprenavir, asunaprevir, atazanavir, boceprevir, brivudine, cidofovir, cobicistat, dasabuvir, daclatasvir, darunavir, delavirdine; didanosine; dipivoxil, docosanol, dolutegravir, efavirenz, elbasvir, elvitegravir, emtricitabine, enfuvirtide, entecavir, etravirine, famciclovir, favipiravir, fomivirsen, fosamprenavir, foscarnet, ganciclovir, grazoprevir, idoxuridine, imiquimod, indinavir, interferon alfacon 1, lamivudine, laninamivir octanoate, ledipasvir, lopinavir, maraviroc, nelfinavir, nevirapine, ombitasvir, oseltamivir, palivizumab, paritaprevir, pegylated interferon alfa 2a, penciclovir, peramivir, podofilox, raltegravir, ribavirin, rilpivirine, rimantadine, ritonavir, RSV-IGIV, Saquinavir, simeprevir, sofosbuvir, stavudine, telaprevir, telbivudine, tenofovir alafenamide, tenofovir disoproxil fumarate, tipranavir, trifluridine, valacyclovir, valganciclovir, vaniprevir, variZIG, vidarabine, VZIG, zalcitabine, zanamivir and zidovudine.

In some embodiments, the therapeutic agent of the invention is a drug from the drug class described herein. In some embodiments, the therapeutic agent of the invention is a prodrug of the therapeutic agents described herein, wherein the prodrug is activated before the therapeutic effect, e.g., at the target site of the loaded carrier particle. Prodrugs and design thereof are known to the person skilled in the art (see, e.g., Rautio, J. et al., 2008, Nature Reviews Drug Discovery, 7(3), 255-270; Dubey, S., and Valecha, V., 2014, World Journal of Pharmaceutical Research, 3(7), 277-297). In some embodiments, the therapeutic agent of the invention is a pharmaceutically acceptable salt of the therapeutic agent described herein. In some embodiments, the therapeutic agent of the invention is a structural analog of the therapeutic agents described herein and is used for an equivalent therapeutic indication as the therapeutic agent described herein.

The means and method provided herein can improve the drug delivery and action of the anxiolytic agent, sedative agent, narcotic agent, antidepressant agent, anti-migraine agent, anti-inflammatory agent, and/or anti-infective agent described herein.

For example, some of the therapeutic agents described herein require a fast absorption to unfold their full therapeutic potential. Other therapeutic agents described herein are usually administered to patients with problems swallowing or require chronic intake. The means and method provided facilitate absorption and facilitate intake of the therapeutic agent.

Accordingly, the invention is at least in part based on the surprising finding that the means and method provided by the invention can enhance and/or support the therapeutic effect of anxiolytic agents, sedative agents, narcotic agents, antidepressant agents, anti-migraine agents, anti-inflammatory agents, and/or anti-infective agents.

In certain embodiments, the invention relates to the solid pharmaceutical composition according to the invention, wherein the solid pharmaceutical composition comprises at least one adjuvant.

The term "adjuvant", as used herein, refers to an ingredient of the solid pharmaceutical composition that is generally non-toxic to recipients at the dosages employed and that is not a carrier particle, compressed carrier matter, or a therapeutic agent.

In some embodiments, the adjuvant described herein comprises at least one adjuvant selected from the group of anti-adherents, binders, fillers, coatings, disintegrants, taste altering agents, smell altering agents, appearance altering agents, flowability enhancement agents, lubricants, preservatives, and sorbents.

In some embodiments, the adjuvant described herein improves the adhesion of the loaded carrier particle at the target site, e.g., at the mucosa.

In some embodiments, the content of adjuvant described herein is between 0.1 and 40 % by weight based on the weight of the solid pharmaceutical composition.

The adjuvant can facilitate production or further enhance the desired drug delivery properties of the solid pharmaceutical composition according to the invention.

Accordingly, the invention is at least in part based on the surprising finding that the solid pharmaceutical composition according to the invention can be further improved by at least one adjuvant.

In certain embodiments, the invention relates to the solid pharmaceutical composition according to the invention, wherein the at least one adjuvant is selected from the group of disintegrant, lubricant, and flowability enhancement agent.

In certain embodiments, the invention relates to the solid pharmaceutical composition according to the invention, wherein the at least one adjuvant is a disintegrant.

The term "disintegrant", as used herein, refers to an agent for the preparation of a solid pharmaceutical composition, which causes the solid pharmaceutical composition to disintegrate and release the therapeutic agent on contact with moisture. Agents with disintegrant properties are known to the person skilled in the art (see, e.g., Desai, P. M. t al., 2016, Journal of pharmaceutical sciences, 105(9), 2545-2555). In some embodiments, the content of the disintegrant described herein is between 2 % and 25 % by weight based on the weight of the solid pharmaceutical composition. In some embodiments, disintegrant described herein comprises at least one disintegrant selected from the group of cellulose derivative, starch, bentonite, sodium alginate, pectin and cross-linked polyvinylpyrrolidone. In some embodiments, the disintegrant described herein comprises at least one disintegrant selected from the group of sodium cellulose glycolate, tyloses, primojel, explotab, bentonite, sodium alginate, pectin and crospovidone.

In certain embodiments, the disintegrant described herein is a disintegrant that alters properties for site specific drug delivery (e.g., increasing mucoadhesiveness). In certain embodiments, the disintegrant described herein is a disintegrant that jellifies in alkali environment. In certain embodiments, the disintegrant described herein comprises croscarmellose sodium.

In certain embodiments, the invention relates to the solid pharmaceutical composition according to the invention, wherein the at least one adjuvant is a lubricant.

The term "lubricant", as used herein, refers to an agent that prevents the ingredients if the solid pharmaceutical composition from clumping together and/or from sticking to the equipment that is in contact with the solid pharmaceutical composition during the production process.

Agents with lubricant properties are known to the person skilled in the art (see, e.g., Wang, J., et al., 2010, European journal of pharmaceutics and biopharmaceutics, 75(1), 1-15; US 5843477; Paul, S. et al., 2018, European Journal of Pharmaceutical Sciences, 117, 118-127).

In some embodiments, the content of lubricant described herein is between 0.25 and 5 % by weight based on the weight of the solid pharmaceutical composition.

In some embodiments, the lubricant described herein is a hydrophilic lubricant. In some embodiments, the lubricant described herein is a hydrophobic lubricant. In some embodiments, the lubricant described herein is a solid fatty acid or a salt thereof. In some embodiments, the lubricant described herein comprises at least one lubricant selected from the group of stearic acid, palmitic acid, myristic acid. In some embodiments, the lubricant described herein comprises at least one lubricant selected from the group of magnesium silicate, stearic acid, sodium stearyl fumarate, boric acid, Carbowax (PEG) 4000/6000, sodium oleate, sodium benzoate, sodium acetate sodium lauryl sulfate, Mg-Lauryl sulfate, Metal (Mg, Ca, Na) stearate, Sterotex, Talc, Waxes, Stear-O-Wet, Glyceryl behenate.

In some embodiments, the lubricant described herein has additional properties of a flowability enhancement agent. In some embodiments, the lubricant described herein has additional properties of a disintegrant.

In certain embodiments, the invention relates to the solid pharmaceutical composition according to the invention, wherein the at least one adjuvant is a flowability enhancement agent.

The term "flowability enhancement agent", as used herein, refers to an agent that reduces interparticle friction and cohesion. Agents with flowability enhancing properties are known to the person skilled in the art (see, e.g., Augsburger, L. L., and Shangraw, R. F, 1966, Effect of glidants in tableting. Journal of Pharmaceutical Sciences, 55(4), 418-423; Armstrong, N. A., 2008, In Pharmaceutical Dosage Forms-Tablets (pp. 267-284). CRC Press). Certain flowability enhancement agents enhance flowability only in a certain concentration range. Accordingly, the concentration of the flowability enhancement agent in the solid pharmaceutical composition depends on the specific flowability enhancement agent. In some embodiments, the flowability enhancement agent described herein comprises at least one flowability enhancement agent selected from the group of silica gel, fumed silica, talc, and magnesium carbonate and hydrated sodium silicoaluminate.

Techniques for prediction and determination of adequate flowability are known to the person skilled in the art (see, e.g., Hildebrandt, C, et al., 2019, Pharmaceutical development and technology, 24(1), 35-47; Morin, G., & Briens, L, 2013, AAPS Pharmscitech, 14(3), 1158-1168).

The disintegrant, lubricant, and/or flowability enhancement agent can facilitate production or further enhance the desired drug delivery properties of the solid pharmaceutical composition according to the invention.

Accordingly, the invention is at least in part based on the surprising finding that the properties of the solid pharmaceutical composition according to the invention can be further improved by a disintegrant, a lubricant, and/or a flowability enhancement agent.

In certain embodiments, the invention relates to the solid pharmaceutical composition according to the invention, wherein the at least one adjuvant is selected from the group taste altering agents, smell altering agents, and appearance altering agents.

In certain embodiments, the invention relates to the solid pharmaceutical composition according to the invention, wherein at least one adjuvant is a smell-altering agent.

The term "smell altering agent", as used herein, refers to any adjuvant that can induce a smell alteration detectable by a human subject and/or an olfactometer. Olfactometers are known to the person skilled in the art such as flow-olfactometers, dynamic dilution olfactometers, and field olfactometers. Smell-altering agents that can be used to alter the smell of a solid pharmaceutical composition are known to the person skilled in the art (see, e.g., US 20120164217; US 6667059). In some embodiments, the smell-altering agent described herein is a scenting agent selected from the group of lemon oil, scented oils from flowers, such as lilac, honeysuckle, rose, carnation, and other such oils with GRAS status. Oils with GRAS status include, without limitation, basil (ocimum basilicum), bergamot (citrus bergamia), black pepper (piper nigrum), cassia (cinnamomum cassia), cinnamon (cinnamomum zeylanicum), clary sage (salvia sclarea), clove (eugenia caryophyllata), coriander (coriandrum sativum), cumin (cuminum cyminum), fennel (foeniculum vulgare), geranium (pelargonium graveolens), ginger (zingiber officinale), grapefruit (citrus x paradisi), juniper berry (juniperus communis), lemon (citrus limon), lemongrass (cymbopogon flexuosus), lime (citrus aurantifolia), marjoram (origanum majorana), melissa (melissa officinalis), oregano (origanum vulgare), peppermint (mentha piperita), petitgrain (citrus aurantium), roman chamomile (anthemis nobilis), rosemary (rosmarinus officinalis), spearmint (mentha spicata), tangerine (citrus reticulate), thyme (thymus vulgaris), wild orange (citrus sinensis) and ylang ylang (cananga odorata).

In some embodiments, the content of smell-altering agent described herein is between 0.1 and 10 % by weight based on the weight of the solid pharmaceutical composition. Higher specific surface of the loaded carrier particles intensifies the action of the smell-altering agent.

In some embodiments, the smell-altering agent described herein, alters the smell perceived by the ingesting subject during ingestion of the solid pharmaceutical.

In some embodiments, the smell altering agent described herein, alters the smell perceived by the ingesting subject after ingestion of the solid pharmaceutical.

In certain embodiments, the invention relates to the solid pharmaceutical composition according to the invention, wherein at least one adjuvant is an appearance-altering agent.

The term "appearance-altering agent", as used herein, refers to an adjuvant that alters the color and/or the shape of a solid pharmaceutical composition.

Appearance-altering agents and their use are well known to the person skilled in the art (see, e.g., Biswal, P. K. et al., 2015, International Journal of Pharmaceutical, Chemical & Biological Sciences, 5(4).)

In some embodiments, the appearance-altering agent described herein alters the appearance of the intact solid pharmaceutical composition. In some embodiments, the appearance altering agent described herein alters the appearance of the disintegrated solid pharmaceutical composition.

In some embodiments, the appearance-altering agent enables the identification, flavor perception (e.g., red for cherry), brand identification, quality perception, and/or counterfeit prevention of the solid pharmaceutical composition according to the invention.

In some embodiments, the content of the appearance-altering agent described herein is between 0.1 and 2 % by weight based on the weight of the solid pharmaceutical composition. High specific surface area of the carrier material intensifies action of the appearance-altering agents.

In certain embodiments, the invention relates to the solid pharmaceutical composition according to the invention, wherein at least one adjuvant is a taste-altering agent.

The term "taste altering agent", as used herein, refers to an adjuvant that alters the flavor of a solid pharmaceutical composition. The determination of flavor can be done by various mass spectrometric techniques or by organoleptic testing by humans. Taste altering agents are well known to the person skilled in the art (see, e.g., Ahire, S. B, et al., 2012, Pharma Science Monitor, 3(3); US20070122475; WO1998043675)

In some embodiments, the taste-altering agent described herein is a taste-altering agent selected from the group of lemon, orange, grapefruit, berry flavors, peppermint, licorice, and menthol.

In some embodiments, the taste altering agent described herein is a taste altering agent selected from the group of manzanate, diacetyl, acetyl propionyl, acetoin, isoamyl acetate, benzaldehyde, cinnamaldehyde, ethyl propionate, methyl anthranilate, limonene, ethyl decadienoate, allyl hexanoate, ethyl maltol, 2,4-dithiapentane, ethylvanillin, methyl salicylate, Carbomer 934, Carbomer 971, Carbomer 974, PEG-5M, carrageenan, chondroitin sulfate, dextran sulfate, alginic acid, dielan gum, xanthan gum, zinc salt.

The content of the taste-altering agent described herein depends on the intensity of the taste-altering agent. The high specific surface area of the loaded carrier particles may intensify the taste-altering action of the taste-altering agent.

In some embodiments, the content of the appearance altering agent described herein is between 0.1 and 20 % by weight based on the weight of the solid pharmaceutical composition.

The use of a smell-altering agent and appearance-altering agent, particularly a taste-altering agent described herein, can improve the smell, taste, and/or appearance of the solid pharmaceutical composition according to the invention. An improved smell, taste, and/or appearance can facilitate intake of the solid pharmaceutical composition according to the invention.

Accordingly, the invention is at least in part based on the surprising finding that taste altering agents, smell altering agents, and/or appearance altering agents can facilitate intake of the solid pharmaceutical composition according to the invention.

In certain embodiments, the invention relates to the solid pharmaceutical composition according to the invention, wherein the taste altering agent is selected from the group of artificial sweeteners, acidity modifiers, gum, cellulose derivatives, hard fat, and salt. The high surface area of the loaded carrier particle of the invention may intensify the effect on the olfactory sensation of the taste altering agents described herein.

The term "artificial sweetener", as used herein, refers to an adjuvant that provides a sweet taste like that of sugar. Artificial sweeteners may be derived through the manufacturing of plant extracts or processed by chemical synthesis. In some embodiments, the artificial sweetener described herein is an artificial sweetener selected from the group of sucralose, thaumatin, neohesperidine, aspartame, saccharin, acesulfame, erythritol, xylitol, sorbitol, and stevia.

Concentrations of the artificial sweetener to be used in the solid pharmaceutical composition according to the invention are known to the person skilled in the art and depend on the intensity of the artificial sweetener.

The term "acidity modifiers", as used herein, refers to an adjuvant that alters the perceived acidity upon intake of a pharmaceutical composition. In some embodiments, the acidity modifier described herein is citric acid, phosphoric acid, and/or a salt thereof.

Concentrations of the acidity modifiers to be used in the solid pharmaceutical composition according to the invention are known to the person skilled in the art and depend on the intensity of the acidity modifier and on the acidity to be modified.

The term "gum", as used herein, refers to an adjuvant that alters the perceived consistency upon intake of a pharmaceutical composition. In some embodiments, the gum described herein improves the adhesion of the carrier particle at the target site, e.g., at the mucosa.

In some embodiments, the gum described herein is a gum selected from the group of alginate esters, carrageenan, xanthanes, agar-agar, pectines, pectic acid, gum arabic, gum tragacanth and gum karaya, guar gums, and quaternized guar gums.

The term "cellulose derivative", as used herein, refers to a polysaccharide polymer such as cellulose ether derivatives and cellulose ester derivatives that can be used to mask or alter an unpleasant taste of a pharmaceutical composition.

In some embodiments, the cellulose derivative described herein is a cellulose derivative selected from the group of methylcellulose, hydroxypropyl cellulose, hydroxypropyl methylcellulose, and carboxymethyl cellulose. In some embodiments, the cellulose derivative described herein is a water-insoluble polymer such as ethyl cellulose. In some embodiments, the cellulose derivative described herein is a watersoluble polymer such as a polymer of hydroxypropyl methylcellulose. In some embodiments, the cellulose derivative described herein is a nonionic cellulose ether such as ethylcellulose, hydroxyethyl cellulose, hydroxypropyl cellulose, methylcellulose, carboxymethyl cellulose, or hydroxypropyl methylcellulose.

In some embodiments, the cellulose derivative described herein is an anionic ether derivative like sodium carboxymethyl cellulose.

In some embodiments, the solid pharmaceutical composition according to the invention and/or the loaded carrier particle in the solid pharmaceutical composition according to the invention is/are coated with a cellulose derivative to mask an unpleasant taste.

In some embodiments, the taste-altering agents, such as hard fat and/or salt, induces or enhances the perception of a pleasant taste.

In some embodiments, the taste-altering agents, such as the cellulose derivative or gum, reduces the diffusion of an unpleasant taste (e.g., bitter taste).

The use of a taste-altering agent, particularly a taste-altering agent described herein, can improve the taste of the solid pharmaceutical composition according to the invention or mask the taste of an ingredient of the solid pharmaceutical composition according to the invention. An improved taste can facilitate intake of the solid pharmaceutical composition according to the invention.

Accordingly, the invention is at least in part based on the surprising finding that artificial sweetener, acidity modifiers, gum, cellulose derivative, hard fat, and/or salt can facilitate intake of the solid pharmaceutical composition according to the invention.

In certain embodiments, the invention relates to the solid pharmaceutical composition according to the invention, for use in treatment.

In certain embodiments, the invention relates to the compacted loaded carrier matter produced according to the invention for use in treatment.

In certain embodiments, the invention relates to the loaded carrier particle according to the invention for use in treatment.

The phrases "for use in treatment" or "for use in the treatment of a disease or disorder" can also mean "for use in the treatment of symptoms of a disease or disorder". Symptoms of diseases or disorders can be symptoms described herein and/or symptoms of a disease or disorder known to the person skilled in the art, such as symptoms in the description of the ICD-11 regarding a certain disease or disorder (World Health Organization, 2018, ICD-11 for mortality and morbidity statistics) or symptoms or diagnostic criteria described in the DSM-5 (American Psychiatric Association. Diagnostic and Statistical Manual of Mental Disorders. 5th ed. 2013).

The phrases "for use in treatment" or "for use in the treatment of a disease or disorder" can refer to treatment in any age group, including pediatric patients, adults and/or geriatric patients.

In certain embodiments, the invention relates to the solid pharmaceutical composition according to the invention, for use in treatment, wherein the solid pharmaceutical composition according to the invention comprises a therapeutic agent described herein.

In certain embodiments, the invention relates to the compacted loaded carrier matter produced according to the invention, for use in treatment, wherein the compacted loaded carrier matter produced according to the invention comprises a therapeutic agent described herein.

In certain embodiments, the invention relates to the loaded carrier particle according to the invention, for use in treatment, wherein the loaded carrier particle according to the invention comprises a therapeutic agent described herein.

For example, a therapeutic agent can be administered in an effective dose and a patient, wherein the solid pharmaceutical composition according to the invention, the compacted loaded carrier matter produced according to the invention, or the loaded carrier particle according to the invention facilitates drug delivery and/or enhances the therapeutic effect of the therapeutic agents by the improved drug delivery properties.

Accordingly, the invention is at least in part based on the surprising finding the solid pharmaceutical composition according to the invention, the compacted loaded carrier matter produced according to the invention, or the loaded carrier particle according to the invention can improve treatment.

In certain embodiments, the invention relates to the solid pharmaceutical composition for use according to the invention for use in the treatment of a geriatric disease or disorder

In certain embodiments, the invention relates to the compacted loaded carrier matter for use according to the invention for use in the treatment of a geriatric disease or disorder.

In certain embodiments, the invention relates to the loaded carrier particle for use according to the invention for use in the treatment of a geriatric disease or disorder.

The term "geriatric disease or disorder", as used herein, refers to a disease or disorder that affects elderly people. In some embodiments, a geriatric disease or disorder as described herein is a disease or disorder that primarily affects elderly people, that is that elderly people are a bigger patient group than younger adults and children, particularly disease or disorders wherein elderly people are at least 40%, 50%, 60%, 70%, 80% or 90% of the patient population. In some embodiments, a geriatric disease or disorder as described herein is a disease or disorder age-related morbidity and/or mortality increase. In some embodiments, the phrase "for use in the treatment of a geriatric disease or disorder" refers to use in the treatment of a disease or disorder in an elderly patient.

In some embodiments, elderly people described herein are people above a certain age such as above the age of 50, 55, 60, 65, 70, 75, 80, or 85 years. However, some behaviors can accelerate the aging of the body. Smokers, for example, consume their respiratory system reserve earlier resulting in a more rapid aging process of the lung and other organs. Accordingly, in certain embodiments, elderly people described herein are defined by other or additional criteria than age. In some embodiments the elderly people described herein comprise adults with certain age-related morbidities (e.g., tobacco consumption, cancer, diabetes). In some embodiments the elderly people described herein comprise adults with certain age accelerating morbidities (e.g., genetic disease or disorder). In some embodiments, elderly people described herein are defined by a certain guideline such as one of the guidelines analyzed and/or described by Singh, S., & Bajorek, B., 2014, Pharmacy practice, 12(4), 489.

In some embodiments, the geriatric disease or disorder is at least one disease or disorder selected from the group of osteoporosis, diabetes mellitus, cancer, benign prostatic hyperplasia, and cardiovascular disease.

Symptoms of geriatric disease or disorders include, without limitation, decreased cognitive function, depression, incontinence, frailty, vitamin d deficit, kidney failure, and chronic pain.

Treatment of geriatric disease or disorders differs from standard adult medicine because the unique needs of elderly patients need to be considered. The aged body differs physiologically from the younger adult body, e.g., during old age, the decline of various organ systems becomes manifest. Depending for example on previous health issues, lifestyle choices, and remaining reserves different factors may become relevant for treatment in elderly patients. Such factors relevant for treatment in elderly patients include for example limited ability to swallow oral medication, compliance to treatment, altered absorption, altered distribution, altered metabolism, altered excretion, increased probability for drug interactions, increased probability for adverse drug reactions, increased quality of life concerns, decreased social support and decreased functional abilities.

The means and methods provided by the invention are particularly useful for treatment in elderly patients. For example, improved desired drug delivery properties such as fast disintegration time, e.g., of an ODT comprising a high dose of the therapeutic agent as described herein may improve drug absorption and facilitate compliance in the context of treatment of a geriatric disease or disorder. The improved mechanical stability of, e.g., a solid pharmaceutical composition may allow packaging that is easier to open without damaging the solid pharmaceutical composition and meets the needs of elderly patients with decreased functional abilities.

Accordingly, the invention is at least in part based on the surprising finding the solid pharmaceutical composition according to the invention, the compacted loaded carrier matter produced according to the invention or the loaded carrier particle according to the invention can improve the treatment of a geriatric disease or disorder.

In certain embodiments, the invention relates to the solid pharmaceutical composition for use according to the invention, for use in the treatment of a pediatric disease or disorder.

In certain embodiments, the invention relates to the compacted loaded carrier matter for use according to the invention for use in the treatment of a pediatric disease or disorder.

In certain embodiments, the invention relates to the loaded carrier particle for use according to the invention, for use in the treatment of a pediatric disease or disorder.

The term "pediatric disease or disorder", as used herein, refers to a disease or disorder that affects infants and children. In some embodiments, a pediatric disease or disorder as described herein is a disease or disorder that primarily affects infants and children, that is that infants and children are a bigger patient group than adults and elderly people, particularly disease or disorders wherein infants and children are at least 40%, 50%, 60%, 70%, 80% or 90% of the patient population. In some embodiments, the phrase "for use in the treatment of a pediatric disease or disorder" refers to use in the treatment of a disease or disorder in an infant or a child.

In some embodiments, infants and children described herein are people below a certain age such as below the age of 21, 20, 19, 18, 17, 16, 15, 14, 13, 12, 11, 10, 9, 8, 7, 6, 5, 4, 3, 2, 1 or 0.5 year(s). Other or additional factors than age may be relevant in the use in the treatment of a pediatric disease or disorder. In some embodiments, the infants and children described herein are people below a certain bodyweight such as below 44kg, 42kg, 40kg, 38kg, 36kg, 34kg, 32kg, 30kg, 28kg, 26kg, 24kg, 22kg or 20kg. In some embodiments, the infants and children described herein are people defined by another maturation marker or a further maturation marker.

In some embodiments, the pediatric disease or disorder described herein is a pain in a pediatric patient and/or an infection in a pediatric patient.

In some embodiments, the pediatric disease or disorder described herein is at least one disease or disorder selected from the group of anemia, asthma, chickenpox, diphtheria, leukemia, measles, mumps, pneumonia, polio, tuberculosis, whooping cough, Lyme disease, fever, down's syndrome, dental caries, cystic fibrosis, Chagas disease, candidiasis, cancer, and bronchiolitis.

In some embodiments, the pediatric disease or disorder described herein is a mental disorder such as pediatric generalized anxiety disorder and/or childhood-onset bipolar disorder.

The body of a child infant or neonate differs substantially in physiology from that of an adult. Children are not simply "little adults". The immature physiology of the child infant or neonate must be taken into account and factors relevant for treatment in pediatric patients may have to be considered. Such factors relevant for treatment in pediatric patients include for example limited ability to swallow oral medication, compliance to treatment, altered absorption, altered distribution, altered metabolism, altered excretion, concerns regarding developmental issues, and limited functional abilities.

The means and methods provided by the invention are particularly useful for treatment in pediatric patients. For example, the improved desired drug delivery properties such as fast disintegration time, e.g., of an ODT comprising a high dose of the therapeutic agent as described herein may improve drug absorption and facilitate compliance in the context of treatment of a pediatric disease or disorder.

Furthermore, the means and methods provided herein enable a particularly low water binding of ODTs (see, e.g., Example 6). This property is particularly relevant because large volumes of bound water can result in local swelling and subsequent choking hazard. Choking hazards are relevant in any patient group but are particularly relevant in paediatrics, e.g., by application of non-trained caregivers (e.g. parents) and/or in stressful situations such as in the night.Accordingly, the invention is at least in part based on the surprising finding the solid pharmaceutical composition according to the invention, the compacted loaded carrier matter produced according to the invention, or the loaded carrier particle according to the invention can improve the treatment of a pediatric disease or disorder.

In certain embodiments, the invention relates to the solid pharmaceutical composition for use according to the invention, wherein the geriatric disease or disorder is a geriatric and pediatric disease or disorder.

In certain embodiments, the invention relates to the compacted loaded carrier matter for use according to the invention, or wherein the geriatric disease or disorder is a geriatric and pediatric disease or disorder.

In certain embodiments, the invention relates to the loaded carrier particle for use according to the invention, wherein the geriatric disease or disorder is a geriatric and pediatric disease or disorder.

The term "geriatric and pediatric disease or disorder", as used herein, refers to a disease or disorder that affects elderly people as well as infants and/or children. In some embodiments, a geriatric and pediatric disease or disorder as described herein is a disease or disorder that primarily affects geriatric patients and pediatric patients, that is that geriatric patients and pediatric patients are a bigger patient group than nongeriatric adults, particularly disease or disorders wherein geriatric patients and pediatric patients are at least 51 %, 60%, 70%, 80% or 90% of the patient population. In some embodiments, the phrase "for use in the treatment of a geriatric and pediatric disease or disorder" refers to use in the treatment of a disease or disorder in geriatric patients and pediatric patients.

Geriatric patients and pediatric patients have overlapping limitations and needs. Factors relevant for treatment in geriatric patients and pediatric patients include for example limited ability to swallow oral medication, compliance to treatment, altered absorption, altered distribution, altered metabolism, altered excretion, and limited functional abilities.

The means and methods provided by the invention are particularly useful for treatment in geriatric patients and pediatric patients. For example, the improved desired drug delivery properties such as fast disintegration time, e.g., of an ODT comprising a high dose of the therapeutic agent as described herein may improve drug absorption and facilitate compliance in the context of treatment of a geriatric and pediatric disease or disorders.

Accordingly, the invention is at least in part based on the surprising finding the solid pharmaceutical composition according to the invention, the compacted loaded carrier matter produced according to the invention, or the loaded carrier particle according to the invention can improve the treatment of a geriatric and pediatric disease or disorder.

In certain embodiments, the invention relates to the solid pharmaceutical composition for use according to the invention, for use in the treatment of a disease or disorder selected from the group of anxiety disorder, bipolar disorder, pain, infection, migraine, sleeping disorder, and depressive disorder.

In certain embodiments, the invention relates to the compacted loaded carrier matter for use according to the invention, for use in the treatment of a disease or disorder selected from the group of anxiety disorders, bipolar disorders, pain, infection, migraine, sleeping disorders, and depressive disorders.

In certain embodiments, the invention relates to the loaded carrier particle for use according to the invention, for use in the treatment of a disease or disorder selected from the group of anxiety disorders, bipolar disorders, pain, infections, migraine, sleeping disorders, and depressive disorders.

Symptoms of a disease or disorder selected from the group of anxiety disorder, bipolar disorder, pain, infection, migraine, sleeping disorder, and depressive disorder can complicate intake of therapeutic agents during therapy. The means and methods provided by the invention facilitate the intake of therapeutic agents in the context of a disease or disorder selected from the group of anxiety disorders, bipolar disorders, pain, infections, migraine, sleeping disorders, and depressive disorders.

Accordingly, the invention is at least in part based on the surprising finding the solid pharmaceutical composition according to the invention, the compacted loaded carrier matter produced according to the invention or the loaded carrier particle according to the invention can improve the treatment of a disease or disorder selected from the group of anxiety disorders, bipolar disorders, pain, infections, migraine, sleeping disorders, and depressive disorders.

In certain embodiments, the invention relates to the solid pharmaceutical composition for use according to the invention, for use in the treatment of an anxiety disorder.

In certain embodiments, the invention relates to the compacted loaded carrier matter for use according to the invention, for use in the treatment of an anxiety disorder.

In certain embodiments, the invention relates to the loaded carrier particle for use according to the invention, for use in the treatment of an anxiety disorder.

The term "anxiety disorder", as used herein, refers to disorders that share features of excessive fear and anxiety and related behavioral disturbances.

Examples of anxiety disorders described herein include, but are not limited to: panic attack, agoraphobia, acute stress disorder, specific phobia, panic disorder, psychoactive substance anxiety disorder, organic anxiety disorder, obsessivecompulsive anxiety disorder, posttraumatic stress disorder, separation anxiety disorder, social anxiety disorder, and generalized anxiety disorder. Anxiety as referred to herein also includes situational anxiety (e.g. as experienced by a performer before a performance). In certain embodiments, the anxiety disorder described herein is an anxiety disorder diagnosed according to the DSM-5 (American Psychiatric Association. Diagnostic and Statistical Manual of Mental Disorders. 5th ed. 2013).

Symptoms of an anxiety disorder include, without limitation, anxiety, restlessness, fatigue, difficulty concentrating, irritability, muscle tension, sleep disturbance, fear, sweating, trembling, gastrointestinal problems, increased heart rate, increased breathing rate, suicidal thoughts, and suicidal behaviors.

In certain embodiments, the invention relates to the solid pharmaceutical composition for use according to the invention, for use in the treatment of an anxiety disorder, wherein the solid pharmaceutical composition comprises an anxiolytic agent, in particular an anxiolytic agent described herein.

In certain embodiments, the invention relates to the compacted loaded carrier matter for use according to the invention, for use in the treatment of an anxiety disorder, wherein the compacted loaded carrier matter comprises an anxiolytic agent, in particular an anxiolytic agent described herein.

In certain embodiments, the invention relates to the loaded carrier particle for use according to the invention, for use in the treatment of an anxiety disorder, wherein the compacted loaded carrier matter comprises an anxiolytic agent, in particular an anxiolytic agent described herein.

Anxious patients tend to poor therapy compliance and some symptoms of anxiety disorders such as panic attacks require fast absorption of the therapeutic agent to ensure a fast onset of action. The means and methods provided by the invention facilitate intake of therapeutic agents in the context of anxiety disorders and absorption can be accelerated.

Accordingly, the invention is at least in part based on the surprising finding the solid pharmaceutical composition according to the invention, the compacted loaded carrier matter produced according to the invention, and/or the loaded carrier particle according to the invention can improve the treatment of an anxiety disorder.

In certain embodiments, the invention relates to the solid pharmaceutical composition for use according to the invention, for use in the treatment of a bipolar disorder.

In certain embodiments, the invention relates to the compacted loaded carrier matter for use according to the invention, for use in the treatment of a bipolar disorder.

In certain embodiments, the invention relates to the loaded carrier particle for use according to the invention, for use in the treatment of a bipolar disorder.

The term "bipolar disorder", as used herein, refers to a disorder characterized by unusually intense emotional states that occur in distinct periods such as mood episodes. An overly elated or overexcited state is called a manic episode, and an extremely sad or hopeless state is called a depressive episode. Symptoms of bipolar disorder include, without limitation, mood cycling (i.e., cycling between manic episodes, depressive episodes, and normal moods), obsessive fear of harm, severe aggression, territorial aggression, thermal dysregulation, night sweats, inability to fall asleep, inability to stay asleep, disorganized speech, rapid speech, loud speech, unclear speech, unusual speech timbre, disorganized thoughts, excessive ritualization, reliance on transitional objects, hoarding, extreme separation anxiety, hallucinations, delusions, and sweet cravings.

Individuals who experience manic episodes also commonly experience depressive episodes or symptoms, or mixed episodes in which features of both mania and depression are present at the same time. These episodes are usually separated by periods of "normal" mood, but in some individuals, depression and mania may rapidly alternate, known as rapid cycling. Extreme manic episodes can sometimes lead to psychotic symptoms such as delusions and hallucinations. Patients affected by bipolar disorder have had at least one manic or hypomanic (mild mania) episode. Patients with full manias and depression are indicated as having bipolar I disorder. In some embodiments, the bipolar disorder described herein refers to bipolar I disorder. Patients with hypomania and depressions are described as having bipolar II disorder. In some embodiments, the bipolar disorder described herein refers to bipolar II disorder.

The onset of episodes tends to be acute, with symptoms developing over days to weeks. Symptoms of mania or a manic episode include both mood changes and behavioral changes. Mood changes include the following: a long period of feeling "high," or an overly happy or outgoing mood; and extremely irritable mood, agitation, feeling "jumpy" or "wired." Behavioral changes include the following: talking very fast, jumping from one idea to another, having racing thoughts; being easily distracted; increasing goal-directed activities, such as taking on new projects; being restless; sleeping little; having an unrealistic belief in one's abilities; behaving impulsively and taking part in a lot of pleasurable; and high-risk behaviors, such as spending sprees, impulsive sex, and impulsive business investments. Symptoms of depression or a depressive episode include both mood changes and behavioral changes. Mood changes include the following: a long period of feeling worried or empty; and loss of interest in activities once enjoyed. Behavioral Changes include the following: feeling tired or "slowed down"; having problems concentrating, remembering, and making decisions; being restless or irritable; changing eating, sleeping, or other habits; and thinking of death or suicide, or attempting suicide.

In certain embodiments, the treatment of a bipolar disorder described herein is the treatment of a childhood-onset bipolar disorder. Childhood-onset bipolar disorder can be detected using any method known in the art. In some embodiments, childhood-onset bipolar disorder is detected by the use of the Childhood Bipolar Questionaire (CBQ).

In certain embodiments, the invention relates to the solid pharmaceutical composition for use according to the invention, for use in the treatment of a bipolar disorder, wherein the solid pharmaceutical composition comprises a sedative agent, in particular, a sedative agent described herein.

In certain embodiments, the invention relates to the compacted loaded carrier matter for use according to the invention, for use in the treatment of a bipolar disorder, wherein the compacted loaded carrier matter comprises a sedative agent, in particular, a sedative agent described herein.

In certain embodiments, the invention relates to the loaded carrier particle for use according to the invention, for use in the treatment of a bipolar disorder, wherein the compacted loaded carrier matter comprises a sedative agent, in particular, a sedative agent described herein.

Bipolar patients tend to poor therapy compliance and some symptoms of bipolar disorder such as suicidal thoughts during depressive episodes require fast absorption of the therapeutic agent to ensure a fast onset of action. The means and methods provided by the invention facilitate intake of therapeutic agents in the context of bipolar disorders and absorption can be accelerated.

Accordingly, the invention is at least in part based on the surprising finding the solid pharmaceutical composition according to the invention, the compacted loaded carrier matter produced according to the invention, or the loaded carrier particle according to the invention can improve the treatment of a bipolar disorder.

In certain embodiments, the invention relates to the solid pharmaceutical composition for use according to the invention, for use in the treatment of pain.

In certain embodiments, the invention relates to the compacted loaded carrier matter for use according to the invention, for use in the treatment of pain.

In certain embodiments, the invention relates to the loaded carrier particle for use according to the invention, for use in the treatment of pain.

The term "pain", as used herein, refers to an unpleasant sensory and emotional experience associated with, or resembling that associated with, actual or potential tissue damage. Methods for the determination of pain are known to the skilled person and include pain measurement scales, cold pressor tests, and dolorimeters.

In some embodiments, pain, as described herein, is selected from the group of headache pain neck pain, odynophagia, toothache, sore throat, pleurodynia, arthralgia, bone pain, myalgia, acute pain, delayed-onset pain, neuralgia, pain disorder, paroxysmal extreme pain disorder, chronic pain, hyperalgesia, hypoalgesia, hyperpathia, referred pain, pelvic pain, proctalgia, cancer-induced pain, withdrawalinduced pain, back pain, and low back pain.

In some embodiments, pain, as described herein, is a symptom of a disease or disorder.

In some embodiments, pain, as described herein, is classified in the classes no pain, mild pain, moderate pain, and severe pain e.g., according to the Numeric Rating Scale (NRS-11) (see, e.g., Farrar, J. T., et al., 2001, Pain, 94(2), 149-158).

In certain embodiments, the invention relates to the solid pharmaceutical composition for use according to the invention, the loaded carrier particle for use according to the invention, and/or the compacted loaded carrier matter for use according to the invention, for use in the treatment of severe pain.

In certain embodiments, the invention relates to the solid pharmaceutical composition for use according to the invention, the loaded carrier particle for use according to the invention, and/or the compacted loaded carrier matter for use according to the invention, for use in the treatment of moderate pain.

In certain embodiments, the invention relates to the solid pharmaceutical composition for use according to the invention, the loaded carrier particle for use according to the invention, and/or the compacted loaded carrier matter for use according to the invention, for use in the treatment of mild pain.

In certain embodiments, the invention relates to the solid pharmaceutical composition for use according to the invention, for use in the treatment of pain, wherein the solid pharmaceutical composition comprises a narcotic agent, in particular a narcotic agent described herein.

In certain embodiments, the invention relates to the compacted loaded carrier matter for use according to the invention, for use in the treatment of pain, wherein the compacted loaded carrier matter comprises a narcotic agent, in particular a narcotic agent described herein.

In certain embodiments, the invention relates to the loaded carrier particle for use according to the invention, for use in the treatment of pain, wherein the compacted loaded carrier matter comprises a narcotic agent, in particular a narcotic agent described herein.

In certain embodiments, the invention relates to the solid pharmaceutical composition for use according to the invention, for use in the treatment of pain, wherein the solid pharmaceutical composition comprises an analgesic agent.

In certain embodiments, the invention relates to the compacted loaded carrier matter for use according to the invention, for use in the treatment of pain, wherein the compacted loaded carrier matter comprises an analgesic agent.

In certain embodiments, the invention relates to the loaded carrier particle for use according to the invention, for use in the treatment of pain, wherein the compacted loaded carrier matter comprises an analgesic agent.

In certain embodiments, the analgesic agent described herein is paracetamol and/or an NSAID. In certain embodiments, the analgesic agent described herein is an NSAID selected from the group of acetylsalicylic acid, ibuprofen, naproxen, diclofenac, indomethacin, piroxicam, and phenylbutazone.

Treatment of pain, in particular acute pain, requires fast absorption of the therapeutic agent to ensure a fast onset of action. Furthermore, certain forms of pain, such as sore throat, can complicate the intake of the therapeutic agent. The means and methods provided by the invention facilitate intake of therapeutic agents in the context of pain and absorption can be accelerated.

Accordingly, the invention is at least in part based on the surprising finding the solid pharmaceutical composition according to the invention, the compacted loaded carrier matter produced according to the invention, or the loaded carrier particle according to the invention can improve the treatment of pain.

In certain embodiments, the invention relates to the solid pharmaceutical composition for use according to the invention, for use in the treatment of an infection.

In certain embodiments, the invention relates to the compacted loaded carrier matter for use according to the invention, for use in the treatment of an infection.

In certain embodiments, the invention relates to the loaded carrier particle for use according to the invention, for use in the treatment of an infection.

The term "infection", as used herein, refers to the invasion of at least one body tissue of a subject by one or more transmittable pathogens, their multiplication, and the reaction of host tissues to the infectious pathogen and the toxins they produce. In some embodiments, the infection described herein is a primary infection. In some embodiments, the infection described herein is an opportunistic infection. In some embodiments, the infection described herein is a secondary infection. In some embodiments, the pathogen causing the infection described herein is a virus, a bacterium, a fungus, or a parasite. In some embodiments, the infection described herein is an infection selected from the group of lower respiratory infections, HIV infection, diarrheal diseases, tuberculosis, and malaria.

In some embodiments, the infection described herein is an infection of a virus. In some embodiments, the infection described herein is an infection of a virus from the genus selected from the group consisting of Adenoviridae, Anelloviridae, Arenaviridae, Astroviridae, Bunyaviridae, Bunyavirus, Caliciviridae, Coronaviridae, Filoviridae, Flaviviridae, Hepadnaviridae, Herpesviridae, Orthomyxoviridae, Papillomaviridae, Paramyxoviridae, Parvoviridae, Picornaviridae, Pneumoviridae, Polyomaviridae, Poxviridae, Reoviridae, Retroviridae, Rhabdoviridae, Rhabdovirus and Togaviridae.

In some embodiments, the infection described herein is an infection of at least one virus selected from the group consisting of: Aichi virus, Australian bat lyssavirus, BK polyomavirus, Banna virus, Barmah forest virus, Bunyamwera virus, Bunyavirus La Crosse, Bunyavirus snowshoe hare, Cercopithecine herpesvirus, Chandipura virus, Chikungunya virus, Cosavirus A, Cowpox virus, Coxsackievirus, Crimean-Congo hemorrhagic fever virus, Dengue virus, Dhori virus, Dugbe virus, Duvenhage virus, Eastern equine encephalitis virus, Ebolavirus, Echovirus, Encephalomyocarditis virus, Epstein-Barr virus, European bat lyssavirus, GB virus C/Hepatitis G virus, Hantaan virus, Hendra virus, Hepatitis A virus, Hepatitis B virus, Hepatitis C virus, Hepatitis E virus, Hepatitis delta virus, Horsepox virus, Human adenovirus, Human astrovirus, Human coronavirus, Human cytomegalovirus, Human enterovirus 68, Human enterovirus 70, Human herpesvirus 1, Human herpesvirus 2, Human herpesvirus 6, Human herpesvirus 7, Human herpesvirus 8, Human immunodeficiency virus, Human papillomavirus 1, Human papillomavirus 2, Human papillomavirus 16, Human papillomavirus 18 Human parainfluenza, Human parvovirus B19, Human respiratory syncytial virus, Human rhinovirus, Human SARS coronavirus, Human spumaretrovirus, Human T-lymphotropic virus, Human torovirus, Influenza A virus, Influenza B virus, Influenza C virus, Isfahan virus, JC polyomavirus, Japanese encephalitis virus, Junin arenavirus, KI Polyomavirus, Kunjin virus, Lagos bat virus, Lake Victoria marburgvirus, Langat virus, Lassa virus, Lordsdale virus, Louping ill virus, Lymphocytic choriomeningitis virus, Machupo virus, Mayaro virus, MERS coronavirus, Measles virus, Mengo encephalomyocarditis virus, Merkel cell polyomavirus, Mokola virus, Molluscum contagiosum virus, Monkeypox virus, Mumps virus, Murray valley encephalitis virus, New York virus, Nipah virus, Norwalk virus, O'nyong-nyong virus, Orf virus, Oropouche virus, Pichinde virus, Poliovirus, Punta toro phlebovirus, Puumala virus, Rabies virus, Rift valley fever virus, Rosavirus A, Ross river virus, Rotavirus A, Rotavirus B, Rotavirus C, Rubella virus, Sagiyama virus, Salivirus A, Sandfly fever sicilian virus, Sapporo virus, SARS coronavirus 2, Semliki forest virus, Seoul virus, Simian foamy virus, Simian virus 5, Sindbis virus, Southampton virus, St. louis encephalitis virus, Tick-borne powassan virus, Torque teno virus, Toscana virus, Uukuniemi virus, Vaccinia virus, Varicella-zoster virus, Variola virus, Venezuelan equine encephalitis virus, Vesicular stomatitis virus, Western equine encephalitis virus, WU polyomavirus, West Nile virus, Yaba monkey tumor virus, Yaba-like disease virus, Yellow fever virus and Zika virus.

In some embodiments, the infection described herein is an infection of at least one bacterium. In some embodiments, the infection described herein is an infection of at least one bacterium from a genus selected from the group consisting of Abiotrophia, Achromobacter, Acidaminococcus, Acidovorax, Acinetobacter, Actinobacillus, Actinobaculum, Actinomadura, Actinomyces, Aerococcus, Aeromonas, Afipia, Agrobacterium, Alcaligenes, Alloiococcus Alteromonas, Amycolata, Amycolatopsis, Anaerobospirillum, Anaerorhabdus, "Anguillina", Arachnia, Arcanobacterium, Arcobacter, Arthrobacter, Atopobium, Aureobacterium, Bacillus, Bacteroides, Balneatrix, Bartonella, Bergeyella, Bifidobacterium, Bilophila, Branhamella, Borrelia, Bordetella, Brachyspira, Brevibacillus, Brevibacterium, Brevundimonas, Brucella, Burkholderia, Buttiauxella, Butyrivibrio, Calymmatobacterium, Campylobacter, Capnocytophaga, Cardiobacterium, Catonella, Cedecea, Cellulomonas, Centipeda, Chlamydia, Chlamydophila, Chromobacterium, Chyseobacterium, Chryseomonas, Citrobacter, Clostridium, Collinsella, Comamonas, Corynebacterium, Coxiella, Cryptobacterium, Delftia, Dermabacter, Dermatophilus, Desulfomonas, Desulfovibrio, Dialister, Dichelobacter, Dolosicoccus, Dolosigranulum, Edwardsiella, Eggerthella, Ehrlichia, Eikenella, Empedobacter, Enterobacter, Enterococcus, Erwinia, Erysipelothrix, Escherichia, Eubacterium, Ewingella, Exiguobacterium, Facklamia, Filifactor, Flavimonas, Flavobacterium, Flexispira, Francisella, Fusobacterium, Gardnerella, Gemella Globicatella, Gordona, Haemophilus, Hafnia, Helicobacter, Helococcus, Holdemania, Ignavigranum, Johnsonella, Kingella, Klebsiella, Kocuria, Koserella, Kurthia, Kytococcus, Lactobacillus, Lactococcus, Lautropia, Leclercia, Legionella, Leminorella, Leptospira, Leptotrichia, Leuconostoc, Listeria, Listonella, Megasphaera, Methylobacterium, Microbacterium, Micrococcus, Mitsuokella, Mobiluncus, Moellerella, Moraxella, Morganella, Mycobacterium, Mycoplasma, Myroides, Neisseria, Nocardia, Nocardiopsis, Ochrobactrum, OeskoviaOligella, Orientia, Paenibacillus, Pantoea, Parachlamydia, Pasteurella, Pediococcus, Peptococcus, Peptostreptococcus, Photobacterium, Photorhabdus, Plesiomonas Porphyrimonas, Prevotella, Propionibacterium, Proteus, Providencia, Pseudomonas, Pseudonocardia, Pseudoramibacter, Psychrobacter, Rahnella, Ralstonia, Rhodococcus, Rickettsia, Rochalimaea, Roseomonas, Rothia, Ruminococcus, Salmonella, Selenomonas, Serpulina, Serratia, Shewenella, Shigella, Simkania, Slackia, Sphingobacterium, Sphingomonas, Spirillum, Staphylococcus, Stenotrophomonas, Stomatococcus, Streptobacillus, Streptococcus, Streptomyces, Succinivibrio, Sutterella, Suttonella, Tatumella, Tissierella, Trabulsiella, Treponema, Tropheryma, Tsakamurella, Turicella, Ureaplasma, Vagococcus, Veillonella, Vibrio, Weeksella, Wolinella, Xanthomonas, Xenorhabdus, Yersinia and Yokenella.

In some embodiments, the infection described herein is an infection of at least one bacterium selected from the group consisting of: Actimomyces europeus, Actimomyces georgiae, Actimomyces gerencseriae, Actimomyces graevenitzii, Actimomyces israelii, Actimomyces meyeri, Actimomyces naeslundii, Actimomyces neuii neuii, Actimomyces neuii anitratus, Actimomyces odontolyticus, Actimomyces radingae, Actimomyces turicensis, Actimomyces viscosus, Arthrobacter creatinolyticus, Arthrobacter cumminsii, Arthrobacter woluwensis, Bacillus anthracis, Bacillus cereus, Bacillus circulans, Bacillus coagulans, Bacillus licheniformis, Bacillus megaterium, Bacillus myroides, Bacillus pumilus, Bacillus sphaericus, Bacillus subtilis, Bacillus thuringiensis, Borrelia afzelii, Borrelia andersonii, Borrelia bissettii, Borrelia burgdorferi, Borrelia garinii, Borrelia japonica, Borrelia lusitaniae, Borrelia tanukii, Borrelia turdi, Borrelia valaisiana Borrelia caucasica, Borrelia crocidurae, Borrelia recurrentis, Borrelia duttoni, Borrelia graingeri, Borrelia hermsii, Borrelia hispanica, Borrelia latyschewii, Borrelia mazzottii, Borrelia parkeri, Borrelia persica, Borrelia recurrentis, Borrelia turicatae, Borrelia venezuelensi, Bordetella bronchiseptica, Bordetella hinzii, Bordetella holmseii, Bordetella parapertussis, Bordetella pertussis, Bordetella trematum, Clostridium absonum, Clostridium argentinense, Clostridium baratii, Clostridium bifermentans, Clostridium beijerinckii, Clostridium butyricum, Clostridium cadaveris, Clostridium carnis, Clostridium celatum, Clostridium clostridioforme, Clostridium cochlearium, Clostridium cocleatum, Clostridium fallax, Clostridium ghonii, Clostridium glycolicum, Clostridium haemolyticum, Clostridium hastiforme, Clostridium histolyticum, Clostridium indolis, Clostridium innocuum, Clostridium irregulare, Clostridium leptum, Clostridium limosum, Clostridium malenominatum, Clostridium novyi, Clostridium oroticum, Clostridium paraputrificum, Clostridium piliforme, Clostridium putrefasciens, Clostridium ramosum, Clostridium septicum, Clostridium sordelii, Clostridium sphenoides, Clostridium sporogenes, Clostridium subterminale, Clostridium symbiosum, Clostridium tertium, Clostridium tetani, Escherichia coli, Escherichia fergusonii, Escherichia hermanii, Escherichia vulneris, Enterococcus avium, Enterococcus casseliflavus, Enterococcus cecorum, Enterococcus dispar, Enterococcus durans, Enterococcus faecalis, Enterococcus faecium, Enterococcus flavescens, Enterococcus gallinarum, Enterococcus hirae, Enterococcus malodoratus, Enterococcus mundtii, Enterococcus pseudoavium, Enterococcus raffinosus, Enterococcus solitarius, Haemophilus aegyptius, Haemophilus aphrophilus, Haemophilus paraphrophilus, Haemophilus parainfluenzae, Haemophilus segnis, Haemophilus ducreyi, Haemophilus influenzae, Klebsiella ornitholytica, Klebsiella oxytoca, Klebsiella planticola, Klebsiella pneumoniae, Klebsiella ozaenae, Klebsiella terrigena, Lysteria ivanovii, Lysteria monocytogenes, Mycobacterium abscessus, Mycobacterium africanum, Mycobacterium alvei, Mycobacterium asiaticum, Mycobacterium aurum, Mycobacterium avium, Mycobacterium bohemicum, Mycobacterium bovis, Mycobacterium branderi, Mycobacterium brumae, Mycobacterium celatum, Mycobacterium chelonae, Mycobacterium chubense, Mycobacterium confluentis, Mycobacterium conspicuum, Mycobacterium cookii, Mycobacterium flavescens, Mycobacterium fortuitum, Mycobacterium gadium, Mycobacterium gastri, Mycobacterium genavense, Mycobacterium gordonae, Mycobacterium goodii, Mycobacterium haemophilum, Mycobacterium hassicum, Mycobacterium intracellulare, Mycobacterium interjectum, Mycobacterium heidelberense, Mycobacterium kansasii, Mycobacterium lentiflavum, Mycobacterium leprae, Mycobacterium malmoense, Mycobacterium marinum, Mycobacterium microgenicum, Mycobacterium microti, Mycobacterium mucogenicum, Mycobacterium neoaurum, Mycobacterium nonchromogenicum, Mycobacterium peregrinum, Mycobacterium phlei, Mycobacterium scrofulaceum, Mycobacterium shimoidei, Mycobacterium simiae, Mycobacterium smegmatis, Mycobacterium szulgai, Mycobacterium terrae, Mycobacterium thermoresistabile, Mycobacterium triplex, Mycobacterium triviale, Mycobacterium tuberculosis, Mycobacterium tusciae, Mycobacterium ulcerans, Mycobacterium vaccae, Mycobacterium wolinskyi, Mycobacterium xenopi, Mycoplasma buccale, Mycoplasma faucium, Mycoplasma fermentans, Mycoplasma genitalium, Mycoplasma hominis, Mycoplasma lipophilum, Mycoplasma orale, Mycoplasma penetrans, Mycoplasma pirum, Mycoplasma pneumoniae, Mycoplasma primatum, Mycoplasma salivarium, Mycoplasma spermatophilum, Pseudomonas aeruginosa, Pseudomonas alcaligenes, Pseudomonas chlororaphis, Pseudomonas fluorescens, Pseudomonas luteola. Pseudomonas mendocina, Pseudomonas monteilii, Pseudomonas oryzihabitans, Pseudomonas pertocinogena, Pseudomonas pseudalcaligenes, Pseudomonas putida, Pseudomonas stutzeri, Rickettsia africae, Rickettsia akari, Rickettsia australis, Rickettsia conorii, Rickettsia felis, Rickettsia honei, Rickettsia japonica, Rickettsia mongolotimonae, Rickettsia prowazekii, Rickettsia rickettsiae, Rickettsia sibirica, Rickettsia slovaca, Rickettsia typhi, Salmonella choleraesuis choleraesuis, Salmonella choleraesuis arizonae, Salmonella choleraesuis bongori, Salmonella choleraesuis diarizonae, Salmonella choleraesuis houtenae, Salmonella choleraesuis indica, Salmonella choleraesuis salamae, Salmonella enteritidis, Salmonella typhi, Salmonella typhimurium, Shigella boydii, Shigella dysentaeriae, Shigella flexneri, Shigella sonnei, Staphylococcus aureus, Staphylococcus auricularis, Staphylococcus capitis capitis, Staphylococcus c. ureolyticus, Staphylococcus caprae, Staphylococcus aureus, Staphylococcus cohnii cohnii, Staphylococcus c. urealyticus, Staphylococcus epidermidis, Staphylococcus equorum, Staphylococcus gallinarum, Staphylococcus haemolyticus, Staphylococcus hominis hominis, Staphylococcus h. novobiosepticius, Staphylococcus hyicus, Staphylococcus intermedius, Staphylococcus lugdunensis, Staphylococcus pasteuri, Staphylococcus saccharolyticus, Staphylococcus saprophyticus, Staphylococcus schleiferi schleiferi, Staphylococcus s. coagulans, Staphylococcus sciuri, Staphylococcus simulans, Staphylococcus warneri, Staphylococcus xylosus, Streptococcus agalactiae, Streptococcus canis, Streptococcus dysgalactiae dysgalactiae, Streptococcus dysgalactiae equisimilis, Streptococcus equi equi, Streptococcus equi zooepidemicus, Streptococcus iniae, Streptococcus porcinus, Streptococcus pyogenes, Streptococcus anginosus, Streptococcus constellatus constellatus, Streptococcus constellatus pharyngidis, Streptococcus intermedius, Streptococcus mitis, Streptococcus oralis, Streptococcus sanguinis, Streptococcus cristatus, Streptococcus gordonii, Streptococcus parasanguinis, Streptococcus salivarius, Streptococcus vestibularis, Streptococcus criceti, Streptococcus mutans, Streptococcus ratti, Streptococcus sobrinus, Streptococcus acidominimus, Streptococcus bovis, Streptococcus equinus, Streptococcus pneumoniae, Streptococcus suis, Vibrio alginolyticus, V, carchariae, Vibrio cholerae, C. cincinnatiensis, Vibrio damsela, Vibrio fluvialis, Vibrio furnissii, Vibrio hollisae, Vibrio metschnikovii, Vibrio mimicus, Vibrio parahaemolyticus, Vibrio vulnificus, Yersinia pestis, Yersinia aldovae, Yersinia bercovieri, Yersinia enterocolitica, Yersinia frederiksenii, Yersinia intermedia, Yersinia kristensenii, Yersinia mollaretii, Yersinia pseudotuberculosis and Yersinia rohdei.

In some embodiments, the infection described herein is an infection of at least one fungus. In some embodiments, the infection described herein is an infection of at least one fungus of a genus selected from the group consisting of Candida, Aspergillus, Cryptococcus, Histoplasma, Pneumocystis, and Stachybotrys. In some embodiments, the infection described herein is an infection of at least one fungus selected from the group consisting of: Aspergillus fumigatus, Aspergillus flavus, Aspergillus Niger, Candida albicans, Candida dubliniensis, Candida glabrata (Torulopsis glabrata, Candida guilliermondii (Pichia guilliermondii; Yamadazyma guilliermondii), Candida krusei (Issatchenkia orientalis), Candida. lusitaniae (Clavispora lusitaniae), Candida parapsilosis, Candida pseudotropicalis (C. kefyr; Kluyveromyces cicerisporus; K. fragilis; K. marxianus), Candida Tropicalis, Coccidioides immitis, Cryptococcus neoformans, Pneumocystis carinii, Pneumocystis jiroveci, Blastomyces dermatitidis and Histoplasma capsulatum.

In some embodiments, the infection described herein is an infection of at least one parasite. In some embodiments, the infection described herein is an infection of at least one parasite from a genus selected from the group consisting of Ectoparasites, Protozoan organisms, and Helminths such as Tapeworms, Flukes, and/or Roundworms. In some embodiments, the infection described herein is an infection of at least one parasite selected from the group consisting of Acanthamoeba spp., Ancylostoma duodenale, Necator americanus, Angiostrongylus, Anisakis, Arachnida, Ixodidae and Argasidae, Arachnida: Trombiculidae, Archiacanthocephala, Moniliformis moniliformis, Ascaris sp. Ascaris lumbricoides, Babesia B. divergens, B. bigemina, B. equi, B. microfti, B. duncani, Balamuthia mandrillaris, Balantidium coli, Baylisascaris procyonis, Bertiella mucronata, Bertiella studeri, Blastocystis spp., Brugia malayi, Brugia timori, Cestoda, Taenia multiceps, Cimicidae, Cimex lectularius, Cimex hemipterus, Clonorchis sinensis, Clonorchis viverrini, Cochliomyia hominivorax, Cryptosporidium spp., Cyclospora cayetanensis, Demodex folliculorum, Demodex brevis, Demodex canis, Dermanyssus gallinae, Dermatobia hominis, Dicrocoelium dendriticum, Dientamoeba fragilis, Dioctophyme renale, Diphyllobothrium latum, Dracunculus medinensis, Echinococcus granulosus, Echinococcus multilocularis, E. vogeli, E. oligarthrus, Echinostoma echinatum, Entamoeba histolytica, Enterobius vermicularis, Enterobius gregorii, Fasciola hepatica, Fasciola gigantic, Fasciolopsis buski, Giardia lamblia, Gnathostoma spinigerum, Gnathostoma hispidum, Halicephalobus gingivalis, Hymenolepis nana, Hymenolepis diminuta, Insecta, Diptera, Isospora belli, Laelaps echidnina, Leishmania spp., Linguatula serrata, Liponyssoides sanguineus, Loa loa filarial, Mansonella streptocerca, Metagonimus yokogawai, Metorchis conjunctus, Naegleria fowleri, Oestroidea, Calliphoridae, Sarcophagidae, Onchocerca volvulus, Opisthorchis viverrini, Opisthorchis felineus, Clonorchis sinensis, Ornithonyssus bacoti, Ornithonyssus bursa, Ornithonyssus sylviarum, Paragonimus westermani, Paragonimus africanus, Paragonimus caliensis, Paragonimus kellicotti, Paragonimus skrjabini, Paragonimus uterobilateralis, Pediculus humanus capitis, Pediculus humanus humanus, Plasmodium falciparum, Plasmodium vivax, Plasmodium ovale curtisi, Plasmodium ovale wallikeri, Plasmodium malariae, Plasmodium knowlesi ,Pthirus pubis, Rhinosporidium seeberi, Sarcocystis bovihominis,Sarcocystis suihominis, Sarcoptes scabiei, Schistosoma haematobium, Schistosoma japonicum, Schistosoma mansoni, Schistosoma intercalatum, Schistosoma mekongi, Schistosoma sp., Siphonaptera, Pulicinae, Spirometra erinaceieuropaei, Strongyloides stercoralis, Taenia saginata, Taenia solium, Thelazia californiensis, Thelazia callipaeda, Toxocara canis, Toxocara cati, Toxascaris leonine, Toxoplasma gondii, Trichinella spiralis, Trichinella britovi, Trichinella nelsoni, Trichinella native, Trichobilharzia regenti, Schistosomatidae, Trichomonas vaginalis, Trichuris trichiura, Trichuris vulpis, Trypanosoma brucei, Trypanosoma cruzi, Tunga penetrans and Wuchereria bancrofti.

In certain embodiments, the invention relates to the solid pharmaceutical composition for use according to the invention, for use in the treatment of an infection, wherein the solid pharmaceutical composition comprises an anti-infective agent, in particular an anti-infective agent described herein.

In certain embodiments, the invention relates to the compacted loaded carrier matter for use according to the invention, for use in the treatment of an infection, wherein the compacted loaded carrier matter comprises an anti-infective agent, in particular an anti-infective agent described herein.

In certain embodiments, the invention relates to the loaded carrier particle for use according to the invention, for use in the treatment of an infection, wherein the compacted loaded carrier matter comprises an anti-infective agent, in particular an anti-infective agent described herein.

Symptoms of infections such as diminished appetite nausea and vomiting can complicate intake of therapeutic agents during therapy. The means and methods provided by the invention facilitate intake of therapeutic agents in the context of infection and absorption can be accelerated.

Accordingly, the invention is at least in part based on the surprising finding the solid pharmaceutical composition according to the invention, the compacted loaded carrier matter produced according to the invention, or the loaded carrier particle according to the invention can improve the treatment of an infection.

In certain embodiments, the invention relates to the solid pharmaceutical composition for use according to the invention, for use in the treatment of a migraine.

In certain embodiments, the invention relates to the compacted loaded carrier matter for use according to the invention, for use in the treatment of a migraine.

In certain embodiments, the invention relates to the loaded carrier particle for use according to the invention, for use in the treatment of a migraine.

The term "migraine", as used herein, refers to headaches that may be accompanied by symptoms including nausea, vomiting, or sensitivity to light. The pain associated with migraines is typically described as unilateral throbbing pain. Migraines may be preceded by visual disturbances such as aura, flashing lights, wavy lines, strange taste or odor, numbness, tingling, vertigo, tinnitus, or a feeling that part of the body is distorted in size or shape. In certain embodiments, the migraine described herein is migraine without aura. In certain embodiments, the migraine described herein is migraine with aura.

In certain embodiments, the invention relates to the solid pharmaceutical composition for use according to the invention, for use in the treatment of migraine, wherein the solid pharmaceutical composition comprises an anti-migraine agent, in particular an anti-migraine agent described herein.

In certain embodiments, the invention relates to the compacted loaded carrier matter for use according to the invention, for use in the treatment of migraine, wherein the compacted loaded carrier matter comprises an anti-migraine agent, in particular an anti-migraine agent described herein.

In certain embodiments, the invention relates to the loaded carrier particle for use according to the invention, for use in the treatment of migraine, wherein the compacted loaded carrier matter comprises an anti-migraine agent, in particular an anti-migraine agent described herein.

Symptoms of migraine such as diminished appetite nausea, reduced absorption, and vomiting can complicate intake of therapeutic agents during therapy. Furthermore, migraine attacks tend to be less severe, when treated early. The means and methods provided by the invention facilitate intake of therapeutic agents in the context of migraine and absorption can be accelerated.

Accordingly, the invention is at least in part based on the surprising finding the solid pharmaceutical composition according to the invention, the compacted loaded carrier matter produced according to the invention, or the loaded carrier particle according to the invention can improve the treatment of migraine.

In certain embodiments, the invention relates to the solid pharmaceutical composition for use according to the invention, for use in the treatment of a sleeping disorder.

In certain embodiments, the invention relates to the compacted loaded carrier matter for use according to the invention, for use in the treatment of a sleeping disorder.

In certain embodiments, the invention relates to the loaded carrier particle for use according to the invention, for use in the treatment of a sleeping disorder.

The term "sleeping disorder", as used herein, refers to disorders related to falling asleep and staying asleep. In some embodiments, the sleeping disorder described herein is a sleeping disorder selected from the group of chronic insomnia, irregular sleep-wake schedules, rotating shift work where a regular sleep schedule cannot be maintained, jet-lag, depression-related sleeping disorder. The term "insomnia" is used to describe all conditions related to the perception by the patient of inadequate or nonrestful sleep. Sleep disorders are among the most common symptoms found in general medical practice. Insomnia is a frequent complaint, being reported by 13% to 45% of the adult population. Symptoms of sleeping disorders include, without limitation frequent or continuous difficulty in falling asleep at night, frequent nocturnal awakenings, and/or early morning awakenings. Sleeplessness itself may take many forms, but it appears to be most closely related to age, sex, and the individual's psychopathological status and is of particular importance in elderly people and women. Therefore, the treatment of sleep disorders can include both inducing and prolonging the sleep of patients in need thereof.

In certain embodiments, the invention relates to the solid pharmaceutical composition for use according to the invention, for use in the treatment of a sleeping disorder, wherein the solid pharmaceutical composition comprises a narcotic agent, in particular a narcotic agent described herein.

In certain embodiments, the invention relates to the compacted loaded carrier matter for use according to the invention, for use in the treatment of a sleeping disorder, wherein the compacted loaded carrier matter comprises a narcotic agent, in particular a narcotic agent described herein.

In certain embodiments, the invention relates to the loaded carrier particle for use according to the invention, for use in the treatment of a sleeping disorder, wherein the compacted loaded carrier matter comprises a narcotic agent, in particular a narcotic agent described herein.

In certain embodiments, the invention relates to the solid pharmaceutical composition for use according to the invention, for use in the treatment of a sleeping disorder, wherein the solid pharmaceutical composition comprises a sedative agent, in particular, a sedative agent described herein.

In certain embodiments, the invention relates to the compacted loaded carrier matter for use according to the invention, for use in the treatment of a sleeping disorder, wherein the compacted loaded carrier matter comprises a sedative agent, in particular, a sedative agent described herein.

In certain embodiments, the invention relates to the loaded carrier particle for use according to the invention, for use in the treatment of a sleeping disorder, wherein the compacted loaded carrier matter comprises a sedative agent, in particular, a sedative agent described herein.

Symptoms of sleeping disorders require fast absorption of the therapeutic agent to ensure a fast onset of action. The means and methods provided by the invention facilitate intake of therapeutic agents in the context of sleeping disorders and absorption can be accelerated.

Accordingly, the invention is at least in part based on the surprising finding the solid pharmaceutical composition according to the invention, the compacted loaded carrier matter produced according to the invention, or the loaded carrier particle according to the invention can improve the treatment of a sleeping disorder.

In certain embodiments, the invention relates to the solid pharmaceutical composition for use according to the invention, for use in the treatment of a depressive disorder.

In certain embodiments, the invention relates to the compacted loaded carrier matter for use according to the invention, for use in the treatment of a depressive disorder.

In certain embodiments, the invention relates to the loaded carrier particle for use according to the invention, for use in the treatment of a depressive disorder.

The term "depressive disorder", as used herein, refers to a mental disorder typically characterized by a lasting sad mood and/or loss of interest or pleasure in most activities.

In certain embodiments, the depressive disorder described herein is a depressive disorder selected from the group of major depressive disorder, unipolar depression, depression with anxious distress, dysthymia (also referred to as dysthymic disorder), atypical depression, depression (mood), melancholic depression, psychotic depression, elderly depression, psychosocial stress-related depression, and postpartum depression.

In certain embodiments, the invention relates to the solid pharmaceutical composition for use according to the invention, for use in the treatment of a depressive disorder, wherein the solid pharmaceutical composition comprises an antidepressant agent, in particular an antidepressant agent described herein.

In certain embodiments, the invention relates to the compacted loaded carrier matter for use according to the invention, for use in the treatment of a depressive disorder, wherein the compacted loaded carrier matter comprises an antidepressant agent, in particular an antidepressant agent described herein.

In certain embodiments, the invention relates to the loaded carrier particle for use according to the invention, for use in the treatment of a depressive disorder, wherein the compacted loaded carrier matter comprises an antidepressant agent, in particular an antidepressant agent described herein.

Patients with depressive disorder tend to poor therapy compliance and some symptoms of the depressive disorder such as suicidal thoughts during depressive episodes require fast absorption of the therapeutic agent to ensure a fast onset of action. The means and methods provided by the invention facilitate intake of therapeutic agents in the context of depressive disorders and absorption can be accelerated.

Accordingly, the invention is at least in part based on the surprising finding the solid pharmaceutical composition according to the invention, the compacted loaded carrier matter produced according to the invention, or the loaded carrier particle according to the invention can improve the treatment of a depressive disorder.

In certain embodiments, the invention relates to the solid pharmaceutical composition for use according to the invention, wherein the pediatric disease or disorder is selected from the group of anxiety disorder, bipolar disorder, pain, infection, migraine, sleeping disorder, and depressive disorder.

In certain embodiments, the invention relates to the solid pharmaceutical composition for use according to the invention, wherein the geriatric disease or disorder is selected from the group of anxiety disorder, bipolar disorder, pain, infection, migraine, sleeping disorder, and depressive disorder.

In certain embodiments, the invention relates to the solid pharmaceutical composition for use according to the invention, wherein the geriatric and pediatric disease or disorder are selected from the group of anxiety disorder, bipolar disorder, pain, infection, migraine, sleeping disorder, and depressive disorder.

In certain embodiments, the invention relates to the compacted loaded carrier matter for use according to the invention, wherein the pediatric disease or disorder is selected from the group of anxiety disorder, bipolar disorder, pain, infection, migraine, sleeping disorder, and depressive disorder.

In certain embodiments, the invention relates to the compacted loaded carrier matter for use according to the invention, wherein the geriatric disease or disorder is selected from the group of anxiety disorder, bipolar disorder, pain, infection, migraine, sleeping disorder, and depressive disorder.

In certain embodiments, the invention relates to the compacted loaded carrier matter for use according to the invention, wherein the geriatric and pediatric disease or disorder is selected from the group of anxiety disorder, bipolar disorder, pain, infection, migraine, sleeping disorder, and depressive disorder.

In certain embodiments, the invention relates to the loaded carrier particle for use according to the invention, wherein the pediatric disease or disorder is selected from the group of anxiety disorder, bipolar disorder, pain, infection, migraine, sleeping disorder, and depressive disorder.

In certain embodiments, the invention relates to the loaded carrier particle for use according to the invention, wherein the geriatric disease or disorder is selected from the group of anxiety disorder, bipolar disorder, pain, infection, migraine, sleeping disorder, and depressive disorder.

In certain embodiments, the invention relates to the loaded carrier particle for use according to the invention, wherein the geriatric and pediatric disease or disorder is selected from the group of anxiety disorder, bipolar disorder, pain, infection, migraine, sleeping disorder, and depressive disorder.

In certain embodiments, the invention relates to the solid pharmaceutical composition for use in the treatment of a veterinary disease or disorder.

In certain embodiments, the invention relates to the compacted loaded carrier matter for use in the treatment of a veterinary disease or disorder.

In certain embodiments, the invention relates to the loaded carrier particle for use in the treatment of a veterinary disease or disorder.

The term "veterinary disease or disorder", as used herein, refers to any disease or disorder of a non-human animal that can be treated by a therapeutic agent described herein.

In some embodiments, the non-human animal described herein refers to a vertebrate organism. In some embodiments, the non-human animal described herein refers to a mammal. In some embodiments, the non-human animal described herein refers to a non-human animal selected from the group of cows, pigs, mice, rats, cats, dogs, camels, llamas, horses, goats, rabbits, ovis, hamsters, guinea pigs, whales, birds (e.g., a duck, a chicken, a goose), non-human primates, monkeys, apes, baboons and chimpanzees.

In some embodiments, veterinary disease or disorder described herein refers to at least one disease or disorder selected from the group of Anthrax, Brucellosis, Campylobacteriosis, Contagious ecthyma, Cryptosporidiosis, E. coli., Influenza, Leptospirosis, Listeriosis, Q fever, Rabies, Ringworm, and Salmonellosis.

Administration of treatment to a non-human animal can be complicated due to limited cooperation of the non-human animal. A veterinary disease or disorder can further hinder administration. For example, the non-human animal may have difficulties swallowing a drug due to the veterinary disease or disorder or due to lack of willingness to cooperate. The means and methods of the invention enable, facilitate and/or accelerate nasal, buccal, sublingual, intrabronchial, vaginal, urethral, and/or rectal administration of a therapeutic agent to a non-human animal.

Accordingly, the invention is at least in part based on the surprising finding the solid pharmaceutical composition according to the invention, the compacted loaded carrier matter produced according to the invention, or the loaded carrier particle according to the invention can improve the treatment of a veterinary disease or disorder.

In certain embodiments, the invention relates to the solid pharmaceutical composition for use in diagnostic purposes.

In certain embodiments, the invention relates to the compacted loaded carrier matter for use in diagnostic purposes.

In certain embodiments, the invention relates to the loaded carrier particle for use in diagnostic purposes.

In certain embodiments, the invention relates to the solid pharmaceutical composition, to the compacted loaded carrier matter and/or to the loaded carrier particle that comprise and/or are loaded with a diagnostic marker.

The term "diagnostic marker", as described herein, can be any marker that is useful in an imaging technique upon administration to a subject. In some embodiments, the diagnostic marker described herein comprises a metal selected from the group of Fe, Mg, Al, Mn, V, Ti, Cu, Ga, Ge, Ag, Au, Sm, U, Zn, Pt and Sn. In certain embodiments, the diagnostic marker described herein comprises at least one non-metal selected from the group of Si, S, Sb, I, and C. In some embodiments, the diagnostic marker described herein comprises at least one contrast agent. In some embodiments, the diagnostic marker described herein comprises at least one enriched isotope. In some embodiments, the enriched isotope is technetium-99m, iodine-123 and/or thallium-201. In some embodiments, the diagnostic marker described herein emits gamma radiation.

In certain embodiments, the invention relates to the solid pharmaceutical composition for use in scintigraphy.

In certain embodiments, the invention relates to the compacted loaded carrier matter for use in scintigraphy.

In certain embodiments, the invention relates to the loaded carrier particle for use in scintigraphy.

The use of the means and methods of the invention in scintigraphy can be enabled by the certain diagnostic markers described herein.

In the context of diagnostic purposes simple, fast and/or target specific delivery (e.g., of a diagnostic marker) can be crucial for the speed and precision of the diagnostic procedure. The means and methods of the invention can improve, facilitate and/or accelerate delivery of a diagnostic marker in a diagnostic procedure.

Accordingly, the invention is at least in part based on the surprising finding the solid pharmaceutical composition according to the invention, the compacted loaded carrier matter produced according to the invention, or the loaded carrier particle according to the invention can improve or facilitate diagnostic procedures.

"a," "an," and "the" are used herein to refer to one or to more than one (i.e., to at least one, or to one or more) of the grammatical object of the article.

"or" should be understood to mean either one, both, or any combination thereof of the alternatives.

"and/or" should be understood to mean either one, or both of the alternatives.

Throughout this specification, unless the context requires otherwise, the words "comprise", "comprises" and "comprising" will be understood to imply the inclusion of a stated step or element or group of steps or elements but not the exclusion of any other step or element or group of steps or elements.

The terms "include" and "comprise" are used synonymously. "preferably" means one option out of a series of options not excluding other options. "e.g." means one example without restriction to the mentioned example. By "consisting of" is meant including, and limited to, whatever follows the phrase "consisting of."

The terms "about" or "approximately", as used herein, refer to "within 20%", more preferably "within 10%", and even more preferably "within 5%", of a given value or range.

Reference throughout this specification to "one embodiment", "an embodiment", "a particular embodiment", "a related embodiment", "a certain embodiment", "an additional embodiment", "some embodiments", "a specific embodiment" or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments. It is also understood that the positive recitation of a feature in one embodiment, serves as a basis for excluding the feature in a particular embodiment.

An "effective amount" of an agent, e.g., a therapeutic agent, refers to an amount effective, at dosages and for periods of time necessary, to achieve the desired therapeutic or prophylactic result. Furthermore, the effective amount may depend on the individual patient's history, age, weight, family history, genetic makeup (e.g. HLA genotype), stage of myocarditis, the types of preceding or concomitant treatments, if any, and other individual characteristics of the subject to be treated.

As used herein as "subject" is an animal, such as a mammal, including a primate (such as a human a non-human primate, e.g. a monkey, and a chimpanzee), a non-primate (such as a cow a pig, a camel, a llama, a horse, a goat, a rabbit, a sheep, a hamster, a guinea pig, a cat, a dog, a rat, a mouse, a horse, and a whale), or a bird (e.g. a duck or a goose).

The invention will be further described in the following examples, which do not limit the scope of the invention described in the claims.

Brief description of Figures

Fig. 1: Electron microphotograph scan of the carrier particles before template material removal. The lamellae structure of the stratum layer is seen as petals.

Fig. 2: Electron microphotograph scan of the templated inverted particles (TIP) prepared according to the invention. The hollow voids are resulting from the removal of the template material from the particles.

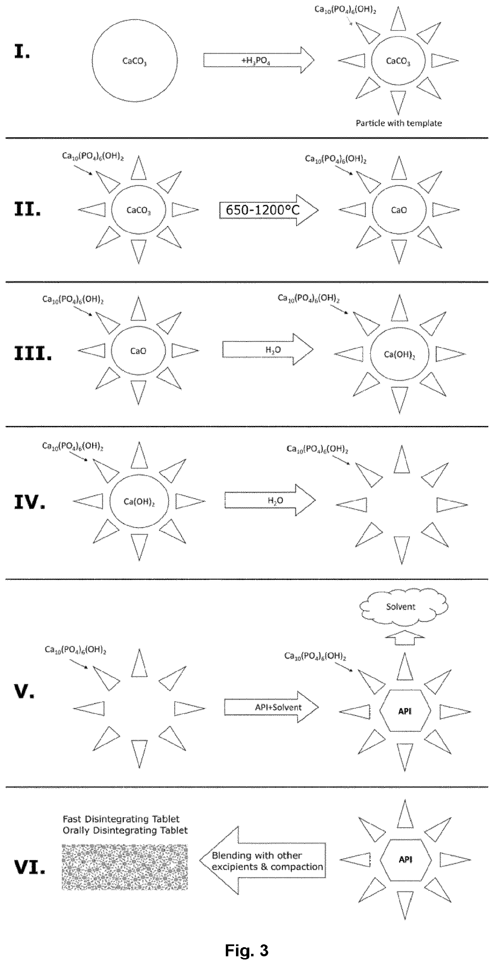

Fig. 3: Schematic illustration of an embodiment of the invention.

Fig. 4: A) Cross-section of TIP particle embedded in epoxy resin. The larger inner diameter (indicated by the longest arrow) is 23.6 micrometers, shorter inner diameter (indicated by the second longest arrow) is 18.8 micrometers. Distances between small arrows (Shell thicknesses) are (a) 5.87, (b) 4.63, (c) 6.06, (d) 5.27, and (e) 5.14 micrometers, respectively. TM4000 5kV 7.5mm x 2.50k BSE L; scale 20.0$\mu$m B) An overview of several particles.

Fig. 5: Ivermectin-loaded TIP particle. The ball-like structure is corresponding to the loaded Ivermectin. TM4000 5kV 7.8mm x 3.00k BSE H; scale 10.0$\mu$m.

Fig. 6: Partek ODT disintegration. Photograph taken after 10 seconds after the tablet is put into distilled water. Total disintegration time is approx. 60 seconds.

Fig. 7: TIP ODT disintegration. The photograph is taken after 6 seconds after contact with distilled water. At this time, the tablet is completely disintegrated.

Fig. 8: Photographs of tablet disintegration taken 1 minute after the tablets were dropped into distilled water. A) Comparison of the disintegration times between tablets prepared according to the method disclosed in CN1292803C (left) and TIP ODT (right). B) Disintegration time test for compacts prepared according to the method disclosed in US8940203. The photograph was taken after 1 minute since the porous object was dropped into distilled water. There are no signs of disintegration observable.

Fig. 9: View on the resulting porous ceramic after extraction out of the crucible.

Fig. 10: Tablet from particles made with the method disclosed in US8940203 (Example 1). Tablet is weak and is falling apart after the ejection out of the die.

Fig. 11: SEM microphotograph of the resulting ceramic made with method disclosed in US8940203 (Example 1). Cut structure to reveal the internal pores. The porous structure is clearly seen in the photo (large pores).

Fig. 12: Close-up view on a single pore. Surround material is microporous sintered hydroxyapatite.

Fig. 13: Porous ceramic prepared by the sintering method, cut to reveal the internal porous structure.

Fig. 14: TIP particles loaded with KCL up to 15% drug loading: the resulting drug-loaded TIP particles are shown. The target drug load was set to 15% w/w. The absence of the agglomerated particles and the appearance of the crystalline solid inside the TIP particles indicates successful loading

Fig. 15: TIP particles loaded with KCL up to 25% drug loading: the resulting loading with 25% target drug loading is shown. The crystal growth is initiated and proceeded until the complete cavity of the TIP particle is fully occupied.

Fig. 16: Magnolol-loaded (65% w/w drug loading) TIP particles using the capillary method and mechanical agitation. The particles loaded with 65% Magnolol are shown. The absence of external crystallization indicates that all the substance was loaded into the TIP particles.

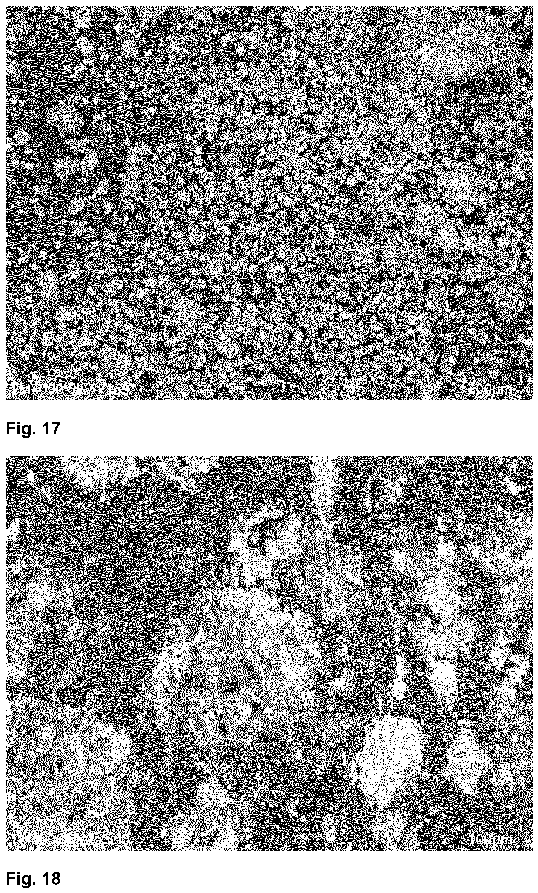

Fig. 17: Overview of MGL-TIP particles. External crystallization of Magnolol is not observed, suggesting the internalization of the drug in the TIP particles.

Fig. 18: Crossection of the MGL-TIP particles. Light gray color indicates drug substance loaded into TIP particle. White is the TIP material. Dark gray is external magnesium stearate as a support matrix for cross-sectioning.

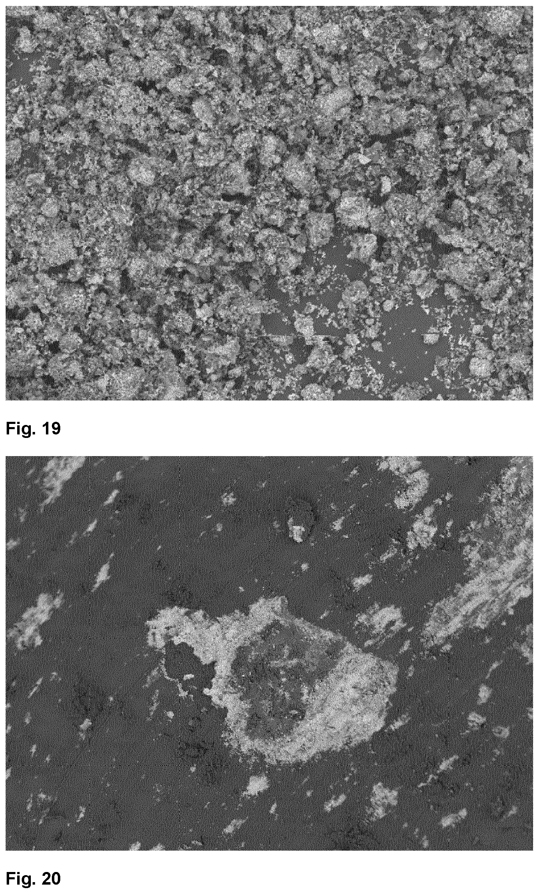

Fig. 19: Overview of RVT-TIP particles. External crystallization of Magnolol is not observed, suggesting the internalization of the drug in the TIP particles.

Fig. 20: Crossection of the RVT-TIP particles. Light gray color indicates drug substance loaded into TIP particle. White is the TIP material. Dark gray is external magnesium stearate as a support matrix for cross-sectioning.

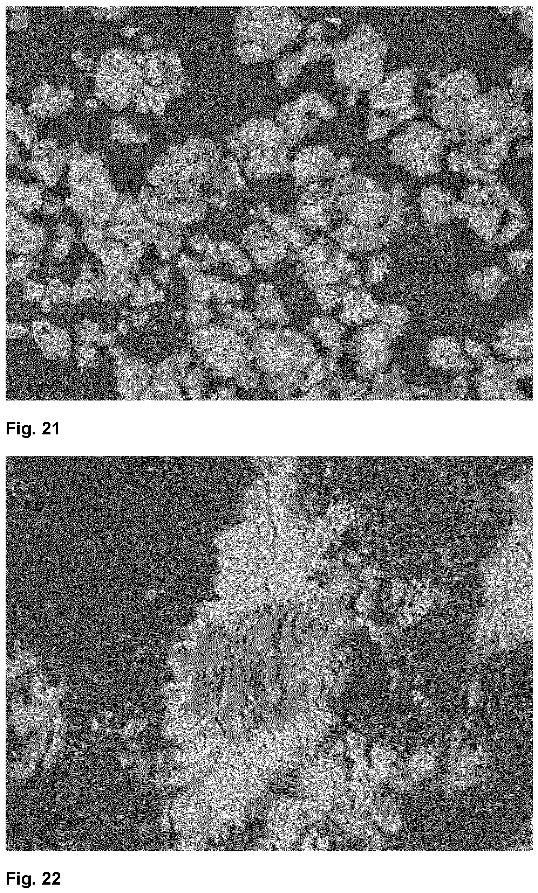

Fig. 21: Overview of P5P-TIP particles. External crystallization of Magnolol is not observed, suggesting the internalization of the drug in the TIP particles.

Fig. 22: Crossection of the P5P-TIP particles. Light gray color indicates drug substance loaded into TIP particle. White is the TIP material. Dark gray is external magnesium stearate as a support matrix for cross-sectioning.

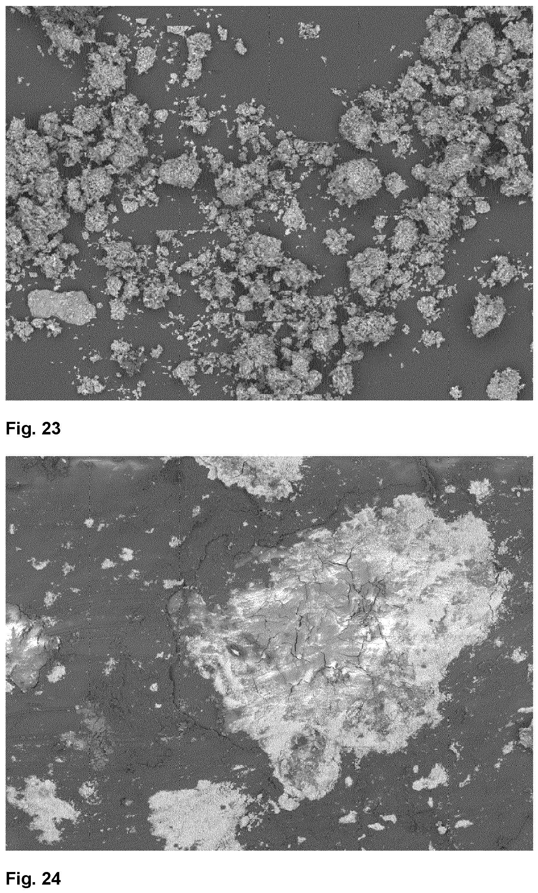

Fig. 23: Overview of ERG-TIP particles. External crystallization of Magnolol is not observed, suggesting the internalization of the drug in the TIP particles.

Fig. 24: Crossection of the ERG-TIP particles. Light gray color indicates drug substance loaded into TIP particle. White is the TIP material. Dark gray is external magnesium stearate as a support matrix for cross-sectioning.

Examples

Example 1: Production of Templated Drug Carriers

Template material (Calcium carbonate 200g, Nature 330, Lehmann&Foss&Co., Germany) was suspended in water under vigorous stirring (200-700RPM) in a smooth glass-lined or baffled reactor vessel (vessel volume 1.2 liters) with water volume of 650 ml. The set agitation speed in combination with reactor and stirrer geometries must ensure $Re > 10e+4$ (i.e., stable turbulent mixing). The chemical precipitation process is carried out by pumping a solution of orthophosphoric acid (41.1ml $H_3PO_4$(85%) diluted up to 150ml with de-ionized water) into calcium carbonate water suspension at 1.4 g acid solution per minute. During this process, the hydroxyapatite ($Ca_{10}(PO_4)_6(OH)_2$) lamellae started growing on the surface of template material thus forming the stratum layer (primary structure). The rection's stoichiometry is as follows:

$$2\ CaCO_3(solid) + 1.2\ H_3PO_4(liq.) \rightarrow 0.2\ Ca_{10}(PO_4)_6(OH)_2 + 1.6\ H_2O + 2\ CO_2(gas)$$

The template material and the carrier material were heated for 1 hour to 1100°C to induce CaO formation of the template material. Water (2 liters) was added with Büchner funnel to induce $Ca(OH)_2$ formation in the template material and to dissolve and remove the transformed template material. This step was repeated 15 times. Carrier particles with secondary internal structures were obtained after filtration and removal of water and dissolved template material.

Example 2: Therapeutic agent loading in a solvent

Midazolam hydrochloride (Hänseler AG, Switzerland) 3.5g was dissolved in ethanol (50ml). Carrier particulate material (100g) was mixed with ethanol/drug solution and dried in rotary evaporator under 40°C water bath temperature and 100 mbar pressure.

Example 3: Addition of adjuvants such as smell-modifying agent, taste-modifying agent and disintegrant

The resulting drug-loaded particles were mixed with ethanol solution of raspberry aroma (Givaudan, Switzerland), with flavor concentration of 1 drop per 10ml and dried in a vacuum cabinet for 2 hours. Cyclamate and saccharine powder mixtures, Sanaro SA, Switzerland, (1%, w/w) were added to the dry powder of loaded and flavored carrier particles. Powder blending was carried out in Turbula blender for 10 minutes. Subsequently the disintegrant was added: The sodium starch glycolate (Pharmatrans SANAQ, Switzerland) in the amount corresponding to 5% (w/w) was blended with the previously prepared powder mixture.

Example 4: Compacting

The carrier particles obtained in Example 1 were compacted using a Korsch XP 1 - R&D Single Punch Press to obtain a 6mm concave tablet with a tensile strength of $\geq$ 2.5MPa.
Tensile strength calculated according to Stanley, P.; Newton, J.M. The tensile fracture stress of capsule-shaped tablets. J. Pharm. Pharmacol. 1980, 32(12), 852-854 and Pitt, K.G.; Newton, J.M.; Stanley, P. Tensile fracture of doubly-convex cylindrical discs under diametral loading. J. Mater. Sci. 1988, 23, 2723-2728.
Registered tablet hardness was 60N, corresponding tensile strength is 2.51 Mpa.

Example 5

Manufacturing of Templated Inverted Particles (TIP) Drug Carriers

The ground calcium carbonate (Ph. Eur, USP NF, BP grade) was used as a starter material. The 200g carbonate powder was treated in a stirrer reactor vessel with 2.5L volume selected for the required batch size.
Carbonate particles were treated with 150ml of 4M phosphoric acid (Ph. Eur, USP NF, BP grade) to yield conversion to between 40% and 50%v/v. The entire volume of 4M phosphoric acid was introduced at a 2.5ml/min pumping rate.
Reactor treatment took place in deionized water. The orthophosphoric acid addition rate is controlled at 2.5ml/min. The temperature of the reactor vessel has to be maintained and kept at 90-95°C during the entire activation step, i.e., during the addition of the orthophosphoric acid.
The obtained material is filtered, washed with water, and dried. During the last washing step, it is recommended to treat the particles under elevated shear stress to reduce the particle surface roughness to increase the flowability of the final product. This intermediate material is herein called TIP-L.
TIP-L consists of 40 to 50% of hydroxyapatite in petals firmly attached to the surface of the remaining 60 to 50% of calcium carbonate.
The next step is the calcination in a high-temperature oven at the temperature range between 650° till 700°C. The calcination step was carried out at a constant temperature for 14 hours or longer.
The calcinated material was washed to dissolve the remaining template material with 0°C water, approximatively in 30L of water per 200g of TIP material. Washed material is dried in a shelf drier and packaged in bags or drums

Example 6

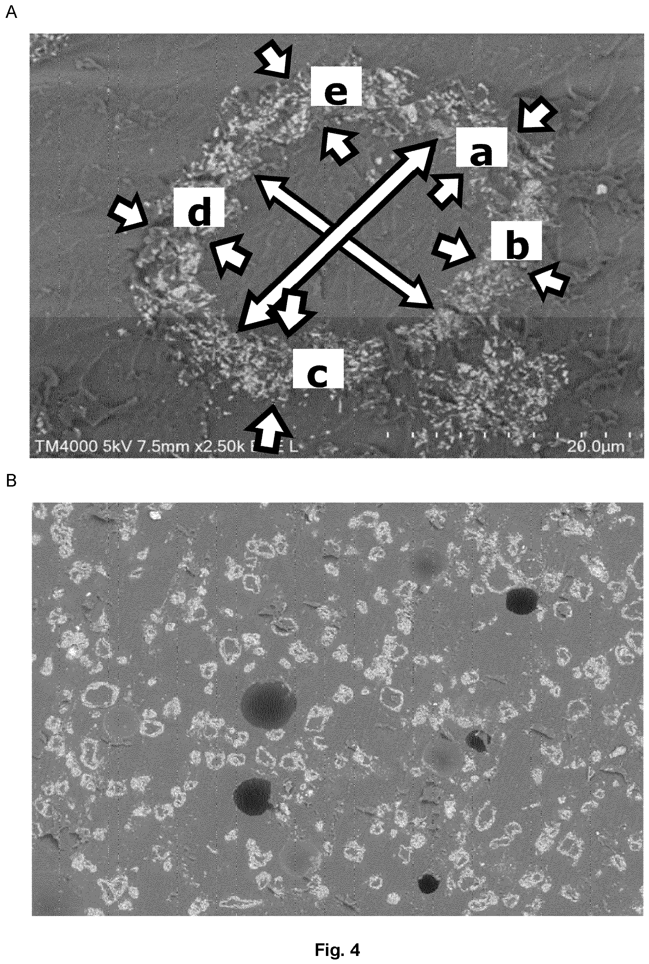

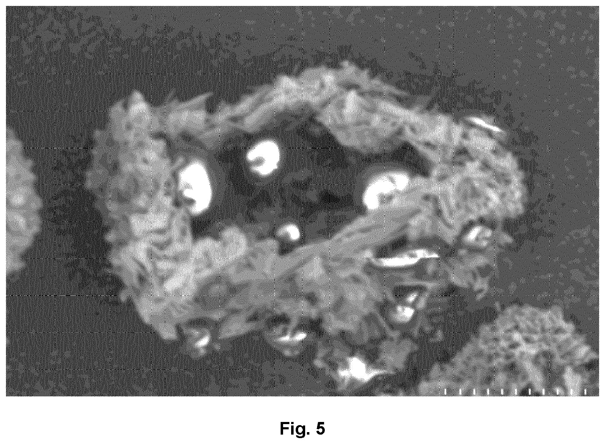

TIP material is single hollow particles with an outer diameter of 85pprox.. 20-60 microns consist of a porous hydroxyapatite shell and hollow cavity occupying not less than 30% (v/v) of a single TIP particle. TIP particles have polyhedral geometry with an aspect ratio close to 1. Each TIP particle is a single unit (Figure 4A) and not substantially aggregating with other particles (Figure 4B).
TIP particles were loaded with pharmaceutical APIs with a simple solvent evaporation method yielded particles with a smooth surface with acceptable flowability and resulted in hard yet rapidly disintegrating tablets suitable for ODT/ODF formulation development (Figure 5).

The loaded API substances were contained within the TIP particle, mainly in the hollow cavity of each individual TIP particle (Figure 5). Depending on the molecule, the API resides in either crystalline or amorphous form in the cavity.

Loading of the ivermectin was achieved by a solvent evaporation process.

The 0.100 g of ivermectin (LOT) is dissolved in absolute ethanol (100ml) and mixed with 0.200 g of TIP material. Solvent removal is carried out in the rotary evaporator (Büchi, Switzerland) under 300 mbar pressure and 50-60°C temperature. Vessel rotation speed was set to 50 RPM. The nitrogen gas injection rate is 200 cc per min. After 15 minutes the pressure was set to 200mbar for 30min, then to 100mbar for 15 min, then to 20mbar for 15 min.

The process of loading is topped when the remaining powder in the vessel is dry without visible traces of liquid.

The resulting powder is inspected for traces of external crystallization visually under the SEM. Loading of internal cavities of the TIP particles is confirmed with an SEM microphotograph in Figure 5.

The loaded TIP material is blended with disintegrant (3%, w/w), sweeteners (86pprox.. 1%, w/w) and aromas (86pprox.. 0.5%, w/w).

Simple powder blending was used for the mixing of TIP particles. The resulting powder mixture was tabletted directly under 0.5-ton compressive force.

TIP material is suitable for solidifying the oil-containing solutions, e.g., oil-soluble vitamins or aromas. Tablets prepared with TIP material have high mechanical strength, e.g., the hardness of $5\times1$mm tablet is reaching 50N, which is equivalent to 6.4Mpa tensile strength.

The resulting TIP-tablets have ultra-rapid disintegration requiring minimal, (e.g. 30% (w/w)) liquid. The latter makes TIP material very attractive for ODT/FDT solid dosage forms. After the disintegration of the tablet, the TIP particles remained intact; they do not bind more than 30% water.

Example 7 - reference method as published in CN1292803C

Reference template material was prepared as described herein and following steps of CN1292803C. In this method, polystyrene beads were used to manufacture the template material. PVC beads were prepared by the spheroidization following a "solvent exchange" mechanism according to the methods published in [J. Wang, F. Wang, H. Duan, Y. Li, J. Xu, Y. Huang, B. Liu, T. Zhang, ChemSusChem 2020, 13, 6426.] The polyvinyl chloride plastic was dissolved in dimethylacetamide and carefully dropped into the liquid containing water-ethanol mixture. The beads are immediately formed. Those beads were dried and used as templates in the next steps.

The PVC beads wetted with the bonding agent (dissolved polystyrol, Styropor) were placed into the tableting die 10mm and slightly fixed with upper and lower punches to force the beads to stick together and take the shape of the tablet.

The resulting aggregate was dried to ensure pores between the particles. The hydroxyapatite particles were mixed with a dispersing agent to yield a homogeneous mass, and the template complex was dipped in it to ensure complete penetration of the HA-PVA-PVB (Mowiol 8-88, Sigma-Aldrich, mixture of polyvinyl alcohol and traces of polyvinyl butyral) mixture inside the porous structure. The resulting mold was dried in an oven at 80-100°C for 1 hour. These last two steps were repeated five times. After the fifth drying, the mold was placed into the muffle oven. The oven temperature was raised to 600°C at the rate of 5°C per minute. After reaching the temperature, the oven was kept at 600°C for 2 hours. By the end of the 2hour hold, the temperature was set to 1100°C at the rate of 5°C/minute. The oven was held at 1100°C for 4 hours; after that, the heating was stopped, and the oven was cooled down to room temperature overnight.

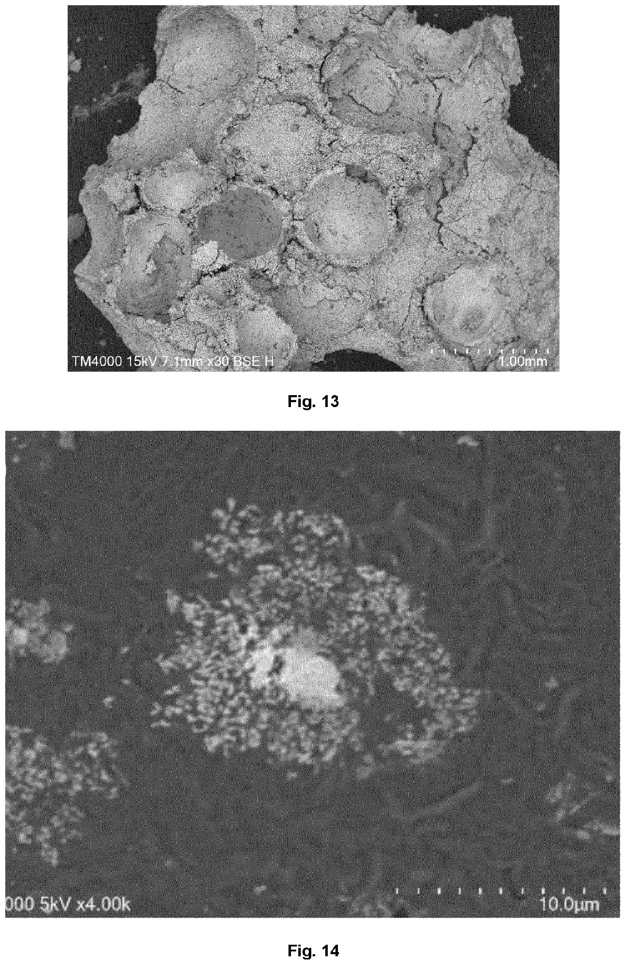

The resulting sintered mold was cut to investigate the internal structure. The SEM figure of the produced material is seen in Figure 13.

As shown in Figure 13, the porous structure was created, the pores are the same as the size of the used PVC templates. The obtained structure was compressed into a 7x2mm tablet (87pprox.. 70mg) and tested for hardness. The resulting compact was too weak to be tested, and the measured hardness was 0 or undetectable. The tablet was very difficult to handle, and it was falling apart to primary particles after a light touch with a finger.

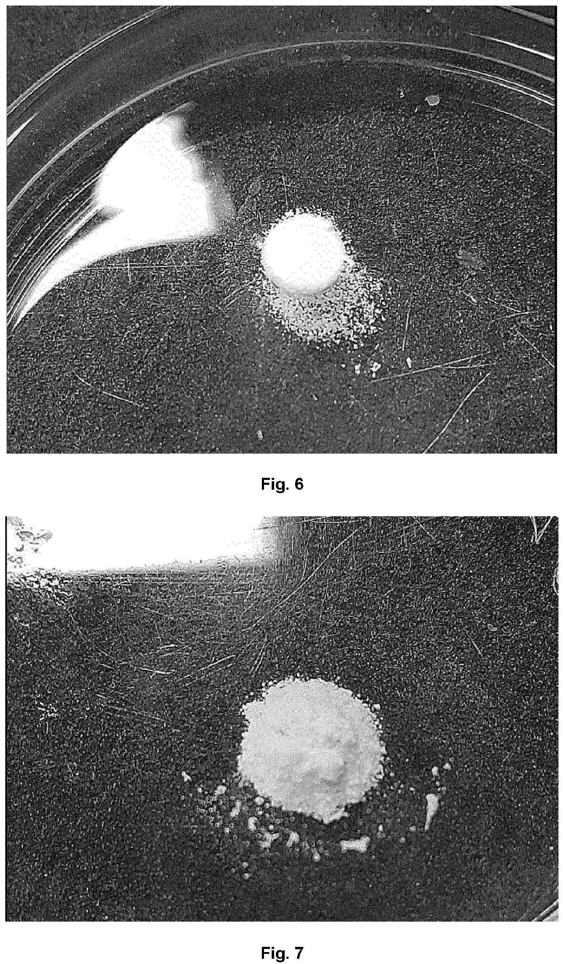

The porous structure of the mold (as in Figure 6) was lost after compression. Due to fragile tablets, it was not possible to test tablet disintegration.

Conclusion

The reference method to produce the particles with a hollow structure is not suitable for the further use of those particles in oral drug delivery. The tablets formed with this method are weak, making it impossible to handle by a patient or trained medical personal. The overall templated structure is lost after compressing it into tablets.

Example 8 - reference method as published in US8940203

The poly(N-isopropylacrylamide-co-methacrylic acid) (pNIPAM-MA) was not synthesized but purchased from Sigma-Aldrich (lot MKCF2244). All other steps done according to Example 1 of US8940203.

Slurry Preparation

1.25 g of hydroxyapatite (Hap, 21223-1KG, Sigma-Aldrich), was kept at 800° C for 1 hour. The 0.05 g of dispersant (polyacrylic acid) (PAA, Sigma-Aldrich, lot STBG0155V) were mixed together uniformly in a 50 ml glass beaker. 1ml of pNIPAM-MA hydrogel solution (1 volume pNIPAM-MA to 1 volume distilled water) was mixed by the homogenizer (Polytron PT2100, Switzerland).

The polyethylene particles were sieved out from the ground low-density polyethylene; the size fraction from the sieves was taken between 850 and 1000 micrometers. The 120 mg of the resulting polyethylene particles were added to the beaker with the pNIPAM-MA hydrogel and stirred uniformly by hand.

The resulting mass was transferred from the beaker into a 50 ml ceramic crucible. The crucible was used as a mold geometry.

Sintering Procedure

Sintering of the prepared material in the crucible was following the patent US8940203. The process consists of four stages:

Stage 1: heating to 650°C in 2 hours (about 5°C per min), holding for 30 minutes;
Stage 2: heating to 1100°C at 20°C per min; holding for 10 min.
Stage 3: Holding at 1100°C for 3 hours 10 min.
Stage 4: Cooling down to 25°C.

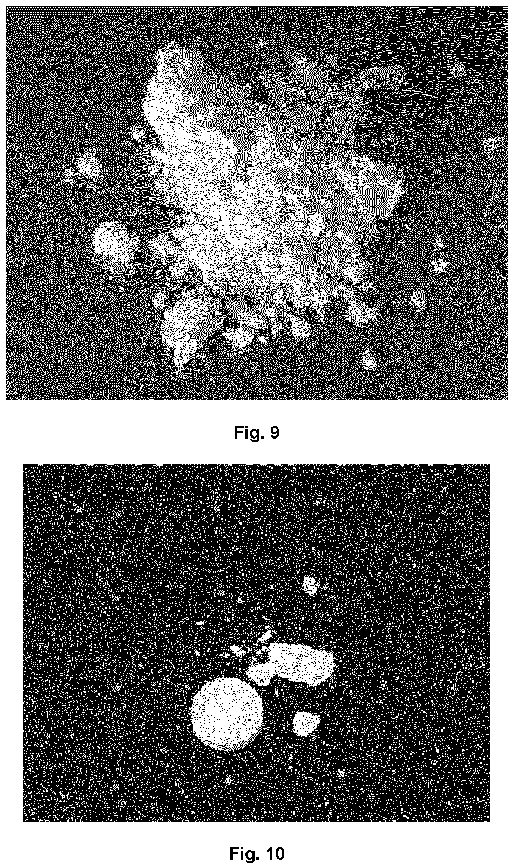

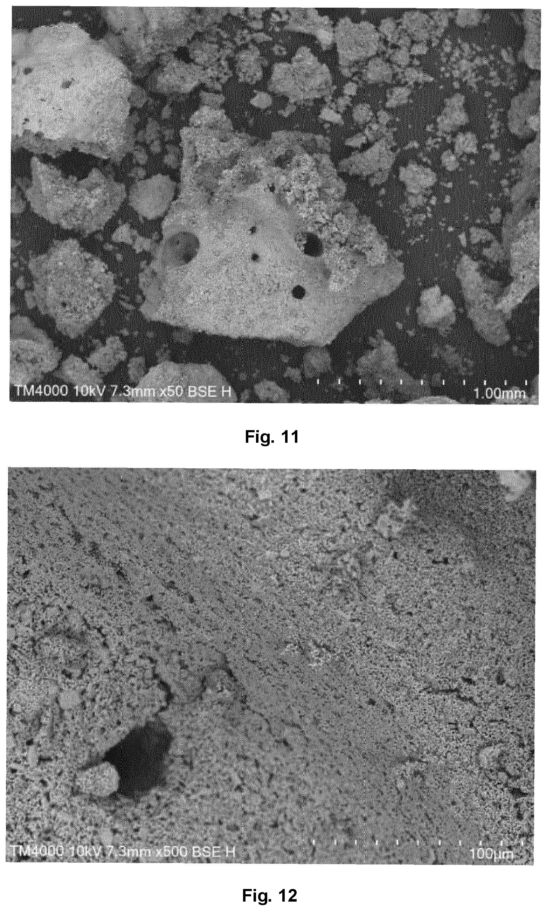

The resulting porous ceramic (Figure 9) followed the crucible shape where the sintering step was carried out. The shape was lost during the extraction due to the weak structure of the resulting ceramic (Figure 9). The SEM figures (HITACHI TM4000, Japan) were taken from the surface of the resulting pellet and ground powder (Figure 11 and Figure 12).

The porous ceramic pellet was ground through the 1000micon sieve, and the powder (100mg) was used to compress a tablet (5mm in diameter) under 1000 kg compressive force. The compressed powder formed a fragile tablet, and it disintegrated right after removing it from the die. The out-of-die hardness (Dr.Schleuniger, Switzerland) could not be measured as the powder fell apart after a light touch (Figure 10).

Due to the fact, the tablet was not formed, the disintegration time of the resulting tablet was not measured. The SEM photographs show the block of sintered HA particles with hollow cavities in the size of the PE polymer template used. These blocks can be ground into smaller particles; however, the hollow structure is lost during this operation. Therefore, loading of the API is only possible by adsorption on the surface of the ground material.

The flowability of the intact material is not possible to measure, as it is only one single particle. The ground material flowability is poor due to rough irregular-shaped particles.

Conclusion

The proposed method to produce the particles with a hollow structure is not suitable for the further use of those particles in oral drug delivery. The tablets formed with this method are weak, making it impossible to handle by a patient or trained medical personnel. The overall templated structure is lost after the grinding step or after compressing it into tablets.

Example 9 comparison of Partek ODT, the product of Example 7 and 8 to the product of the method of the invention

The primary goal was to study the formation and properties of particles according to the descriptions mentioned above, including the TIP material, and compare those characteristics to the TIP requirements.

List of comparative experiments:

1. Particle morphology. TIP is a single particle hollow capsule with a porous shell with 90pprox.. diameter of 50-100microns. Materials produced according to methods in Examples 6, 7, and 8 will be investigated under an SEM microscope and measured.

2. Particles compressibility. Tablets of 11mm in diameter will be made from equal amounts of the materials prepared according to methods in Examples 6, 7, and 8. The tablets will be compressed under a compressive pressure of 1 tonne and measured with a tablet hardness tester. Expected hardness > 50N.

3. Tablet disintegration. The materials prepared according to methods in Examples 6, 7, and 8 will be mixed with 3% w/w Croscarmellose Sodium and compressed under a compressive force of 0.5-ton. To prepare TIP-ODT the TIP material is used. To prepare TIP-L ODT the TIP-L material is used. The Amount of Croscarmellose Sodium for TIP and TIP-L ODTs is the same. Tablets will be studied for the disintegration time. Expected values < 10 seconds for complete disintegration.

4. Drug loading. The materials prepared according to methods in Examples 6, 7, and 8 will be loaded with ivermectin drug substance from saturated ethanol solution of ivermectin. Drying will be carried out in the drying cabinet under the chemical hood. The expected loading capacity is 50%. Successful loading will be investigated under the SEM microscope (Preisig et al., Drug loading into porous calcium carbonate microparticles by solvent evaporation, EJPB, 87-3, 2014) without the extra particulate crystallization.

Characterization of TIP and TIP-L materials

The SEM microphotograph of the TIP material is shown in Figure 4.

Characterization of TIP ODTs and comparative analysis

Tablet disintegration times of tablets made with TIP were compared to conventional and patent formulations. The formulations from patents US8940203 and CN1292803C were compared with TIP tablets. As a traditional formulation of ODT, a ready mixture EMPROVE® ESSENTIAL Partek® ODT (Partek ODT) mannitol-based mixture was used. The results of the comparative analysis are presented in Table 1. The parameters were measured for tablets or porous structures with 11 mm in diameter and 90pprox.. 300mg weight. Tablets were compressed at 500kg compressive force. Forms prepared according to patents US8940203 and CN1292803C were not compacted but used as-is from the cast after sintering.

Table 1. Performance characterization of compacts and forms prepared with different materials.

|  | TIP-ODT | TIP-L ODT | Partek ODT | US8940203 | CN1292803C |
|---|---|---|---|---|---|
| Disintegration time, s | <10sec. | <10sec. | Approx. 60 sec. | Not disintegrating | Not disintegrating |
| Hardness, N | 60 | 120 | 111 | 0 | 0 |

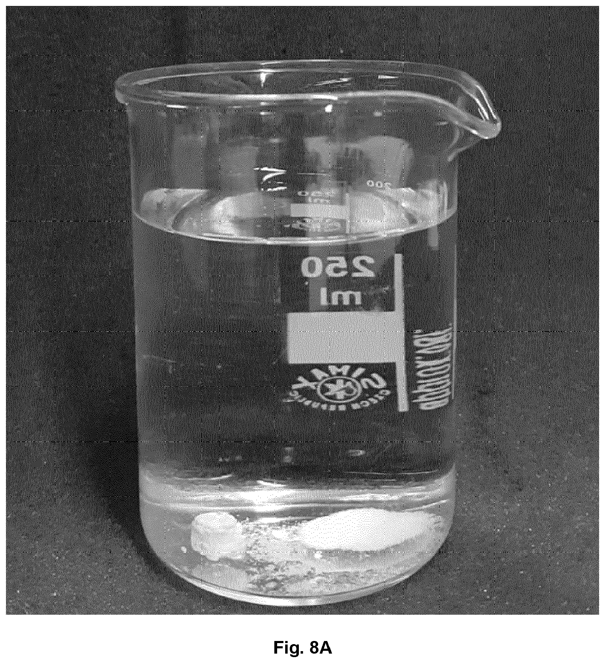

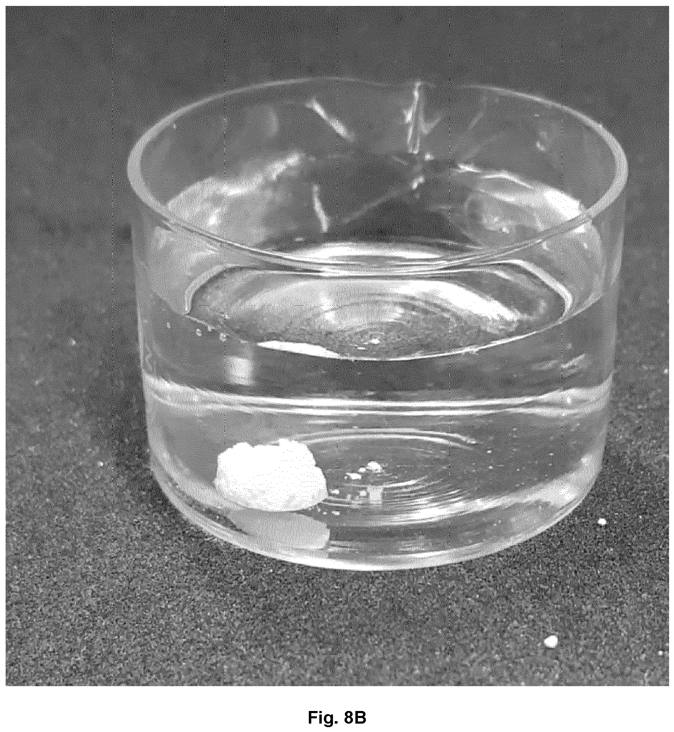

Figures 6 (Partek ODT) and 7(TIP-ODT) show the video screenshot after 10 seconds of disintegration.As seen from the experimental results and video screenshots, the TIP ODTs disintegrate significantly faster than the Partek ODT mixture. The formulations made according to the Examples 7 and 8 do not disintegrate, as shown in Figure 8A/B. The hardness of the TIP-L ODTs is higher than those of Partek ODT and TIP ODT. The tablets made according to the formulations from the Examples 7 and 8 are not stable enough to be measured with standard pharmaceutical testing equipment. The reduction in tablet hardness after thermal treatment is due to changes in the elasticity of the petals of the TIP particles under thermal stress.

Example 10: Theoretical Background

TIP particles have porous shells with the submicron size of pores and a single manifold cavity connecting all shell pores.

$$\Delta P = \frac{2\gamma \cdot \cos(\theta)}{R} \qquad \text{Eq. 1a,}$$

$$\Delta P = \frac{2\gamma}{R_c} \qquad \text{Eq. 1b}$$

This structure can be seen as a tapered capillary assembly with taper diameter increasing towards particles' centers. Such geometry is advantageous for facilitated capillary pump action delivering liquid directly into the core of the TIP particles. This effect can be calculated with the Young-Laplace equation for horizontal straight (a) and tapered (b) capillary as follows:

Where R and Rc are the radii of capillary and meniscus, respectively; $\gamma$ is the surface tension, $\theta$ is the contact angle, and delta P is the resulting counter pressure directed from the particle center to the outside of the particle.

As seen from equation 1, the counter pressure in the tapered capillary (eq. 1b) is less than that from the straight capillary (eq. 1a). The liquid flow direction due to capillary action is from the smaller orifice towards the greater one. The geometry of the TIP particles fully satisfies the best possible geometry to maximize the action potential of the capillary pumping action.

Provided that the external pressures and concentration gradients satisfy the conditions where solvent transported into the cavity of the particles can evaporate, TIP particles can be loaded with remaining solid crystalline or amorphous material until the cavities are fully occupied with the solid material.

The above-mentioned macro conditions can be easily achieved in the fluidized bed equipment where carrier gas can constantly remove solvent vapors from the surface, thus ensuring a significant concentration gradient of solvent inside and outside particles. Liquid addition with dissolved solids can be introduced with a spray nozzle which atomizes the liquid into droplets, which in turn get absorbed by the porous capillaries of the TIP particles upon collision with their surfaces.

A similar effect is achieved in the powder mixers, where the liquid distribution is effectuated through slow spray rates and mechanical mixing of the powder to ensure homogeneous contact with the spray or drops of the solvent with dissolved solids. The solvent vapors are removed by convection with or without carrier gas.

Example 11: Fluidized Bed Process Loading

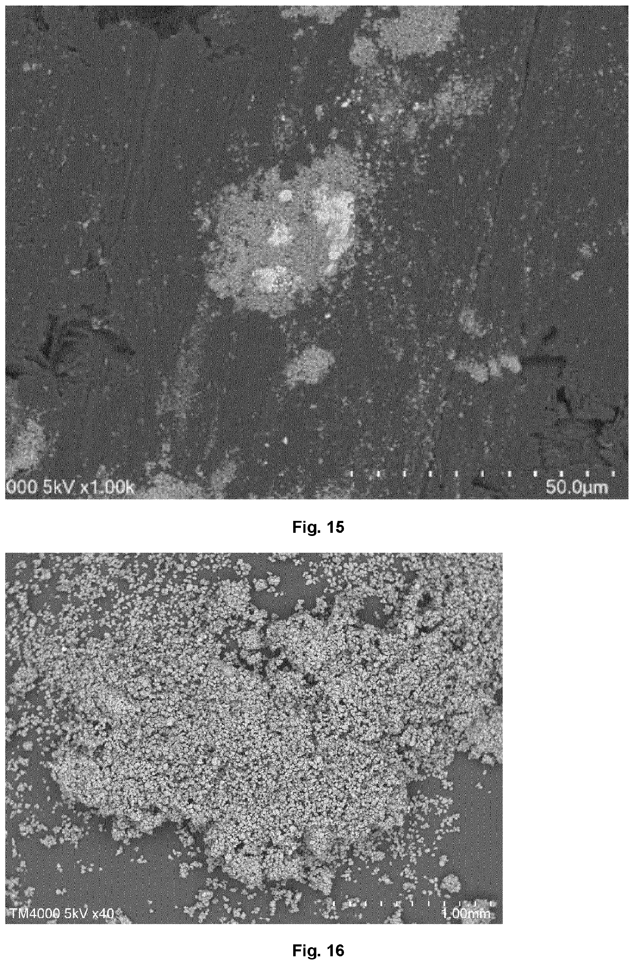

As a model substance, potassium chloride salt was used. The experiment was carried out in a small-scale fluidized bed installation with a maximum batch size of 100g. The carried gas varied from 6 to 8$m^3$/h. The temperature was set to 85°C. The spray rate of the KCl solution (15%w/v) was set to 10ml/min. The mass of empty TIP particles was 8.5g. (Figure 14, 15)

Example 12: Mechanical Mixing Loading Process

The Magnolol model substance was chosen for loading with a mechanical mixing process. This substance is insoluble in the water yet is freely soluble in ethanol. The concentration of Magnolol in Ethanol at 0.5mg/ml was used to drop on the mixing TIP powder in a small-scale powder mixer. The additional rate was set to 0.1ml/minute. This liquid addition rate was sufficient to ensure solvent evaporation from the TIP particles. The targeted drug load was set to 65% (w/w).

In Figure 16, the particles loaded with 65% Magnolol are shown. The absence of external crystallization indicates that all the substance was loaded into the TIP particles.

Example 13

Magnolol (Lot 012011069M) loaded TIP particles (MGL-TIP) were produced by dissolving 9.750g magnolol in 534ml ethanol. Empty TIP particles (14.625g) were put into a glass vessel with magnetic stirrer at approx. 50RPM. Magnolol solution was dropped into TIP particles at the rate of 1 drop per second. After every 10 drops the solution addition was interrupted to let particles to dry. The extent of drying was determined visually until the TIP particles behaviour under mixing was returning back to the same flow as for the dry particles. As soon as all of the solution was transferred into the particles the drying was carried out to remove all of the remaining ethanol. The extent of the final drying was determined visually.

Trans-resveratrol (Lot 21008) loaded TIP particles (RVT-TIP) were produced by dissolving 1.3g Trans-resveratrol in 64ml ethanol. Empty TIP particles (2.4g) were put into a glass vessel with magnetic stirrer at approx. 50RPM. Trans-resveratrol solution was dropped into TIP particles at the rate of 1 drop per second. After every 10 drops the solution addition was interrupted to let particles to dry. The extent of drying was determined visually until the TIP particles behaviour under mixing was returning back to the same flow as for the dry particles. As soon as all of the solution was transferred into the particles the drying was carried out to remove all of the remaining ethanol. The extent of the final drying was determined visually.

Pyridoxal-5-Phospahte (Lot 20210520-08-100g) loaded TIP particles (P5P-TIP) were produced by dissolving 221mg Pyridoxal-5-Phospahte in 10ml saturated aqueous calcium hydroxide solution. Empty TIP particles (663mg) were put into a glass vessel with magnetic stirrer at approx. 50RPM. Pyridoxal-5-Phospahte solution was dropped into TIP particles at the rate of 1 drop per second. After every 10 drops the solution addition was interrupted to let particles to dry. The extent of drying was determined visually until the TIP particles behaviour under mixing was returning back to the same flow as for the dry particles. As soon as all of the solution was transferred into the particles the drying was carried out to remove all of the remaining ethanol. The extent of the final drying was determined visually.

L-Ergothioneine (Lot 26034) loaded TIP particles (ERG-TIP) were produced by dissolving 65mg L-Ergothioneine in 138ml water. Empty TIP particles (195mg) were put into a glass vessel with magnetic stirrer at approx. 50RPM. L-Ergothioneine solution was dropped into TIP particles at the rate of 1 drop per second. After every 10 drops the solution addition was interrupted to let particles to dry. The extent of drying was determined visually until the TIP particles behaviour under mixing was returning back to the same flow as for the dry particles. As soon as all of the solution was transferred into the particles the drying was carried out to remove all of the remaining ethanol. The extent of the final drying was determined visually.

Imaging was made with SEM Hitachi TM4000 without gold sputtering. All samples were surface and cross-section imaged. The surface imaging was carried out by placing minute amounts on the SEM sample holder with sticky carbon film and directly imaged. The cross-section images were prepared the following way: The powder samples were mixed with magnesium stearate (approx.. 50:50 proportion); the resulting powder mixture was compressed into a tablet under 0.1kN compressive force; the resulting tablet was cut with a tomography blade. Imaging was carried out at 5kV accelerating voltage.

## Claims

1. A method for loading a carrier particle with secondary internal structures, the method comprising the steps of:

  i) providing a carrier particle with secondary internal structures;
  ii) bringing the carrier particle into contact with a loading liquid comprising a loading agent, wherein the loading liquid is partially wetting the carrier particle; and
  iii) removing the loading liquid, whereby the loading agent remains on the carrier particle;
  **characterized in that** the carrier particle is obtainable by the steps of:

    a) combining a carrier material with a template material, wherein the carrier material forms a primary structure around the template material;
    b) transforming the template material;
    c) removing the transformed template material; and
    d) obtaining carrier particles with secondary internal structures,

  preferably I) wherein combining a carrier material with a template material comprises chemical precipitation, layering and/or crystallization of the carrier material on the template material;
  II) wherein removing the template material comprises dissolution of the transformed template material to form secondary internal structures; and/or
  III) wherein transforming the template material comprises heating to a temperature from 600 °C to 1200 °C, preferably i) wherein transforming the template material comprises heating to a temperature from 600 °C to 900 °C;
  ii) wherein the step of transforming the template material comprises calcination, and/or
  iii) wherein the step of transforming the template material comprises a subsequent addition of water, preferably wherein the addition of water is an exothermic reaction.

2. The method of claim 1, wherein bringing the carrier particle into contact with a loading liquid comprises a spray loading method and/or a single droplet loading method, preferably a method selected from the group consisting of: fluidized bed, spouted bed, and fluidized bed with Wurster insert.

3. The method according to claim 1 or 2, wherein the template material is an inorganic material or consists primarily of inorganic material,
  preferably wherein the template material comprises calcium carbonate.

4. The method according to any one of the claims 1 to 3, wherein the carrier material is an inorganic material or consists primarily of inorganic material.

5. The method according to claim 3 or 4, wherein the carrier material and the template material are inorganic salts or consist primarily of inorganic salts.

6. The method according to claim 4 or 5, wherein the carrier material comprises at least one salt and/or complex selected from the group of calcium phosphate and magnesium phosphate,

preferably, wherein the carrier particles have a diameter of 1 to 300 $\mu$m; and/or
wherein the total volume of the secondary internal structures in the obtained carrier particles with secondary internal structures is in the range of $\geq$ 10% to $\leq$ 90% of the particle volume.

7. The method according to claim 6, wherein the carrier particles have a surface area between 15m2/g to 400m2/g; and/or
wherein the secondary internal structure comprises pores having a diameter size in the range of $\geq$ 0.2 $\mu$m and $\leq$ 1.5 $\mu$m.

8. The method according to any one of the claims 1 to 7, wherein the loading liquid is provided in form of droplets, preferably wherein the droplet has an average diameter size in the range of 50$\mu$m and 3mm.

9. The method according to any one of the claims 1 to 8, wherein the carrier particle is provided in a batch comprising a plurality of particles and having a batch volume, wherein the volume of the loading liquid in contact with the batch is smaller than the batch volume.

10. The method of any one of the claims 2 to 9, wherein removing the loading liquid comprises evaporation.

11. A loaded carrier particle with secondary internal structures obtainable by the method according to any one of claims 1 to 10.
preferably, wherein the carrier particle is loaded $\geq$ 60% v/v and/or wherein the loading agent is a therapeutic agent, preferably wherein the therapeutic agent is selected from the group consisting of: ivermectin, midazolam, nalbuphine, buprenorphine, omeprazole, esomeprazole, caffeine, rizatriptan, zolmitriptan, and sumatriptan.

12. A method for the production of a compacted loaded carrier matter, the method comprising the steps of:

a) loading a carrier particle according to any one of claims 1 to 11; and
b) compacting the loaded carrier particles with secondary internal structures to obtain the compacted loaded carrier matter.

13. A solid pharmaceutical composition comprising the loaded carrier particle according to any one of claims 11 or the compacted loaded carrier matter produced according to claim 12,

preferably a) wherein the therapeutic agent is selected from the group of anxiolytic agents, sedative agents, narcotic agents, antidepressant agents, anti-migraine agents, anti-inflammatory agents, and anti-infective agents, preferably an agent selected from the group consisting of: ivermectin, midazolam, nalbuphine, buprenorphine, omeprazole, esomeprazole, caffeine, rizatriptan, zolmitriptan and sumatriptan; and/or
b) wherein the solid pharmaceutical composition comprises at least one adjuvant,
preferably i) wherein the at least one adjuvant is selected from the group of disintegrants, lubricants, and flowability enhancement agents; and/or
ii) wherein the at least one adjuvant is selected from the group taste altering agents, smell altering agents, and appearance altering agents, preferably wherein the taste altering agent is selected from the group of artificial sweeteners, acidity modifiers, gums, cellulose derivatives, hard fats, and salts.

14. The solid pharmaceutical composition according to any of claims 13, the compacted loaded carrier matter produced according to claim 12, or the loaded carrier particle according to claim 11 for use as a medicine

preferably a) for use in the treatment of a veterinary disease or disorder;
b) for use in the treatment of a geriatric disease or disorder,
c) for use in the treatment of a pediatric disease or disorder; and/or
d) for use in the treatment of a disease or disorder selected from the group of anxiety disorders, bipolar disorders, pain, infections, migraine, sleeping disorders, and depressive disorders.

15. The solid pharmaceutical composition according to claim 13, the compacted loaded carrier matter produced according to claim 12, or the loaded carrier particle according to claim 11 for use in diagnostic purposes, preferably for use in scintigraphy.

| 12/18/2020 | WD | det | mode | mag | HV | HFW | —— 10 μm —— |
| 1:40:37 PM | 10.1 mm | ETD | SE | 5 000 x | 5.00 kV | 59.7 μm | Nova NanoSEM 230 |

Fig. 1

| 12/18/2020 | WD | det | mode | mag | HV | HFW | 5 μm |
|---|---|---|---|---|---|---|---|
| 2:32:00 PM | 9.7 mm | ETD | SE | 10 219 x | 5.00 kV | 29.2 μm | Nova NanoSEM 230 |

Fig. 2

**Fig. 3**

A

B

Fig. 4

Fig. 5

Fig. 6

Fig. 7

Fig. 8A

Fig. 8B

Fig. 9

Fig. 10

TM4000 10kV 7.3mm x50 BSE H                    1.00mm

Fig. 11

TM4000 10kV 7.3mm x500 BSE H                   100μm

Fig. 12

Fig. 13

Fig. 14

**Fig. 15**

**Fig. 16**

**Fig. 17**

**Fig. 18**

Fig. 19

Fig. 20

Fig. 21

Fig. 22

Fig. 23

Fig. 24

Europäisches Patentamt
European Patent Office
Office européen des brevets

# EUROPEAN SEARCH REPORT

Application Number

EP 23 15 1848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2022/162023 A1 (GALVITA AG [CH]) 4 August 2022 (2022-08-04) * the whole document * | 1-15 | INV. A61K9/50 |
| X | EP 3 260 114 A1 (OMYA INT AG [CH]) 27 December 2017 (2017-12-27) | 7-15 | |
| Y | * paragraph [0188] * * page 24 * * paragraph [0047] * | 1-15 | |
| Y | WO 2021/219458 A1 (OMYA INT AG [CH]) 4 November 2021 (2021-11-04) * the whole document * | 1-15 | |

TECHNICAL FIELDS SEARCHED (IPC)

A61K

The present search report has been drawn up for all claims

| Place of search | Date of completion of the search | Examiner |
|---|---|---|
| Munich | 18 August 2023 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 15 1848

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2023

| Patent document cited in search report | | Publication date | Patent family member(s) | | Publication date |
|---|---|---|---|---|---|
| WO 2022162023 | A1 | 04-08-2022 | AU | 2022215028 A1 | 29-06-2023 |
| | | | CA | 3200444 A1 | 04-08-2022 |
| | | | IL | 303952 A | 01-08-2023 |
| | | | WO | 2022162023 A1 | 04-08-2022 |
| EP 3260114 | A1 | 27-12-2017 | BR | 112018076573 A2 | 16-04-2019 |
| | | | CA | 3028247 A1 | 28-12-2017 |
| | | | CN | 109310636 A | 05-02-2019 |
| | | | EP | 3260114 A1 | 27-12-2017 |
| | | | EP | 3471707 A1 | 24-04-2019 |
| | | | JP | 7014787 B2 | 01-02-2022 |
| | | | JP | 2019520428 A | 18-07-2019 |
| | | | KR | 20190020055 A | 27-02-2019 |
| | | | RU | 2019101040 A | 21-07-2020 |
| | | | TW | 201803554 A | 01-02-2018 |
| | | | US | 2019328668 A1 | 31-10-2019 |
| | | | WO | 2017220498 A1 | 28-12-2017 |
| WO 2021219458 | A1 | 04-11-2021 | BR | 112022017424 A2 | 08-11-2022 |
| | | | CN | 115427003 A | 02-12-2022 |
| | | | EP | 4142675 A1 | 08-03-2023 |
| | | | TW | 202200206 A | 01-01-2022 |
| | | | US | 2023181468 A1 | 15-06-2023 |
| | | | WO | 2021219458 A1 | 04-11-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1999047253 A
- US 5376347 A
- US 6419954 B
- US 5843477 A
- US 20120164217 A
- US 6667059 B
- US 20070122475 A
- WO 1998043675 A
- CN 1292803 C
- US 8940203 B

### Non-patent literature cited in the description

- **KITAZAWA, S. et al.** *The Journal of pharmacy and pharmacology,* 1975, vol. 27 (10), 765-770
- **ROSENHOLM, J. M. et al.** *Nanoscale,* 2010, vol. 2 (10), 1870-1883
- **DONATH, E. et al.** *Angewandte Chemie International Edition,* 1998, vol. 37 (16), 2201-2205
- **DECHER, G. H. J. D. et al.** *Thin solid films,* 1992, vol. 210, 831-835
- **CARUSO, F et al.** *Science,* 1998, vol. 282 (5391), 1111-1114
- **SUCH, G. K. et al.** *Chemical Society Reviews,* 2010, vol. 40 (1), 19-29
- **SERIZAWA, T. ; KAMIMURA, S. et al.** *Langmuir,* 2002, vol. 18 (22), 8381-8385
- **ZHANG, Y. et al.** *Macromolecules,* 2003, vol. 36 (11), 4238-4240
- **CHANDRAN, R. ; PANDA, S.K. ; MALLIK, A.** A short review on the advancements in electroplating of CuInGaSe2 thin films. *Mater Renew Sustain Energy,* 2018, vol. 7, 6
- **OKADA, M. et al.** *Journal of nanoparticle research,* 2014, vol. 16 (7), 1-9
- **PATEL, R. P. et al.** *Asian Journal of Pharmaceutics (AJP),* 2014, vol. 2 (4
- **DAVID, J. ; PETER, R.** *Fundamentals of Early Clinical Drug Development: From Synthesis Design to Formulation,* 2006, 247
- **KEMPKES, M. ; EGGERS, J. ; MAZZOTTI, M.** *Chemical Engineering Science,* 2008, vol. 63 (19), 4656-4675
- **ALLEN, T.** Particle size measurement. Springer, 2013
- **AKASHKINA, L.V. ; EZERSKII, M.L.** *Pharm Chem J,* 2000, vol. 34, 324-326
- **BAUER, J. F.** *Journal of Validation Technology,* 2009, vol. 15 (1), 37-45
- **MARKL, D. et al.** *International Journal of Pharmaceutics,* 2018, vol. 538 (1-2), 188-214
- **ODEKU, O. A. et al.** *Pharmaceutical Reviews,* 2007, vol. 5 (2
- **PIETRZAK, K. et al.** *European journal of pharmaceutics and biopharmaceutics,* 2015, vol. 96, 380-387
- **DE SOUSA RODRIGUES, L. A. et al.** *Colloids and Surfaces B: Biointerfaces,* 2013, vol. 103, 642-651
- **RAUTIO, J. et al.** *Nature Reviews Drug Discovery,* 2008, vol. 7 (3), 255-270
- **DUBEY, S. ; VALECHA, V.** *World Journal of Pharmaceutical Research,* 2014, vol. 3 (7), 277-297
- **DESAI, P. M.** *Journal of pharmaceutical sciences,* 2016, vol. 105 (9), 2545-2555
- **WANG, J. et al.** *European journal of pharmaceutics and biopharmaceutics,* 2010, vol. 75 (1), 1-15
- **PAUL, S. et al.** *European Journal of Pharmaceutical Sciences,* 2018, vol. 117, 118-127
- **AUGSBURGER, L. L. ; SHANGRAW, R. F.** Effect of glidants in tableting. *Journal of Pharmaceutical Sciences,* 1966, vol. 55 (4), 418-423
- **ARMSTRONG, N. A.** Pharmaceutical Dosage Forms-Tablets. CRC Press, 2008, 267-284
- **HILDEBRANDT, C et al.** *Pharmaceutical development and technology,* 2019, vol. 24 (1), 35-47
- **MORIN, G. ; BRIENS, L.** *AAPS Pharmscitech,* 2013, vol. 14 (3), 1158-1168
- **BISWAL, P. K. et al.** *International Journal of Pharmaceutical, Chemical & Biological Sciences,* 2015, vol. 5 (4
- **AHIRE, S. B et al.** *Pharma Science Monitor,* 2012, vol. 3 (3
- ICD-11 for mortality and morbidity statistics. 2018
- Diagnostic and Statistical Manual of Mental Disorders. 2013
- **SINGH, S. ; BAJOREK, B.** *Pharmacy practice,* 2014, vol. 12 (4), 489
- **FARRAR, J. T. et al.** *Pain,* 2001, vol. 94 (2), 149-158

- **STANLEY, P. ; NEWTON, J.M.** The tensile fracture stress of capsule-shaped tablets. *J. Pharm. Pharmacol.,* 1980, vol. 32 (12), 852-854
- **PITT, K.G. ; NEWTON, J.M. ; STANLEY, P.** Tensile fracture of doubly-convex cylindrical discs under diametral loading. *J. Mater. Sci.,* 1988, vol. 23, 2723-2728
- **J. WANG ; F. WANG ; H. DUAN ; Y. LI ; J. XU ; Y. HUANG ; B. LIU ; T. ZHANG.** *ChemSusChem,* 2020, vol. 13, 6426
- **PREISIG et al.** Drug loading into porous calcium carbonate microparticles by solvent evaporation. *EJPB,* 2014, 87-3